# EUROPÄISCHE PATENTSCHRIFT

(11) **EP 1 856 022 B1**
(45) Veröffentlichungstag und Bekanntmachung des Hinweises auf die Patenterteilung: **19.08.2015**
(21) Anmeldenummer: 06708581.1
(22) Anmeldetag: 01.03.2006
(51) Int. Cl.: C07C 51/25

(54) **VERFAHREN ZUR HERSTELLUNG WENIGSTENS EINER ORGANISCHEN ZIELVERBINDUNG DURCH HETEROGEN KATALYSIERTE GASPHASEN-PARTIALOXIDATION**
METHOD FOR THE PRODUCTION OF AT LEAST ONE ORGANIC TARGET COMPOUND BY MEANS OF HETEROGENEOUSLY CATALYZED PARTIAL GAS PHASE OXIDATION
PROCEDE POUR PRODUIRE AU MOINS UN COMPOSE ORGANIQUE CIBLE PAR OXYDATION PARTIELLE EN PHASE GAZEUSE A CATALYSE HETEROGENE

(30) Priorität: 01.03.2005 DE 102005009882; 01.03.2005 US 656881 P; 12.04.2005 DE 102005017050; 12.04.2005 US 670289 P
(43) Veröffentlichungstag der Anmeldung: 21.11.2007
(73) Patentinhaber: BASF SE, 67056 Ludwigshafen (DE)
(72) Erfinder: DIETERLE, Martin, 68167 Mannheim (DE); MÜLLER-ENGEL, Klaus Joachim, 76297 Stutensee (DE); PETZOLDT, Jochen, 67273 Weisenheim am Berg (DE); HAMMON, Ulrich, 68163 Mannheim (DE)
(86) Internationale Anmeldenummer: PCT/EP2006/060360
(87) Internationale Veröffentlichungsnummer: WO 2006/092410

(56) Entgegenhaltungen:
- WO-A-01/96271
- DE-A1- 19 902 562

## Beschreibung

Vorliegende Erfindung betrifft ein Verfahren zur Herstellung wenigstens einer organischen Zielverbindung durch
a) heterogen katalysierte Gasphasen-Partialoxidation wenigstens einer organischen Vorläuferverbindung mit molekularem Sauerstoff in wenigstens zwei parallel betriebenen, Katalysatorbeschickungen enthaltenden, Oxidationsreaktorsystemen unter Erhalt von wenigstens zwei die Zielverbindung jeweils enthaltenden und jeweils auf eines der wenigstens zwei Oxidationsreaktorsysteme zurückgehenden Produktgasströmen und
b) anschließende Abtrennung der wenigstens einen Zielverbindung aus den wenigstens zwei Produktgasströmen unter Erzeugung von wenigstens einem Roh-Zielproduktstrom, bei dem man
c) vor der Abtrennung wenigstens zwei der wenigstens zwei Produktgasströme, oder im Verlauf der Abtrennung wenigstens zwei der auf dem Weg von den wenigstens zwei Produktgasgemischströmen zu dem wenigstens einen Roh-Zielproduktstrom gegebenenfalls erzeugten Zielprodukt enthaltenden Folgeströme und/oder nach der Abtrennung aus den wenigstens zwei Produktgasströmen im Verlauf der Abtrennung gegebenenfalls erzeugte Roh-Zielproduktströme miteinander zu einem Gemischstrom vermischt.

Unter einer vollständigen Oxidation einer organischen Verbindung mit molekularem Sauerstoff wird hier verstanden, dass die organische Verbindung unter reaktiver Einwirkung von molekularem Sauerstoff so umgesetzt wird, dass der in der organischen Verbindung insgesamt enthaltene Kohlenstoff in Oxide des Kohlenstoffs und der in der organischen Verbindung insgesamt enthaltene Wasserstoff in Oxide des Wasserstoffs umgewandelt wird.

Alle davon verschiedenen Umsetzungen einer organischen Verbindung unter reaktiver Einwirkung von molekularem Sauerstoff werden hier als Partialoxidation einer organischen Verbindung zusammengefasst.

D.h., der Begriff der Partialoxidation soll in dieser Schrift insbesondere auch partielle Ammoxidationen umfassen, die dadurch charakterisiert sind, dass die partielle oxidative Umsetzung der organischen Verbindung im Beisein von Ammoniak erfolgt.

Im besonderen sollen hier unter Partialoxidationen solche Umsetzungen organischer Verbindungen unter reaktiver Einwirkung von molekularem Sauerstoff verstanden werden, bei denen die partiell zu oxidierende organische Verbindung (die organische Vorläuferverbindung) nach beendeter Umsetzung wenigstens ein Sauerstoffatom mehr chemisch gebunden enthält, als vor Durchführung der Partialoxidation.

Es ist allgemein bekannt, dass durch partielle und heterogen katalysierte Oxidation verschiedenster organischer Vorläuferverbindungen mit molekularem Sauerstoff in der Gasphase zahlreiche Grundchemikalien erzeugt werden können. Beispielhaft genannt seien die Umsetzung von tert.-Butanol, iso-Buten, iso-Butan, iso-Butyraldehyd oder der Methylether des tert.-Butanols zu Methacrolein und/oder Methacrylsäure (vgl. z.B. DE-A 25 26 238, EP-A 092 097, EP-A 058 927, DE-A 41 32 263, DE-A 41 32 684 und DE-A 40 22 212), die Umsetzung von Acrolein zu Acrylsäure, die Umsetzung von Methacrolein zu Methacrylsäure (vgl. z.B. DE-A 25 26 238), die Umsetzung von o-Xylol und/oder Naphtalin zu Phthalsäureanhydrid (vgl. z.B. EP-A 522 871), von m-Xylol zu Isophthalsäure, von p-Xylol zu Terephthalsäure oder Dimethylterephthalat sowie die Umsetzung von Butadien zu Maleinsäureanhydrid (vgl. z.B. DE-A 21 06 796 und DE-A 16 24 921), die Umsetzung von n-Butan zu Maleinsäureanhydrid (vgl. z.B. GB-A 1 464 198 und GB 1 291 354), die vorgenannten Umsetzungen unter Erhalt der den Anhydriden entsprechenden Säuren, die Umsetzung von Propylen zu Acrolein und/oder Acrylsäure (vgl. z.B. DE-A 23 51 151), die Umsetzung von Indanen zu z.B. Anthrachinon (vgl. z.B. DE-A 20 25 430), die Umsetzung von Ethylen zu Ethlyenoxid oder von Propylen zu Propylenoxid (vgl. z.B. DE-AS 12 54 137, DE-A 21 59 346, EP-A 372 972, WO 89/0710, DE-A 43 11 608 und Beyer, Lehrbuch der organischen Chemie, 17. Auflage (1973), Hirzel Verlag, Stuttgart, Seite 261), die Umsetzung von Propylen und/oder Acrolein zu Acrylnitril (vgl. z.B. DE-A 23 51 151), die Umsetzung von iso-Buten und/oder Methacrolein zu Methacrylnitril, die oxidative Dehydrierung von Kohlenwasserstoffen (vgl. z.B. DE-A 23 51 151), die Umsetzung von Propan zu Acrylnitril, oder zu Acrolein und/oder zu Acrylsäure (vgl. z.B. DE-A 101 31 297, EP-A 1 090 684, EP-A 608 838, DE-A 100 46 672, EP-A 529 853, WO 01/96270 und DE-A 100 28 582), sowie die Reaktionen von Ethan zu Essigsäure, von Benzol zu Phenol sowie von 1-Buten oder 2-Buten zu den entsprechenden Butandiolen etc..

Nachteilig an den Verfahren der heterogen katalysierten Gasphasen-Partialoxidation organischer Vorläuferverbindungen ist, dass die resultierenden Produktgase die organische Zielverbindung nicht in reiner Form, sondern als Bestandteil eines Gemischs enthalten, das in der Regel zusätzlich Nebenprodukte, nicht umgesetzte Reaktanden und inerte Verdünnungsgase enthält (als ein sich unter den Bedingungen einer heterogen katalysierten Gasphasenpartialoxidation im wesentlichen inert verhaltendes Verdünnungsgas werden in dieser Schrift solche Verdünnungsgase verstanden, deren Bestandteile unter den Bedingungen der heterogen katalysierten Gasphasenpartialoxidation - jeder Bestandteil für sich betrachtet - zu mehr als 95 mol-%, vorzugsweise zu mehr als 99 mol-% chemisch unverändert erhalten bleiben).

Aus diesen Produktgas(gemisch)en muss die Zielverbindung abgetrennt werden. In der Regel wird dazu die organische Zielverbindung aus dem Produktgas (gasförmigen Produktgemisch) zunächst (gegebenenfalls nach zuvor erfolgter direkter und/oder indirekter Abkühlung) in dazu geeigneten Vorrichtungen in die kondensierte (flüssig und/oder fest) Phase überführt. Diese Überführung kann z.B. durch vollständige oder durch partielle Kondensation des Produktgases erfolgen. In einer bevorzugten Ausführungsform erfolgt sie durch fraktionierende Kondensation (z.B. in einer trennwirksame Einbauten enthaltenden Kolonne; vgl. z.B. DE-A 103 32 758 und darin zitierter Stand der Technik).

Alternativ kann die Überführung in die kondensierte Phase auch dadurch erfolgen, dass die Zielverbindung aus dem zuvor gegebenenfalls abgekühlten Produkt-gas(gemisch) in einer Absorptionsvorrichtung (z.B. in einer trennwirksame Einbauten enthaltenden Absorptionskolonne) in ein geeignetes flüssiges Absorptionsmittel aufgenommen wird (vgl. z.B. DE-A 103 36 386, US-A 2004/0242826 und den in diesen Schriften zitierten Stand der Technik). Ferner besteht die Möglichkeit, die organische Zielverbindung aus dem Produktgas(gemisch) durch Adsorption an festen Adsorptionsmaterialien oder durch ausgefrieren in die kondensierte Phase zu überführen.

Entweder enthält die kondensierte Phase die Zielverbindung (das Zielprodukt) bereits in der für die Weiterverwendung des Zielprodukts gewünschten Reinheit (dann bildet die kondensierte Phase bereits den angestrebten Roh-Zielproduktstrom; die Vorsilbe "Roh" soll dabei zum Ausdruck bringen, das der Roh-Zielproduktstrom neben der erwünschten Zielverbindung normalerweise zusätzlich noch wenigstens einen vom Zielprodukt verschiedenen Bestandteil in analytisch nachweisbaren Mengen enthält), oder es ist eine höhere Reinheit des Roh-Zielproduktstroms erwünscht. Im letzteren Fall bildet die kondensierte Phase lediglich einen Folgestrom, aus welchem der gewünschte Roh-Zielproduktstrom in an sich bekannter Weise durch Anwendung weiterer nachgeschalteter (hintereinander) Abtrennverfahren gewonnen wird. Derartige weitere Abtrennverfahren sind in der Regel hintereinandergeschaltete Extraktions- und/oder Rektifikationstrennverfahren. Gegebenenfalls können diese vorab ihrer Anwendung oder zwischendurch durch Leichtsiederstrippungen ergänzt werden (unter Leichtsiedern sollen Nebenkomponenten verstanden werden, deren Siedepunkt bei Normalbedingungen (25°C, 1atm) unterhalb des entsprechenden Siedepunktes der Zielverbindung liegt). Außerdem können die vorgenannten Abtrennverfahren durch zwischengeschaltete kristallisative Abtrennverfahren unterstützt werden. Auch können solche kristallisativen Abtrennverfahren die alleinigen Weiterreinigungsverfahren der kondensierten Phase bilden. Der von einer Reinigungsstufe (Reinigungsvorrichtung) zur nächsten Reinigungsstufe (Reinigungsvorrichtung) geförderte Zielprodukt enthaltende Stoffstrom bildet jeweils einen Folgestrom im Sinne dieser Schrift. Im allgemeinen enthält der aus einem vorausgehenden Folgestrom in einer weiteren Reinigungsstufe (Reinigungsvorrichtung) erzeugte nachfolgende Folgestrom die Zielverbindung in erhöhter Reinheit. Ein weiteres Merkmal der Herstellung organischer Zielverbindungen über heterogen katalysierte Gasphasen-Partialoxidationen organischer Vorläuferverbindungen mit molekularem Sauerstoff ist, dass es sich bei den in den heterogen katalysierten Gasphasen-Partialoxidationen zu verwendenden Katalysatoren normalerweise um Festkörper handelt.

Besonders häufig handelt es sich bei den verwendeten Katalysatoren um Oxidmassen oder um Edelmetalle (z.B. Ag). Die katalytisch aktive Oxidmasse kann neben Sauerstoff lediglich ein anderes Element oder mehr als ein anderes Element (Multielementoxidmassen) enthalten. Besonders häufig kommen als katalytisch aktive Oxidmassen solche zur Anwendung, die mehr als ein metallisches, insbesondere übergangsmetallisches, Element umfassen. In diesem Fall spricht man von Multimetalloxidmassen. Üblicherweise sind Multielementoxidmassen keine einfachen physikalischen Gemische von Oxiden der elementaren Konstituenten, sondern heterogene Gemische von komplexen Polyverbindungen dieser Elemente.

Ferner werden heterogen katalysierte Gasphasen-Partialoxidationen, insbesondere die vorgenannten, bei erhöhter Temperatur (in der Regel einige hundert °C, üblicherweise 100 bis 600°C) durchgeführt.

Da die meisten heterogen katalysierten Gasphasenpartialoxidationen stark exotherm verlaufen, führt man sie aus Gründen der Wärmeabfuhr zweckmäßigerweise häufig im Wirbelbett oder in (meist isothermen) Festbettreaktoren durch, wo sie sich in einem Reaktionsraum befinden, um den ein Wärmeaustauschmittel zum Zweck des indirekten Wärmeaustausch geleitet wird (z.B. kann sich das Katalysatorbett als Festbett in den Kontaktrohren eines Rohrbündelreaktors befinden, um die zur Wärmeabfuhr eine Salzschmelze geleitet wird).

Heterogen katalysierte Gasphasenpartialoxidationen können prinzipiell aber auch an in adiabaten Reaktoren befindlichen Katalysatorbetten durchgeführt werden.

Es ist bekannt, dass der Arbeitsdruck (Absolutdruck) bei heterogen katalysierten Gasphasenpartialoxidationen sowohl unter 1 bar, bei 1 bar oder über 1 bar liegen kann. In der Regel beträgt er 1 bis 10 bar, meist 1 bis 3 bar.

Die Umsetzung der wenigstens einen organischen Vorläuferverbindung zur Zielverbindung (die Zielumsetzung) erfolgt während der Verweilzeit des Reaktionsgasgemischs in der Katalysatorbeschickung, durch die es geleitet wird.

Aufgrund des in der Regel ausgeprägt exothermen Charakters der meisten heterogen katalysierten Gasphasenpartialoxidationen organischer Vorläuferverbindungen mit molekularem Sauerstoff, werden die Reaktionspartner üblicherweise mit einem sich unter den Bedingungen der gasphasenkatalytischen Partialoxidation im wesentlichen inerten Gas verdünnt, das mit seiner Wärmkapazität frei werdende Reaktionswärme zu absorbieren vermag.

Eines der häufigsten mitverwendeten inerten Verdünnungsgase ist molekularer Stickstoff, der automatisch immer dann zur Anwendung kommt, wenn als Sauerstoffquelle für die heterogen katalysierte Gasphasenpartialoxidation Luft verwendet wird.

Ein anderes vielfach mitverwendetes inertes Verdünnungsgas ist wegen seiner allgemeinen Verfügbarkeit Wasserdampf. Vielfach wird auch Kreisgas als inertes Verdünnungsgas mitverwendet (vgl. z.B. EP-A 1 180 508). Gemäß dem Vorstehenden besteht somit bei den meisten heterogen katalysierten Gasphasen-Partialoxidationen organischer Verbindungen das mitverwendete inerte Verdünnungsgas zu ≥90 Vol.-%, häufig zu ≥95 Vol.-%, aus N₂, H₂O und/oder CO₂. Die mitverwendeten inerten Verdünnungsgase sind einerseits dabei behilflich, die Reaktionswärme aufzunehmen und gewährleisten andererseits einen sicheren Betrieb der heterogen katalysierten Gasphasenpartialoxidation einer organischen Verbindung, indem sie das Reaktionsgasgemisch außerhalb des Explosionsbereichs halten. Bei heterogen katalysierten Gasphasenpartialoxidationen ungesättigter organischer Verbindung werden häufig auch gesättigte Kohlenwasserstoffe, d.h. brennbare Gase, als inerte Verdünnungsgase mitverwendet.

Vielfach wird die heterogen katalysierte Gasphasenpartialoxidation nicht in einem, sondern in zwei oder mehreren hintereinandergeschalteten Reaktoren durchgeführt (die in einem gemeinsamen Gehäuse auch nahtlos ineinander übergehen können). Sowohl solche Hintereinanderschaltungen von Oxidationsreaktoren als auch einzelne für sich eingesetzte Reaktoren sollen in dieser Schrift unter dem Begriff "Oxidationsreaktorsystem" subsummiert werden. In gleicher Weise soll sowohl eine einzelne Vorrichtung, ein einzelner Apparat, der zur Abtrennung der wenigstens einen Zielverbindung aus dem Produktgas(gemisch) der Partialoxidation für sich eingesetzt wird, als auch die Hintereinanderschaltung solcher Abtrennapparate (Abtrennvorrichtungen) in dieser Schrift als Abtrennsystem bezeichnet werden. Weder der Begriff Oxidationsreaktorsystem noch der Begriff Abtrennsystem beinhaltet Parallelbetrieb.

Normalerweise bildet nun eine Hintereinanderschaltung aus einem Oxidationsreaktorsystem (einer Reaktorstrasse) und einem Abtrennsystem (eine Aufarbeitungsstrasse) ein Produktionssystem (eine Produktionsstrasse) zur Herstellung organischer Zielverbindungen durch heterogen katalysierte Gasphasen-Partialoxidation wenigstens einer organischen Vorläuferverbindung mit molekularem Sauerstoff. Im Oxidationsreaktorsystem wird das wenigstens eine organische Vorläuferverbindung, molekularen Sauerstoff und wenigstens ein inertes Verdünnungsgas enthaltende Reaktionsgasausgangsgemisch durch wenigstens ein bei erhöhter Temperatur befindliches Katalysatorfestbett geführt und im Abtrennsystem wird die Zielverbindung aus dem Produktgas(gemisch) der Partialoxidation als Roh-Zielproduktstrom abgetrennt. Besteht das Abtrennsystem aus mehreren hintereinandergeschalteten Abtrennapparaten (Abtrennvorrichtungen), so bildet das Produktgas(gemisch) der Partialoxidation den der ersten Abtrennvorrichtung des Abtrennsystems zugeführten Stoffstrom. Der die letzte Abtrennvorrichtung des Abtrennsystems verlassende Stoffstrom ist der Roh-Zielproduktstrom und die innerhalb des Abtrennsystems von vorgeschalteten zu nachgeschalteten Abtrennapparaten geführten Stoffströme bilden, wie bereits ausgeführt, in dieser Schrift "Folgeströme".

Ein aus mehr als einem Reaktor bestehendes Oxidationsreaktorsystem wird insbesondere dann angewandt, wenn sich die Partialoxidation in aufeinanderfolgenden Schritten vollzieht. In diesen Fällen ist es häufig zweckmäßig, sowohl den Katalysator als auch die sonstigen Reaktionsbedingungen an den jeweiligen Reaktionsschritt optimierend anzupassen, und den jeweiligen Reaktionsschritt in einer eigenen Reaktorzone bzw. in einem eigenen Reaktor durchzuführen. In typischer Weise wird ein solches mehrstufiges Oxidationsreaktorsystem z.B. bei der Partialoxidation von Propylen zu Acrylsäure eingesetzt. In der ersten Reaktionszone (im ersten Reaktor, in der ersten Reaktionsstufe) wird das Propylen zum Acrolein, und in der zweiten Reaktionszone (im zweiten Reaktor, in der zweiten Reaktionsstufe) das Acrolein zur Acrylsäure oxidiert. In entsprechender Weise wird in der Regel auch die Methacrylsäureherstellung, meist ausgehend von iso-Buten, in zwei hintereinandergeschalteten Reaktionszonen (in zwei hintereinandergeschalteten Reaktoren) durchgeführt.

Selbstverständlich kann in einem Oxidationsreaktorsystem das Reaktionsgasgemisch zwischen zwei hintereinandergeschalteten Oxidationsreaktoren abgekühlt und/oder mit molekularem Sauerstoff (z.B. durch Luftzusatz) und/oder Inertgas ergänzt werden. Beide vorgenannten Partialoxidationen können aber auch in sogenannten Single-Reaktorsystemen durchgeführt werden, bei denen die beiden mit unterschiedlichen Katalysatoren beschickten hintereinandergeschalteten Reaktionszonen in einem einzigen, dann meist zwei Temperaturzonen aufweisenden, Reaktor eingehaust sind. Beide vorgenannten Partialoxidationen können bei Verwendung geeigneter Katalysatoren aber auch in einem einzigen, nur eine Temperaturzone aufweisenden Reaktor durchgeführt werden.

Eine Hintereinanderschaltung mehrerer Oxidationsreaktoren wird vielfach aber auch angewendet, um aus Gründen der Wärmeabfuhr oder aus anderen Gründen (vgl. DE-A 199 02 562) den Umsatz auf mehrere hintereinandergeschaltete Reaktoren zu verschmieren. In typischer Weise werden heterogen katalysierte Gasphasen-Partialoxidationen in Rohrbündelreaktoren durchgeführt, wie sie z.B. in der deutschen Anmeldung DE-A 10 2004 025 445 ausgeführt sind.

Das Abtrennsystem für durch heterogen katalysierte Partialoxidation von Propan und/oder Propylen hergestellte Acrylsäure besteht in typischer Weise aus einer Hintereinanderschaltung von Direktkühlung, Absorption, Strippung, Rektifikation(en) und gegebenenfalls Kristallisation(en) (vgl. z.B. DE-A 103 36 386 und US-A 2004/0242826).

Während es konstruktionstechnisch vergleichsweise einfach ist, Abtrennapparate für große Produktionskapazitäten zur Verfügung zu stellen, stoßen Oxidationsreaktoren hier früher an Grenzen. Dies rührt ursächlich daher, dass heterogen katalysierte Gasphasen-Partialoxidationen in der Regel ausgeprägt exotherm verlaufen. Dies führt dazu, dass mit zunehmender Produktionsgröße eines Einzelreaktors die Aufgabe der ausreichenden Wärmeabfuhr nicht mehr beherrschbar wird.

Aus Process Economics Program Report No. 6C, Acrylic Acids and Acrylic Esters, SRI International, Menlo Park California 94025 (1987), Seite 1 bis 40 ist daher bekannt, zur Herstellung von Acrylsäure zwei Reaktorstrassen parallel zu betreiben, von denen jede aus einer Hintereinanderschaltung eines Einstufenreaktors (Propylen → Acrolein) und eines Zweistufenreaktors (Acrolein → Acrylsäure) besteht. Man spricht auch von Parallelbetrieb zweier Tandemreaktoranordnungen. Das die jeweilige Tandemreaktoranordnung verlassende Produktgas wird dann mit dem die parallel betriebene Tandemreaktoranordnung verlassenden Produktgas zu einem Gemischstrom vermischt und dieser Gemischstrom nachfolgend zur Abtrennung der Acrylsäure in nur eine, den beiden Reaktorstrassen gemeinsame, Abtrennstrasse (Aufarbeitungsstrasse) geführt. Vorgenannte Betriebsweise empfiehlt auch Figur 6 der WO 01/96271 und in der DE-A 199 02 562 wird sie als klassische Parallelschaltung bezeichnet und nochmals beispielhaft ausgeführt. Der vorgenannte SRI Report war auch Bestandteil der öffentlichen Einspruchsverfahren gegen die Patente EP-B 700 714 und EP-B 700 893 und in der US-A 2004/0242826 versucht die Einsprechende die klassische Parallelschaltung nochmals zum Patent anzumelden.

Nachteilig an der klassischen Parallelschaltung, bei der die Katalysatorbeschickungen beider Reaktorstrassen sowohl parallel in Betrieb genommen als auch nachfolgend parallel betrieben werden, ist jedoch, dass sich sowohl die Zielproduktselektivität als auch die Nebenproduktselektivität in beiden Reaktorstrassen über ihre Betriebszeit synchron entwickeln.

In typischer Weise betragen solche Betriebszeiten von Katalysatorbeschickungen für heterogen katalysierte Gasphasen-Partialoxidationen je nach Katalysatorsystem und Partialoxidation mehrere Monate bis mehrere Jahre. Eine sich über solche Betriebszeiträume synchron entwickelnde Ziel- und Nebenproduktselektivität von in klassischer Weise parallel betriebenen Partialoxidationskatalysatorbeschickungen ist insofern von Nachteil, als über die genannten Betriebszeiten sowohl die Zielproduktselektivität als auch die Nebenproduktselektivitäten der Katalysatorbeschickungen in der Regel nicht konstant bleiben. Vielmehr nimmt in vielen Fällen die Zielproduktselektivität über die Betriebszeit ab und die Nebenproduktselektivität zu. Es sind aber auch Fälle bekannt, bei denen die Zielproduktselektivität über die Betriebszeit der Katalysatorbeschickung zu- und die Nebenproduktselektivität abnimmt. Das Vorgenannte trifft auch dann zu, wenn wie in der EP-A 990 636 und EP-A 1 106 598 empfohlen, der Alterung des Katalysatorbetts dadurch entgegenzuwirken versucht wird, dass im Verlauf der Betriebszeit des Katalysatorbetts unter ansonsten weitgehend gleichbleibenden Betriebsbedingungen die Betriebstemperatur des Katalysatorbetts nach und nach erhöht wird (was in der Regel gleichzeitig eine Beschleunigung des Alterungsprozesses bedingt) und/oder wenn wie in der EP-A 614 872 und in der DE-A 103 50 822 empfohlen, die Katalysatorbeschickung von Zeit zu Zeit regeneriert wird. Sowohl der in der DE-A 102 32 748 empfohlene Teilkatalysatorwechsel, als auch die in der deutschen Anmeldung DE-A 1 Q 2004 025 445 empfohlene Variation des Arbeitsdrucks vermag dem oben genannten Problem der Selektivitätsänderung nicht abzuhelfen.

Eine solchermaßen über die Betriebszeit einhergehende Selektivitätsänderung bildet jedoch eine Belastung für die über die Zeit geforderte Leistungsfähigkeit der Abtrennstrasse. Ist die Nebenproduktselektivität klein, kann sie wenig aufwendig gehalten werden, um die Trennaufgabe in befriedigender Weise zu lösen. Ist die Nebenproduktselektivität groß, muss die Abtrennstrasse aufwendig gestaltet werden, um die dann schwierigere Trennaufgabe befriedigend zu lösen.

Variiert die Nebenproduktselektivität über die Betriebszeit, muss sich die Gestaltung der Abtrennstrasse beim klassischen Parallelbetrieb daher an der höchsten während der gesamten Betriebszeit erzielten Nebenproduktselektivität orientieren, um während der gesamten Betriebszeit (bis zum Wechsel der Katalysatorbeschickung) das Roh-Zielprodukt mit der geforderten Reinheit herstellen zu können. D.h., die Gestaltung der Abtrennstrasse muss mit höchstem Aufwand erfolgen. Letzteres ist wirtschaftlich belastend. Die Aufgabe der vorliegenden Erfindung bestand daher z.B. darin, ein wie eingangs beschriebenes Verfahren zur Herstellung wenigstens einer organischen Zielverbindung durch heterogen katalysierte Gasphasen-Partialoxidation wenigstens einer organischen Vorläuferverbindung mit molekularem Sauerstoff zur Verfügung zu stellen, das wirtschaftlich weniger aufwendig ist.

Demgemäss wurde ein Verfahren zur Herstellung wenigstens einer organischen Zielverbindung durch
a) heterogen katalysierte Gasphasen-Partialoxidation wenigstens einer organischen Vorläuferverbindung mit molekularem Sauerstoff in wenigstens zwei parallel betriebenen, Katalysatorbeschickungen enthaltenden, Oxidationsreaktorsystemen unter Erhalt von wenigstens zwei die Zielverbindung jeweils enthaltenden und jeweils auf eines der wenigstens zwei Oxidationsreaktorsysteme zurückgehenden Produktgas(gemisch)strömen und
b) anschließende Abtrennung der wenigstens einen Zielverbindung aus den wenigstens zwei Produktgas(gemisch)strömen unter Erzeugung von wenigstens einem Roh-Zielproduktstrom, bei dem man
c) vor der Abtrennung wenigstens zwei der wenigstens zwei Produktgas(gemisch)ströme, oder im Verlauf der Abtrennung wenigstens zwei der auf dem Weg von den wenigstens zwei Produktgas(gemisch)strömen zu dem wenigstens einen Roh-Zielproduktstrom gegebenenfalls erzeugten Zielprodukt enthaltenden Folgeströme und/oder nach der Abtrennung aus den wenigstens zwei Produktgas(gemisch)strömen im Verlauf der Abtrennung gegebenenfalls erzeugte Roh-Zielproduktströme zu einem Gemischsstrom miteinander vermischt, gefunden, das
   dadurch gekennzeichnet ist,
   dass wenigstens eine der Katalysatorbeschickungen der wenigstens zwei parallel betriebenen Oxidationsreaktorsysteme eine Katalysatorteilmenge (bezogen auf die Katalysatorbeschickung vorzugsweise wenigstens 20 Gew.-%, bzw. wenigsten 40 Gew.-%, besser wenigstens 60 Gew.-%, noch besser wenigstens 80 Gew.-% und am besten wenigstens die Gesamtmenge der Katalysatorbeschickung) enthält, an der die heterogen katalysierte Gasphasen-Partialoxidation bereits länger durchgeführt wird, als an allen Katalysatorteilmengen der wenigstens einer anderen Katalysatorbeschickung.

In der Regel wird die Anzahl der beim erfindungsgemäßen Verfahren in erfindungsgemäßer Weise parallel betriebenen Oxidationsreaktorsysteme (das sind die Oxidationsreaktorsysteme, an denen die im Gemischstrom enthaltenen Zielverbindungen gebildet wurden) zwei betragen. Diese Anzahl kann aber auch drei, vier, fünf oder mehr betragen. Ferner werden die beim erfindungsgemäßen Verfahren in erfindungsgemäßer Weise parallel betriebenen Oxidationsreaktorsysteme vorzugsweise von identischem Reaktordesign sein. Das heißt, sie sind vorzugsweise für gleiche Produktionskapazitäten an Zielprodukt und dies auf gleiche Art und Weise ausgelegt. Im Fall von Rohrbündelreaktoren heißt dies, dass ihre Kontaktrohre in der Regel von gleicher Beschaffenheit und im wesentlichen gleicher Anzahl sind. Das gleiche gilt für das angewandte Prinzip der Wärmeabfuhr.

Grundsätzlich können die beim erfindungsgemäßen Verfahren in erfindungsgemäßer Weise parallel betriebenen Oxidationsreaktorsysteme aber auch voneinander verschieden sein. Dies gilt im Fall von Rohrbündelreaktoren z.B. sowohl hinsichtlich voneinander verschiedener Kontaktrohrbeschaffenheit (z.B. Länge, Wanddicke, Innendurchmesser, Länge, Material) als auch hinsichtlich voneinander verschiedener Kontaktrohranzahl. Auch können die erfindungsgemäß parallel betriebenen Oxidationsreaktorsysteme von völlig unterschiedlichem Typ sein. Im Regelfall wird das Beschickungsgasgemisch von erfindungsgemäß parallel betriebenen Oxidationsreaktorsystemen identisch sein. D.h., sowohl die Zusammensetzung des Beschickungsgasgemischs als auch die Belastungen der Katalysatorbeschickungen in den Oxidationsreaktorsystemen mit Beschickungsgasgemisch wird im Normalfall bei erfindungsgemäß parallel betriebenen Oxidationsreaktorsystemen gleich sein.

D.h., man kann z.B. zunächst einen Gesamtstrom an die wenigstens eine organische Vorläuferverbindung enthaltendem Reaktionsgasausgangsgemisch erzeugen und diesen anschließend über ein Verteilersystem den wenigstens zwei parallel betriebenen Oxidationsreaktorsystemen (z.B. solchen zur partialoxidativen Herstellung von Acrylsäure) zuführen.

Während man bei vorstehender Variante in natürlicher Weise lediglich einen Luftverdichter (dem auch die gegebenenfalls benötigte Sekundärluft entnommen wird) und lediglich einen Kreisgasverdichter (erfindungsgemäß bevorzugt verbleibt bei der Zielproduktabtrennung lediglich ein Kreisgas) für die wenigstens zwei parallel betriebenen Oxidationsreaktorsysteme anwendet (bevorzugt sind Radialverdichter gemäß der DE-A 10353014; dabei kann die Verdichtung des Kreisgases und der Luft in zwei getrennten Verdichtern, die mit zwei getrennten Motoren angetrieben werden, oder in zwei Verdichtern, die mit einem Motor angetrieben werden, oder in einem einzigen mit einem Motor angetriebenen Verdichter durchgeführt werden), ist es erfindungsgemäß zweckmäßig, auch dann nur einen Luftverdichter (dem auch die gegebenenfalls benötigte Sekundärluft entnommen wird) und nur einen Kreisgasverdichter anzuwenden, wenn das Reaktionsgasausgangsgemisch für jedes der wenigstens zwei parallel betriebenen Oxidationsreaktorsysteme räumlich getrennt abgemischt wird. In diesem Fall wird man die verdichteten Gase z.B. in Leitungen vorhalten, aus selbigen dem jeweiligen statischen Mischer zuführen und dort mit unter entsprechendem Druck befindlicher organischer Vorläuferbindung zum jeweiligen Reaktionsgasausgangsgemisch für das jeweilige Oxidationsreaktorsystem abmischen.

Der Eintritt der Einzelgase in die dem statischen Mischer zugeführte Leitung wird dabei in zweckmäßiger Weise häufig so gewählt, dass das Entstehen explosiver Mischungen vermieden wird (im Fall einer Partialoxidation von Propylen zu z.B. Acrolein und/oder Acrylsäure könnte diese Eintrittsabfolge in zweckmäßiger Weise z.B. zunächst Kreisgas und/oder Wasserdampf, dann (Roh-)Propen und dann Luft lauten). Das individuell erzeugte Reaktionsgasausgangsgemisch wird dann dem ihm jeweils zugeordneten Oxidationsreaktorsystem der wenigstens zwei parallel betriebenen Oxidationsreaktor-systeme zugeführt.

Unter der Belastung eines einen Reaktionsschritt katalysierenden Katalysatorbetts mit Reaktionsgas(ausgangs)gemisch wird dabei in dieser Schrift die Menge an Reaktionsgas(ausgangs)gemisch in Normlitern (= NI; das Volumen in Litern, das die entsprechende Reaktionsgas(ausgangs)gemischmenge bei Normalbedingungen, d.h., bei 25°C und 1 bar, einnehmen würde) verstanden, die pro Stunde durch einen Liter Katalysatorbett geführt wird. Die Belastung kann auch nur auf einen Bestandteil das Reaktionsgas(ausgangs)gemischs bezogen sein. Dann ist es die Menge dieses Bestandteils in NI/I•h, die pro Stunde durch einen Liter des Katalysatorbetts geführt wird. Reine Inertmaterialschnittungen werden dabei nicht zum Katalysatorbett gerechnet.

Das gleiche gilt für den Arbeitsdruck und die Arbeitstemperatur in den erfindungsgemäß parallel betriebenen Oxidationsreaktorsystemen. Selbstredend können die vorgenannten Parameter (Zusammensetzung des Beschickungsgasgemischs (bei gleicher Vorläuferverbindung und gleichem Zielprodukt), Belastung der Katalysatorbeschickungen mit organischer Vorläuferverbindung bzw. Reaktionsgasgemisch, Arbeitstemperatur, Arbeitsdruck) aber auch einzeln oder in Gruppierungen voneinander verschieden sein. Vom Typ her (d.h., in ihrer chemischen und physikalischen Beschaffenheit) wird die Katalysatorbeschickung in den erfindungsgemäß parallel betriebenen Oxidationsreaktorsystemen häufig identisch sein (abgesehen von den durch verschiedene Betriebsdauern bedingten Unterschieden). Die erfindungsgemäß parallel betriebenen Oxidationsreaktorsysteme können aber auch mit Katalysatoren von unterschiedlichem Typ beschickt sein.

Erfindungsgemäß wesentlich ist, dass wenigstens eine der relevanten Katalysatorbeschickungen (das sind die Katalysatorbeschickungen an denen die im Gemischstrom enthaltenen Zielverbindungen gebildet wurden) der wenigstens zwei erfindungsgemäß parallel betriebenen Oxidationsreaktorsysteme wenigstens eine Katalysatorteilmenge enthält, an der die heterogen katalysierte Gasphasen-Partialoxidation bereits über einen längeren Zeitraum durchgeführt wird, als an allen Katalysatorteilmengen der wenigstens einen anderen Katalysatorbeschickung.

Dieses erfindungsgemäße Merkmal ist in einfacher Weise z.B. dadurch realisierbar, dass man zunächst wenigstens zwei Oxidationsreaktorsysteme, die z.B. eine identische Beschickung mit Katalysator aufweisen, parallel mit z.B. identischem Beschickungsgasgemisch und unter identischen sonstigen Reaktionsbedingungen in Betrieb nimmt und über längere Zeit betreibt. Fällt die Selektivität der Zielproduktbildung mit zunehmender Betriebsdauer infolge Alterung der Katalysatorbeschickung (z.B. auf einen Wert, der für die Zielproduktabtrennung bezüglich der gewünschten Reinheit des Roh-Zielprodukts prohibitiv wäre), kann man die erfindungsgemäße Betriebsweise z.B. auf einfache Weise dadurch erreichen, dass man lediglich in einem der wenigstens zwei parallel betriebenen Oxidationsreaktorsysteme einen Teilkatalysatorwechsel z.B. gemäß der DE-A 102 32 748 vornimmt. Daran anschließend kann der Parallelbetrieb in erfindungsgemäßer Weise fortgesetzt werden. Die Zielproduktabtrennung wird lediglich mit einer Nebenproduktmischselektivität belastet und vermag das Roh-Zielprodukt weiterhin mit der gewünschten Reinheit zu produzieren.

Auf diese Weise lässt sich die Standzeit derjenigen Katalysatorbeschickung, an der kein Katalysatorteilwechsel vorgenommen wurde, ohne Mehraufwand signifikant verlängern. Selbstverständlich kann man anstelle nur eine Teilmenge einer Katalysatorbeschickung durch Frischkatalysator zu ersetzen, auch die Gesamtmenge dieser Katalysatorbeschickung durch Frischkatalysator ersetzen und nachfolgend erfindungsgemäß weiterverfahren.

Erfindungsgemäß vorteilhaft wird man die Produktgas(gemisch)ströme von wenigstens zwei in erfindungsgemäßer Weise parallel betriebenen Oxidationsreaktorsystemen vor deren Eintritt in die erste Vorrichtung der Zielproduktabtrennung zu einem Gemischstrom vereinigen. Selbstverständlich kann die Zielproduktabtrennung beim erfindungsgemäßen Verfahren aber zunächst auch in z.B. entsprechender Weise wie die wenigstens zwei parallel betriebenen Oxidationsreaktorsysteme parallel ausgeführt sein. Dies ist dann gegebenenfalls vorteilhaft, wenn die Zielproduktabtrennung aus mehreren hintereinandergeschalteten Trennapparaten besteht, von denen nur einer besonders investitionsintensiv oder auf andere Weise kritisch ist. Es kann nun erfindungsgemäß zweckmäßig sein, die Zielproduktabtrennung bis zu dem besonders kostenintensiven (kritischen) Abtrennapparat parallel auszuführen, und erst die entsprechenden Zielprodukt enthaltenden Folgeströme dann zu einem Gemischstrom zu vereinigen, um diesen Gemischstrom anschließend nur noch einer kritischen Trennvorrichtung zuzuführen. Hinter dieser Trennvorrichtung wird die Parallelausführung der gegebenenfalls weiterführenden Zielproduktabtrennung vorzugsweise in entsprechender Weise aufgehoben sein.

Andererseits kann die Zielproduktabtrennung beim erfindungsgemäßen Verfahren aber auch bis zum Anfallen der Roh-Zielproduktströme parallel ausgeführt sein. In diesem Fall werden erfindungsgemäß normalerweise Roh-Zielproduktströme erhalten, die unterschiedliche Verunreinigungsgehalte aufweisen. Während einer von beiden die vom Markt geforderte Verunreinigungsspezifikation gegebenenfalls übererfüllt, erfüllt sie der andere Roh-Zielproduktstrom eventuell nicht. Mischt man beide Roh-Zielproduktströme ab, kann ein Roh-Zielproduktstrom erhalten werden, der in seiner Gesamtmenge spezifikationsgerecht ist.

Werden drei Oxidationsreaktorsysteme in erfindungsgemäßer Weise parallel betrieben, können z.B. anstelle die Produktgas(gemisch)ströme von allen drei Systemen zu vereinigen und das resultierende Gemisch aufzuarbeiten, auch nur zwei der drei Produktgas(gemisch)ströme vereinigt und im Gemisch aufgearbeitet werden. Die Aufarbeitung des Dritten Produktgasstroms kann separat erfolgen und anschließend könne die beiden anfallenden Roh-Zielproduktströme gemischt werden u.s.w..

Eine unterschiedliche Betriebsdauer der Katalysatorbeschickungen von wenigstens zwei parallel betriebenen Oxidationsreaktorsystemen lässt sich aber auch dadurch einstellen (z.B. auch durch entsprechende zeitversetzte Inbetriebnahme der frischen Katalysatorbeschickungen), dass man die Katalysatorbeschickungen stetig oder während eines terminierten Zeitraums bei unterschiedlichen Temperaturen und/oder unterschiedlichen Belastungen mit Vorläuferverbindung betreibt. D.h., das erfindungsgemäß relevante Maß für die Betriebsdauer einer Katalysatorbeschickung ist nur im Fall identischer Betriebsbedingungen und identischem Katalysatorbeschickungstyp die Zeit im chronometrischen Sinne. Ansonsten ist es die an der Katalysatorbeschickung bereits produzierte Zielproduktmenge. Je mehr Zielprodukt an der Katalysatorbeschickung bereits produziert worden ist, desto größer ist deren gefühltes Alter. Im Fall einer mehrstufigen, über wenigstens ein Zwischenprodukt verlaufenden, heterogen katalysierten Gasphasen-Partialoxidation gilt als Maß für die Betriebsdauer der Katalysatorbeschickung, an welcher die Zwischenproduktbildung erfolgt, in entsprechender Weise die insgesamt an dieser Beschickung bereits produzierte Zwischenproduktmenge.

Im Fall einer zweistufigen Herstellung von Acrylsäure aus Propylen wäre dies für die Katalysatorbeschickung der ersten Reaktionsstufe z.B. die insgesamt an dieser Katalysatorbeschickung bereits gebildete Acroleinmenge.

Im Fall einer zweistufigen Herstellung von Methacrylsäure aus iso-Buten wäre dies für die Katalysatorbeschickung der ersten Reaktionsstufe z.B. die insgesamt an dieser Katalysatorbeschickung bereits gebildete Menge an Methacrolein. Für die Katalysatorbeschickungen der zweiten Reaktionsstufe wäre das entsprechende Maß für die Betriebslänge die an der jeweiligen Katalysatorbeschickung bereits gebildete Acrylsäure- bzw. Methacrylsäuremenge.

D.h., von der erfindungsgemäßen Verfahrensweise macht im Fall einer in wenigstens zwei parallel betriebenen Oxidationsreaktorsystemen durchgeführten mehrstufigen heterogen katalysierten Gasphasen-Partialoxidation auch derjenige bereits Gebrauch, der nach einer gewissen Betriebsdauer nur in einer einzigen Oxidationsstufe des relevanten Oxidationsreaktorsystems wenigstens einen Teil der Katalysatorbeschickung (vorzugsweise wenigstens 20 Gew.-%, besser wenigstens 40 Gew.-%, noch besser wenigstens 60 Gew.-%, oder wenigstens 80 Gew.-% und am besten 100 Gew.-% bezogen auf die Katalysatorbeschickung) durch Frischkatalysator ersetzt und nachfolgend erfindungsgemäß weiterverfährt. Im Fall einer zweistufigen Herstellung von Acrylsäure aus Propylen kann dieser Teil- oder Vollkatalysatorwechsel z.B. nur in der Reaktionsstufe "Propylen → Acrolein" in einem der relevanten wenigstens zwei parallel betriebenen Oxidationsreaktorsysteme vorgenommen werden. Natürlich kann er aber auch in beiden Reaktionsstufen in einem der relevanten wenigstens zwei parallel betriebenen Oxidationsreaktorsysteme vorgenommen werden. Erfindungsgemäß ist aber auch vorstellbar, dass man den Teil- und Vollkatalysatorwechsel in dem einen der relevanten wenigstens zwei parallel betriebenen Oxidationsreaktorsysteme nur in der ersten Reaktionsstufe und in dem anderen der relevanten wenigstens zwei parallel betriebenen Oxidationsreaktorsysteme nur in der zweiten Reaktionsstufe vornimmt.

Wächst die Selektivität der Zielproduktbildung mit zunehmender Betriebsdauer der Katalysatorbeschickung und nimmt mit zunehmender Betriebsdauer nur deren Aktivität ab, kann in entsprechender Weise vorgegangen werden.

So kann man die relevanten wenigstens zwei parallel betriebenen Oxidationsreaktorsysteme mit jeweils frischen Katalysatorbeschickungen zunächst für einige Zeit unter identischen Bedingungen betreiben und den Gemischstrom der wenigstens zwei Produktgasströme einem einzigen Abtrennsystem zuführen. Letzteres kann so ausgelegt sein, dass es auf der Grundlage der anfänglich hohen Selektivität der Nebenproduktbildung zunächst einen Roh-Zielproduktstrom mit vergleichsweise geringer Reinheit erzeugt. Beispielsweise kann dieser Roh-Zielproduktstrom eine Roh-Acrylsäure sein, die lediglich zur Herstellung von Alkylestern (beispielsweise Butyl-, Methyl-, Ethyl- oder 2-Ethylhexylester) weiterverwendbar ist. Führt man anschließend im erfindungsgemäßen Sinn einen Teil- oder Vollkatalysatorwechsel durch, wird im weiteren Verlauf des erfindungsgemäßen Betriebs eine mittlere Zielproduktselektivität im Gemisch der relevanten wenigstens zwei Produktgasströme erzielt, die im selben Abtrennsystem zu einem Roh-Zielproduktstrom mit vergleichsweise erhöhter Reinheit führt. Beispielsweise kann dieser Roh-Zielproduktstrom dann eine "Rein"-Acrylsäure sein, die zur Herstellung von superabsorbierenden Polyacrylsäuren bzw. deren Natriumsalzen geeignet ist.

Selbstverständlich kann man auch zunächst produzierte weniger spezifikationsgerechte Roh-Acrylsäure in einem großen Tank lagern und mit nachfolgender, die gewünschte Spezifikation übererfüllender Roh-Acrylsäure zu einer spezifikationsgerechten Roh-Acrylsäure-Gesamtmenge mischen.

Vom erfindungsgemäßen Verfahren wird aber auch dann Gebrauch gemacht, wenn die relevanten wenigstens zwei parallel betriebenen Oxidationsreaktorsysteme mit Katalysatorbeschickungen beschickt sind, von denen die eine die Zielverbindung mit über die Betriebsdauer zunehmender Selektivität und die andere die Zielverbindung mit über die Betriebsdauer abnehmender Selektivität bildet. Auf diese Weise sind in den wenigstens zwei Katalysatorbeschickungen bereits nach kurzer chronometischer Betriebsdauer unterschiedliche Zielproduktmengen gebildet worden und damit im erfindungsgemäßen Sinn "unterschiedliche" Betriebsdauern der beiden Katalysatorbeschickungen erreicht.

Im Fall einer erfindungsgemäßen mehrstufigen heterogen katalysierten Gasphasen-Partialoxidation wenigstens einer organischen Vorläuferverbindung ist es erfindungsgemäß vorteilhaft, bereits die Produktgasströme der relevanten wenigstens zwei parallel betriebenen ersten Reaktionsstufen zu einem Gemischstrom zu vereinen (wie es bereits in der klassischen Parallelschaltung der DE-A 199 02 562 praktiziert wird) und mit diesem, gegebenenfalls durch Inertgas und/oder molekularen Sauerstoff ergänzt, die wenigstens zwei nachfolgenden Reaktionsstufen parallel zu beschicken und betreiben.

Grundsätzlich eignet sich das erfindungsgemäße Verfahren für alle eingangs dieser Schrift spezifisch aufgeführten heterogen katalysierten Gasphasenpartialoxidationen. Dazu gehört insbesondere auch die in den Schriften WO 01/96270, DE-A 103 16 465, DE-A 102 45 585 und DE-A 102 46 119 beispielhaft beschriebene heterogen katalysierte Gasphasenpartialoxidation von Propan zu Acrylsäure. Die vorgenannten Schriften sollen ebenso wie die US-A 2004/0242826 und die DE-A 103 36 386 als integraler Bestandteil dieser Schrift betrachtet werden. Prinzipiell eignet sich die erfindungsgemäße Verfahrensweise auch für im Katalysator-Wirbelbett durchgeführte heterogen katalysierte Partialoxidationen.

Erfindungsgemäß vorteilhaft kann man die erfindungsgemäße heterogen katalysierte Gasphasen-Partialoxidation wenigstens einer organischen Vorläuferverbindung mit molekularem Sauerstoff auch in wenigstens zwei parallel betriebenen Rohrbündelreaktoren erfindungsgemäß durchführen, von denen der eine über den Reaktor betrachtet im Gleichstrom zum Reaktionsgasgemisch und der andere über den Reaktor betrachtet im Gegenstrom zum Reaktionsgasgemisch betrieben wird. Letzteres führt in der Regel zu einer beschleunigten Alterung der Katalysatorbeschickung, wenn abgesehen von der Richtungsfahrweise unter ansonsten identischen Bedingungen betrieben wird.

Erfindungsgemäß vorteilhaft sollte beim erfindungsgemäßen Verfahren wenigstens eine der relevanten Katalysatorbeschickungen der wenigstens zwei parallel betriebenen Oxidationsreaktorsysteme wenigstens eine Katalysatorteilmenge (im Fall einer mehrstufigen Partialoxidation z.B. die Katalysatorgesamtmenge der ersten und/oder der zweiten Reaktionsstufe, oder Teilmengen der Katalysatorbeschickungen der ersten und/oder zweiten Reaktionsstufe) enthalten, an der die heterogen katalysierte Gas-Partialoxidation bereits wenigstens 30 Kalendertage, vorzugsweise wenigstens 60 Kalendertage oder wenigstens 90 Kalendertage und besonders bevorzugt wenigstens 120 bzw. wenigstens 150 Kalendertage sowie ganz besonders bevorzugt wenigstens 180 Kalendertage oder wenigstens 210 bzw. 240 Kalendertage länger durchgeführt wird als an allen Katalysatorteilmengen der anderen Katalysatorbeschickung. Erfindungsgemäße Verfahren sind aber auch solche, bei denen der vorgenannte Betriebsdauerunterschied wenigstens 270, oder wenigstens 300, oder wenigstens 330, oder wenigstens 360, oder wenigstens 400, oder wenigstens 450, oder wenigstens 500, oder wenigstens 550, oder wenigstens 600, oder wenigstens 700, oder wenigstens 800, oder wenigstens 900, oder wenigstens 1000 Tage, oder wenigstens 2000 Tage, oder wenigstens 3000 Tage oder mehr beträgt. Im Regelfall wird er nicht mehr als drei Jahre oder 1000 Tage, meist nicht mehr als zwei Jahre oder 750 Tage betragen.

Ausgedrückt in an dieser wenigstens einen Katalysatorteilmenge insgesamt bereits mehr produzierten Gesamtmenge an Zielprodukt bzw. Zwischenprodukt kann der vorgenannte Betriebsdauerunterschied wenigsten 10⁶kg, oder wenigstens 2•10⁶kg, oder wenigstens 3•10⁶kg, oder wenigstens 4•10⁶kg, oder wenigstens 5•10⁶kg, oder wenigstens 6•10⁶kg, oder wenigstens 7•10⁶kg, oder wenigstens 8•10⁶kg, oder wenigstens 9•10⁶ kg oder wenigstens 10⁷kg, oder wenigstens 1,5•10⁷kg, oder wenigsten 2•10⁷kg, oder wenigstens 3• bzw. 4•10⁷kg, oder wenigstens 5•10⁷kg, oder wenigstens 6•10⁷kg, oder wenigstens 7•10⁷kg, oder wenigstens 8•10⁷kg, oder wenigstens 10⁸kg, oder wenigstens 2•10⁸kg, oder wenigstens 3•10⁸kg, oder wenigstens 4•10⁸kg betragen. Normalerweise wird der vorgenannte Betriebsdauerunterschied nicht mehr als 10⁹kg, meist nicht mehr als 0,5•10⁹kg, und häufig nicht mehr als 10⁸kg betragen.

Erfindungsgemäß von Bedeutung ist, dass bereits geringe Änderungen der Selektivität der Nebenproduktbildung die Gewinnung von spezifikationsgerechtem Roh-Zielprodukt signifikant erschweren können. Ein typisches Beispiel ist die Propionsäure als Nebenprodukt von Acrylsäure. Um am Markt verkäuflich zu sein, sollte Acrylsäure nicht mehr als z.B. 800 gew.ppm Propionsäure (je nach Verwendungszweck) enthalten. Ähnliche Grenzwerte gelten im Fall der Acrylsäure z.B. für Formaldehyd und Essigsäure als Nebenprodukt-Verunreinigungen. In vielen Fällen ändert sich die Selektivität der Zielproduktbildung bei heterogen katalysierten Gasphasen-Partialoxidationen innerhalb der ersten drei Monate nach Inbetriebnahme der Katalysatorbeschickung um wenigstens 0,1 oder 0,2 mol-%, oder um wenigstens 0,3 bzw. 0,5 mol-%, bzw. um wenigstens 1 mol-% oder um wenigstens 1,5 mol-%, bzw. um wenigstens 2 mol-%, teilweise sogar um wenigstens 3 oder wenigstens 4 bzw. wenigstens 7 mol%. Die Gesamtselektivität der Nebenkomponentenbildung ändert sich in der Regel im gleichen Zeitraum entsprechend vielfach um ebenfalls wenigstens 0,1 bis 7 mol-% und mehr.

Demgemäß kann beim erfindungsgemäßen Verfahren der Unterschied der relevanten wenigstens zwei parallel betriebenen Katalysatorbeschickungen in der Selektivität der Zielproduktbildung (z.B. Acrylsäurebildung) z.B. bis zu 7 mol-% oder mehr (z.B. 0,1, oder 0,2, oder 0,3 bis 7 mol-%) und der Unterschied in der Gesamtseletkivität der Nebenkomponentenbildung ebenfalls bis zu 7 mol-% oder mehr (z.B. 0,1, oder 0,2, oder 0,3 bis 7 mol-%)betragen.

In besonderer Weise eignet sich die erfindungsgemäße Verfahrensweise jedoch für die vorzugsweise im Rohrbündelreaktor einstufig durchgeführte heterogen katalysierte Festbett-Gasphasenpartialoxidation von Propen zu Acrolein und/oder Acrylsäure und für die erste und zweite Stufe einer in Rohrbündelreaktoren zweistufig durchgeführten heterogen katalysierten Festbett-Gasphasenpartialoxidation von Propen über Acrolein zu Acrylsäure, wie sie z.B. in den Schriften EP-A 700 893, EP-A 700 714, DE-A 199 10 508, DE-A 199 10 506, DE-A 103 51 269, DE-A 103 50 812, DE-A 103 50 822, EP-A 11 59 247, DE-A 103 13 208, DE-A 10 2004 021 764, DE-A 199 48 248, EP-A 990 636, EP-A 11 06 598, DE-A 30 02 829 und DE-A 102 32 482 beschrieben sind.

Das erfindungsgemäße Verfahren eignet sich für eine heterogen katalysierte Gasphasenfestbettpartialoxidation von Propen zu Acrolein insbesondere dann, wenn als Katalysatoren solche verwendet werden, deren Aktivmasse ein Multielementoxid, das die Elemente Molybdän und/oder Wolfram sowie wenigstens eines der Elemente Wismut, Tellur, Antimon, Zinn und Kupfer enthält oder ein die Elemente Mo, Bi und Fe enthaltendes Multimetalloxid ist. Erfindungsgemäß besonders geeignete Mo, Bi und Fe enthaltende Multimetalloxidmassen der vorgenannten Art sind vor allem die in der DE-A 10 34 4149 und in der DE-A 10 34 4264 offenbarten Mo, Bi und Fe enthaltenden Multimetalloxidmassen. Dies sind insbesondere auch die Multimetalloxidaktivmassen der allgemeinen Formel I in der DE-A 19 95 5176, die Multimetalloxidaktivmassen der allgemeinen Formel I der DE-A 19 94 8523, die Multimetalloxidaktivmassen der allgemeinen Formeln I, II und III der DE-A 10 10 1695, die Multimetalloxidaktivmassen der allgemeinen Formeln I, II und III der DE-A 19 94 8248 und die Multimetalloxidaktivmassen der allgemeinen Formeln I, II und III der DE-A 19 95 5168 sowie die in der EP-A 700 714 genannten Multimetalloxidaktivmassen.

Ferner eignet sich eine Anwendung des erfindungsgemäßen Verfahrens, wenn man für die erfindungsgemäß zu verwendenden wenigstens zwei Katalysatorfestbetten im Fall der Partialoxidation von Propen zu Acrolein die Mo, Bi und Fe enthaltenden Multimetalloxidkatalysatoren, die in den Schriften DE-A 10 04 6957, DE-A 10 06 3162, DE-C 33 38 380, DE-A 19 90 2562, EP-A 15 565, DE-C 23 80 765, EP-A 807 465, EP-A 279 374, DE-A 33 00 044, EP-A 575 897, US-A 4 438 217, DE-A 19 85 5913, WO 98/24746, DE-A 19 74 6210 (diejenigen der allgemeinen Formel II), JP-A 91/294 239, EP-A 293 224 und EP-A 700 714 offenbart werden, einsetzt. Dies gilt insbesondere für die beispielhaften Ausführungsformen in diesen Schriften, unter denen jene der EP-A 15565, der EP-A 575 897, der DE-A 19 74 6210 und der DE-A 19 85 5913 besonders bevorzugt werden. Besonders hervorzuheben sind in diesem Zusammenhang ein Katalysator gemäß Beispiel 1 c aus der EP-A 15 565 sowie ein in entsprechender Weise herzustellender Katalysator, dessen Aktivmasse jedoch die Zusammensetzung Mo₁₂Ni_{6,5}Zn₂Fe₂Bi₁P_{0,0065}K_{0,06}Ox • 10 SiO₂ aufweist. Ferner sind hervorzuheben das Beispiel mit der laufenden Nr. 3 aus der DE-A 19855913 (Stöchiometrie: Mo₁₂Co₇Fe₃Bi_{0,6}K_{0,08}Si_{1,6}Ox) als Hohlzylindervollkatalysator der Geometrie 5 mm x 3 mm x 2 mm bzw. 5 mm x 2 mm x 2 mm (jeweils Außendurchmesser x Höhe x Innendurchmesser) sowie der Multimetalloxid II - Vollkatalysator gemäß Beispiel 1 der DE-A 19 74 6210. Ferner wären die Multimetalloxid-Katalysatoren der US-A 4438217 zu nennen. Letzteres gilt insbesondere dann, wenn diese eine Hohlzylinder-Geometrie der Ausmaße 5,5 mm x 3 mm x 3,5 mm, oder 5 mm x 2 mm x 2 mm, oder 5 mm x 3 mm x 2 mm, oder 6 mm x 3 mm x 3 mm, oder 7 mm x 3 mm x 4 mm (jeweils Außendurchmesser x Höhe x Innendurchmesser) aufweisen. Ebenso eignen sich im erfindungsgemäße Sinne die Multimetalloxidkatalysatoren und Geometrien der DE-A 10 10 1695 bzw. WO 02/062737.

Weiterhin sind das Beispiel 1 aus der DE-A 10046957 (Stöchiometrie: [Bi₂W₂O₉ x 2WO₃]_{0,5} •[Mo₁₂Co_{5,6}Fe_{2,94}S_{i1,59}K_{0,08}Oₓ]₁) als Hohlzylinder (Ring) vollkatalysator der Geometrie 5 mm x 3 mm x 2 mm bzw. 5 mm x 2 mm x 2 mm (jeweils Außendurchmesser x Länge x Innendurchmesser), sowie die Schalenkatalysatoren 1, 2 und 3 aus der DE-A 10063162 (Stöchiometrie: Mo₁₂zBi_{1,0}Fe₃Co₇Si_{1,6}K_{0,08}), jedoch als ringförmige Schalenkatalysatoren entsprechender Schalendicke und auf Trägerringe der Geometrie 5 mm x 3 mm x 1,5 mm bzw. 7 mm x 3 mm x 1,5 mm (jeweils Außendurchmesser x Länge x Innendurchmesser) aufgebracht, im erfindungsgemäßen Sinn gut geeignet.

Eine Vielzahl von für die Katalysatoren einer Propenpartialoxidation zu Acrolein im erfindungsgemäßen Sinn besonders geeigneten Multimetalloxidaktivmassen lässt sich unter der allgemeinen Formel I,

Mo₁₂BiₐFe_{b}X¹_{c}X²_{d}X³ₑX⁴_{f}Oₙ (I),

in der die Variablen nachfolgende Bedeutung aufweisen:
X¹ = Nickel und/oder Kobalt,
X² = Thallium, ein Alkalimetall und/oder ein Erdalkalimetall,
X³ = Zink, Phosphor, Arsen, Bor, Antimon, Zinn, Cer, Blei und/oder Wolfram,
X⁴ = Silicium, Aluminium, Titan und/oder Zirkonium,

a = 0,5 bis 5,
b = 0,01 bis 5, vorzugsweise 2 bis 4,
c = 0 bis 10, vorzugsweise 3 bis 10,
d = 0 bis 2, vorzugsweise 0,02 bis 2,
e = 0 bis 8, vorzugsweise 0 bis 5,
f = 0 bis 10 und
n = eine Zahl, die durch die Wertigkeit und Häufigkeit der von Sauerstoff verschiedenen Elemente in I bestimmt wird,
subsumieren.

Sie sind in an sich bekannter Weise erhältlich (siehe z.B. die DE-A 4023239) und werden üblicherweise in Substanz zu Kugeln, Ringen oder Zylindern geformt oder auch in Gestalt von Schalenkatalysatoren, d.h., mit der Aktivmasse beschichteten vorgeformten, inerten Trägerkörpern, eingesetzt. Selbstverständlich können sie aber auch in Pulverform als Katalysatoren angewendet werden.

Prinzipiell können Aktivmassen der allgemeinen Formel I in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 650°C calciniert. Die Calcination kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemisch aus Inertgas, NH₃, CO und/oder H₂) erfolgen. Die Calcinationsdauer kann einige Minuten bis einige Stunden betragen und nimmt üblicherweise mit der Temperatur ab. Als Quellen für die elementaren Konstituenten der Multimetalloxidaktivmassen I kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Neben den Oxiden kommen als solche Ausgangsverbindungen vor allem Halogenide, Nitrate, Formiate, Oxalate, Citrate, Acetate, Carbonate, Aminkomplexe, Ammonium-Salze und/oder Hydroxide in Betracht (Verbindungen wie NH₄OH, (NH₄)₂CO₃, NH₄NO₃, NH₄CHO₂, CH₃COOH, NH₄CH₃CO₂ und/oder Ammoniumoxalat, die spätestens beim späteren Calcinieren zu gasförmig entweichenden Verbindungen zerfallen und/oder zersetzt werden können, können in das innige Trockengemisch zusätzlich eingearbeitet werden).

Das innige Vermischen der Ausgangsverbindungen zur Herstellung von Multimetalloxidaktivmassen I kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form. Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperaturen aus dem Sprühturm von 100 bis 150°C erfolgt.

Üblicherweise werden die Multimetalloxidaktivmassen der allgemeinen Formel I im Katalysatorfestbett nicht in Pulverform sondern zu bestimmten Katalysatorgeometrien geformt eingesetzt, wobei die Formgebung vor oder nach der abschließenden Calcination erfolgen kann. Beispielsweise können aus der Pulverform der Aktivmasse oder ihrer uncalcinierten und/oder partiell calcinierten Vorläufermasse durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Eine besonders günstige Hohlzylindergeometrie beträgt 5 mm x 3 mm x 2 mm (Außendurchmesser x Länge x Innendurchmesser), insbesondere im Fall von Volikatalysatoren.

Selbstverständlich kann die Formgebung der pulverförmigen Aktivmasse oder ihrer pulverförmigen, noch nicht und/oder partiell calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z.B. aus der DE-A 2909671, der EP-A 293859 oder aus der EP-A 714700 bekannt ist. Zweckmäßigerweise wird zur Beschichtung der Trägerkörper die aufzubringende Pulvermasse befeuchtet und nach dem Aufbringen, z.B. mittels heißer Luft, wieder getrocknet. Die Schichtdicke der auf den Trägerkörper aufgebrachten Pulvermasse wird zweckmäßigerweise im Bereich 10 bis 1000 µm, bevorzugt im Bereich 50 bis 500 µm und besonders bevorzugt im Bereich 150 bis 250 µm liegend, gewählt. Alternativ kann die aufzubringende Pulvermasse auch unmittelbar aus ihrer Suspension oder Lösung heraus (z.B. in Wasser) auf die Trägerkörper aufgebracht werden.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Sie verhalten sich bezüglich der dem erfindungsgemäßen Verfahren in der ersten Reaktionsstufe unterliegenden Zielreaktion in der Regel im wesentlichen inert. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Geeignet ist die Verwendung von im wesentlichen unporösen, oberflächenrauhen, kugelförmigen Trägern aus Steatit (z.B. Steatit C220 der Fa. CeramTec), deren Durchmesser 1 bis 8 mm, bevorzugt 4 bis 5 mm beträgt. Geeignet ist aber auch die Verwendung von Zylindern als Trägerkörper, deren Länge 2 bis 10 mm (z.B. 8 mm) und deren Außendurchmesser 4 bis 10 mm (z.B. 6 mm) beträgt. Im Fall von erfindungsgemäß geeigneten Ringen als Trägerkörper liegt die Wanddicke darüber hinaus üblicherweise bei 1 bis 4 mm. Erfindungsgemäß bevorzugt zu verwendende ringförmige Trägerkörper besitzen eine Länge von 2 bis 6 mm, einen Außendurchmesser von 4 bis 8 mm und eine Wanddicke von 1 bis 2 mm. Erfindungsgemäß geeignet sind vor allem auch Ringe der Geometrie 7 mm x 3 mm x 4 mm bzw. 5 mm x 3 mm x 2 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Für die Katalysatoren des Katalysatorfestbetts einer Propenpartialoxidation zu Acrolein im erfindungsgemäßen Sinn besonders geeignete Multimetalloxidaktivmassen sind ferner Massen der allgemeinen Formel II,

[Y¹_{a'}Y²_{b'}O_{x'}]ₚ[Y³_{c'}Y⁴_{d'}Y⁵_{e'}Y⁶_{e'}Y⁷_{g'}Y²_{h'}O_{y'}]_{q} (II),

in der die Variablen folgende Bedeutung haben:
- Y¹ =: nur Wismut oder Wismut und wenigstens eines der Elemente Tellur, Antimon, Zinn und Kupfer,
- Y² =: Molybdän oder Molybdän und Wolfram,
- Y³ =: ein Alkalimetall, Thallium und/oder Samarium,
- Y⁴ =: ein Erdalkalimetall, Nickel, Kobalt, Kupfer, Mangan, Zink, Zinn, Cadmium und/oder Quecksilber,
- Y⁵ =: Eisen oder Eisen und wenigstens eines der Elemente Chrom und Cer,
- Y⁶ =: Phosphor, Arsen, Bor und/oder Antimon,
- Y⁷ =: ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,

a' = 0,01 bis 8,
b' = 0,1 bis 30,
c' = 0 bis 4,
d' = 0 bis 20,
e' > 0 bis 20,
f' = 0 bis 6,
g' = 0 bis 15,
h' = 8 bis 16,
x',y'= Zahlen, die durch die Wertigkeit und Häufigkeit der von Sauerstoff verschiedenen Elemente in II bestimmt werden und
p,q = Zahlen, deren Verhältnis p/q 0,1 bis 10 beträgt,
enthaltend dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung Y¹_{a'}Y²_{b'}OX_{x'}, deren Größtdurchmesser (längste durch den Schwerpunkt des Bereichs gehende direkte Verbindungsstrecke zweier auf der Oberfläche (Grenzfläche) des Bereichs befindlicher Punkte) 1 nm bis 100 µm, häufig 10 nm bis 500 nm oder 1 µm bis 50 bzw. 25 µm, beträgt.

Besonders vorteilhafte Multimetalloxidmassen II sind solche, in denen Y¹ nur Wismut ist.

Unter diesen werden wiederum jene bevorzugt, die der allgemeinen Formel III,

[Bi_{a"}Z²_{b"}O_{x"}]_{p"} [Z²₁₂Z³_{c"}Z⁴_{d"}F^{e}_{e"}Z⁵_{f"}Z⁶_{g"}Z⁷_{h"}O_{y"}]_{q"} (III),

in der die Variablen folgende Bedeutung haben:
Z² = Molybdän oder Molybdän und Wolfram,
Z³ = Nickel und/oder Kobalt,
Z⁴ = Thallium, ein Alkalimetall und/oder ein Erdalkalimetall,
Z⁵ = Phosphor, Arsen, Bor, Antimon, Zinn, Cer und/oder Blei,
Z⁶ = Silicium, Aluminium, Titan und/oder Zirkonium,
Z⁷ = Kupfer, Silber und/oder Gold,

a" = 0,1 bis 1,
b" = 0,2 bis 2,
c" = 3 bis 10,
d" = 0,02 bis 2,
e" = 0,01 bis 5, vorzugsweise 0,1 bis 3,
f" = 0 bis 5,
g" = 0 bis 10,
h" = 0 bis 1,
x",y"= Zahlen, die durch die Wertigkeit und Häufigkeit der von Sauerstoff verschiedenen Element in III bestimmt werden,
p",q"=Zahlen, deren Verhältnis p"/q" 0,1 bis 5, vorzugsweise 0,5 bis 2 beträgt,
entsprechen, wobei diejenigen Massen III ganz besonders bevorzugt werden, in denen Z²_{b"} = (Wolfram)_{b"} und Z²₁₂ = (Molybdän)₁₂ ist.

Ferner ist es von Vorteil, wenn wenigstens 25 mol-% (bevorzugt wenigstens 50 mol-% und besonders bevorzugt wenigstens 100 mol-%) des gesamten Anteils [Y¹_{a'}Y²_{b'}O_{x'}]ₚ ([Bi_{a"}Z²_{b"}O_{x"}]_{p"}) der erfindungsgemäß geeigneten Multimetalloxidmassen II (Multimetalloxidmassen III) in den erfindungsgemäß geeigneten Multimetalloxidmassen II (Multimetalloxidmassen III) in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung Y¹_{a'}Y²_{b'}O_{x'} [Bi_{a"}Z²_{b"}O_{x"}) vorliegen, deren Größtdurchmesser im Bereich 1 nm bis 100 µm liegt.

Hinsichtlich der Formgebung gilt bezüglich Multimetalloxidmassen II-Katalysatoren das bei den Multimetalloxidmassen I-Katalysatoren Gesagte.

Die Herstellung von Multimetalloxidmassen II-Aktivmassen ist z.B. in der EP-A 575897 sowie in der DE-A 19855913, der DE-A 10344149 und der DE-A 10344264 beschrieben.

Als Aktivmasse für Katalysatoren wenigstens eines für die Partialoxidation von Acrolein zu Acrylsäure geeigneten Katalysatorfestbetts eignen sich im erfindungsgemäßen Sinn die für diesen Reaktionstyp bekannten Multimetalloxide, die die Elemente Mo und V enthalten.

Solche Mo und V enthaltende Multimetalloxidaktivmassen können beispielsweise der US-A 3775474, der US-A 3954855, der US-A 3893951 und der US-A 4339355 oder der EP-A 614872 bzw. EP-A 1041062 oder der WO 03/055835 bzw. WO 03/057653 entnommen werden.

Insbesondere eignen sich auch die Multimetalloxidaktivmassen der DE-A 10 32 5487 sowie der DE-A 10 325 488.

Ferner eignen sich für die Partialoxidation von Acrolein zu Acrylsäure im erfindungsgemäßen Sinn in besonderer Weise die Multimetalloxidmassen der EP-A 427508, der DE-A 29 09 671, der DE-C 31 51 805, der DE-AS 26 26 887, der DE-A 43 02 991, der EP-A 700 893, der EP-A 714 700 und der DE-A 19 73 6105 als Aktivmassen für die Festbettkatalysatoren. Besonders bevorzugt sind in diesem Zusammenhang die beispielhaften Ausführungsformen der EP-A 714 700 sowie der DE-A 19 73 6105.

Eine Vielzahl dieser die Elemente Mo und V enthaltenden Multimetalloxidaktivmassen lässt sich unter der allgemeinen Formel IV,

Mo-₁₂VₐX¹_{b}X²_{c}X³_{d}X⁴ₑX⁵_{f}X⁶_{g}Oₙ (IV),

in der die Variablen folgende Bedeutung haben:
X¹ = W, Nb, Ta, Cr und/oder Ce,
X² = Cu, Ni, Co, Fe, Mn und/oder Zn,
X³ = Sb und/oder Bi,
X⁴ = eines oder mehrere Alkalimetalle,
X⁵ = eines oder mehrere Erdalkalimetalle,
X⁶ = Si, Al, Ti und/oder Zr,
a = 1 bis 6,
b = 0,2 bis 4,
c = 0,5 bis 18,
d = 0 bis 40,
e = 0 bis 2,
f = 0 bis 4,
g = 0 bis 40 und
n = eine Zahl, die durch die Wertigkeit und Häufigkeit der von Sauerstoff verschiedenen Elemente in IV bestimmt wird,
subsumieren.

Bevorzugte Ausführungsformen innerhalb der aktiven Multimetalloxide IV sind im erfindungsgemäßen Sinn jene, die von nachfolgenden Bedeutungen der Variablen der allgemeinen Formel IV erfasst werden:
X¹ = W, Nb, und/oder Cr,
X² = Cu, Ni, Co, und/oder Fe,
X³ = Sb,
X⁴ = Na und/oder K,
X⁵ = Ca, Sr und/oder Ba,
X⁶ = Si, Al, und/oder Ti,
a = 1,5 bis 5,
b = 0,5 bis 2,
c = 0,5 bis 3,
d = 0 bis 2,
e = 0 bis 0,2,
f = 0 bis 1 und
n = eine Zahl, die durch die Wertigkeit und Häufigkeit der von Sauerstoff verschiedenen Elemente in IV bestimmt wird.

Ganz besonders bevorzugte Multimetalloxide IV sind im erfindungsgemäßen Sinn jedoch jene der allgemeinen Formel V,

Mo₁₂V_{a'}Y¹_{b'}Y²_{c'}Y⁵_{f'}Y⁶_{g'}Oₙ (V),

mit
Y¹ = W und/oder Nb,
Y² = Cu und/oder Ni,
Y⁵ = Ca und/oder Sr,
Y⁶ = Si und/oder Al,
a' = 2 bis 4,
b' = 1 bis 1,5,
c' = 1 bis 3,
f' = 0 bis 0,5
g' = 0 bis 8 und
n' = eine Zahl, die durch die Wertigkeit und Häufigkeit der von Sauerstoff verschiedenen Elementen in V bestimmt wird.

Multimetalloxidaktivmassen (IV) sind in sich bekannter, z.B. in der DE-A 43 35 973 oder in der EP-A 714 700 offenbarter, Weise erhältlich. Insbesondere eignen sich als Mo und V enthaltende Multimetalloxidaktivmassen im erfindungsgemäßen Sinn für die Partialoxidation von Acrolein zu Acrylsäure aber auch die Multimetalloxidaktivmassen der DE-A 10 261 186.

Prinzipiell können solche Mo und V enthaltenden Multimetalloxidaktivmassen, insbesondere solche der allgemeinen Formel IV, in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 600°C calciniert. Die Calcination kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemische aus Inertgas und reduzierenden Gasen wie H₂, NH₃, CO, Methan und/oder Acrolein oder die genannten reduzierend wirkenden Gase für sich) durchgeführt werden. Die Calcinationsdauer kann einige Minuten bis einige Stunden betragen und nimmt üblicherweise mit der Temperatur ab. Als Quellen für die elementaren Konstituenten der Multimetalloxidaktivmassen IV kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Das innige Vermischen der Ausgangsverbindungen zur Herstellung von Multimetalloxidmassen IV kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form.

Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperaturen von 100 bis 150°C erfolgt.

Die Mo und V enthaltenden Multimetalloxidaktivmassen, insbesondere jene der allgemeinen Formel IV, können für das erfindungsgemäße Verfahren einer Partialoxidation von Acrolein zu Acrylsäure sowohl in Pulverform als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten Vorläufermasse durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Selbstverständlich kann die Formgebung der pulverförmigen Aktivmasse oder ihrer pulverförmigen, noch nicht calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z.B. aus der DE-A 2909671, der EP-A 293859 oder aus der EP-A 714700 bekannt ist.

Zweckmäßigerweise wird zur Beschichtung der Trägerkörper die aufzubringende Pulvermasse befeuchtet und nach dem Aufbringen, z.B. mittels heißer Luft, wieder getrocknet. Die Schichtdicke der auf den Trägerkörper aufgebrachten Pulvermasse wird zweckmäßigerweise im Bereich 10 bis 1000 µm, bevorzugt im Bereich 50 bis 500 µm und besonders bevorzugt im Bereich 150 bis 250 µm liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Geeignet ist die Verwendung von im wesentlichen unporösen, oberflächenrauhen, kugelförmigen Trägern aus Steatit, deren Durchmesser 1 bis 10 mm (z.B. 8 mm), bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von erfindungsgemäß geeigneten Ringen als Trägerkörper liegt die Wanddicke darüber hinaus üblicherweise bei 1 bis 4 mm. Erfindungsgemäß bevorzugt zu verwendende ringförmige Trägerkörper besitzen eine Länge von 3 bis 6 mm, einen Außendurchmesser von 4 bis 8 mm und eine Wanddicke von 1 bis 2 mm. Erfindungsgemäß geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Günstige Mo und V enthaltende, im erfindungsgemäßen Sinn für eine Acroleinpartialoxidation zu Acrylsäure zu verwendende Multimetalloxidaktivmassen sind ferner Massen der allgemeinen Formel VI,

[D]ₚ[E]_{q} (VI),

in der die Variablen folgende Bedeutung haben:
D = Mo₁₂V_{a"}Z¹_{b"}Z²_{c"}Z³_{d"}Z⁴_{e"}Z⁵_{f"}Z⁶_{g"}O_{x"},
E = Z⁷₁₂Cu_{h"}H_{i"}O_{y"},
Z¹ = W, Nb, Ta, Cr und/oder Ce,
Z² = Cu, Ni, Co, Fe, Mn und/oder Zn,
Z³ = Sb und/oder Bi,
Z⁴ = Li, Na, K, Rb, Cs und/oder H
Z⁵ = Mg, Ca, Sr und/oder Ba,
Z⁶ = Si, Al, Ti und/oder Zr,
Z⁷ = Mo, W, V, Nb und/oder Ta,

a" = 1 bis 8,
b" = 0,2 bis 5,
c" = 0 bis 23,
d" = 0 bis 50,
e" = 0 bis 2,
f" = 0 bis 5,
g" = 0 bis 50,
h" = 4 bis 30,
i" = 0 bis 20 und
x",y" = Zahlen, die durch die Wertigkeit und Häufigkeit der von Sauerstoff verschiedenen Element in VI bestimmt werden und
p,q = von Null verschiedene Zahlen, deren Verhältnis p/q 160:1 bis 1:1 beträgt,
und die dadurch erhältlich sind, dass man eine Multimetalloxidmasse E,

Z⁷₁₂Cu_{h"}H_{i"}O_{y"} (E),

in feinteiliger Form getrennt vorbildet (Ausgangsmasse 1) und anschließend die vorgebildete feste Ausgangsmasse 1 in eine wässrige Lösung, eine wässrige Suspension oder in ein feinteiliges Trockengemisch von Quellen der Elemente Mo, V, Z¹, Z², Z³, Z⁴, Z⁵, Z⁶, die die vorgenannten Elemente in der Stöchiometrie D,

Mo₁₂V_{a"}Z¹_{b"}Z²_{c"}Z³_{d"}Z⁴_{e"}Z⁵_{f"}Z⁶_{g"} (D),

enthält (Ausgangsmasse 2), im gewünschten Mengenverhältnis p:q einarbeitet, die dabei gegebenenfalls resultierende wässrige Mischung trocknet, und die so gegebene trockene Vorläufermasse vor oder nach ihrer Trocknung zur gewünschten Katalysatorgeometrie bei Temperaturen von 250 bis 600°C calciniert.

Bevorzugt sind dabei jene Multimetalloxidaktivmassen VI, bei denen die Einarbeitung der vorgebildeten festen Ausgangsmasse 1 in eine wässrige Ausgangsmasse 2 bei einer Temperatur < 70°C erfolgt. Eine detaillierte Beschreibung der Herstellung von Multimetalloxidmassen III-Katalysatoren enthalten z.B. die EP-A 668104, die DE-A 19736105 und die DE-A 19528646.

Hinsichtlich der Formgebung gilt bezüglich Multimetalloxidaktivmassen VI-Katalysatoren das bei den Multimetalloxidaktivmassen IV-Katalysatoren Gesagte.

Weitere im beschriebenen Sinn günstige Mo und V enthaltende Multimetalloxidaktivmassen sind ferner Multielementoxidaktivmassen der allgemeinen Formel VII,

[A]ₚ[B]_{q}[C]ᵣ (VII),

in der die Variablen folgende Bedeutung haben:
A = Mo₁₂VₐX¹_{b}X²_{c}X³_{d}X⁴ₑX⁵_{f}X⁶_{g}Oₓ,
B = X₁⁷CuₕHᵢO_{y},
C = X₁⁸SbⱼHₖO_{z},
X¹ = W, Nb, Ta, Cr und/oder Ce, vorzugsweise W, Nb und/oder Cr,
X² = Cu, Ni, Co, Fe, Mn und/oder Zn, vorzugsweise Cu, Ni, Co und/oder Fe,
X³ = Sb und/oder Bi, vorzugsweise Sb,
X⁴ = Li, Na, K, Rb, Cs und/oder H. vorzugsweise Na und/oder K,
X⁵ = Mg, Ca, Sr und/oder Ba, vorzugsweise Ca, Sr und/oder Ba,
X⁶ = Si, Al, Ti und/oder Zr, vorzugsweise Si, Al und/oder Ti,
X⁷ = Mo, W, V, Nb und/oder Ta, vorzugsweise Mo und/oder W,
X⁸ = Cu, Ni, Zn, Co, Fe, Cd, Mn, Mg, Ca, Sr und/oder Ba, vorzugsweise Cu und/oder Zn, besonders bevorzugt Cu,

a = 1 bis 8, vorzugsweise 2 bis 6,
b = 0,2 bis 5, vorzugsweise 0,5 bis 2,5
c = 0 bis 23, vorzugsweise 0 bis 4,
d = 0 bis 50, vorzugsweise 0 bis 3,
e = 0 bis 2, vorzugsweise 0 bis 0,3,
f = 0 bis 5, vorzugsweise 0 bis 2,
g = 0 bis 50, vorzugsweise 0 bis 20,
h = 0,3 bis 2,5, vorzugsweise 0,5 bis 2, besonders bevorzugt 0,75 bis 1,5,
i = 0 bis 2, vorzugsweise 0 bis 1,
j = 0,1 bis 50, vorzugsweise 0,2 bis 20, besonders bevorzugt 0,2 bis 5,
k = 0 bis 50, vorzugsweise 0 bis 20, besonders bevorzugt 0 bis 12,
x,y,z = Zahlen, die durch die Wertigkeit und Häufigkeit der von Sauerstoff verschiedenen Elementen in A, B, C bestimmt werden,
p, q = positive Zahlen
r = 0 oder eine positive Zahl, vorzugsweise eine positive Zahl, wobei das Verhältnis p/(q+r) = 20:1 bis 1:20, vorzugsweise 5:1 bis 1:14 und besonders bevorzugt 2:1 bis 1:8 und , für den Fall, dass r eine positive Zahl ist, das Verhältnis q/r = 20:1 bis 1:20, vorzugsweise 4:1 bis 1:4, besonders bevorzugt 2:1 bis 1:2 und ganz besonders bevorzugt 1:1 beträgt,
die den Anteil [A]ₚ in Form dreidimensional ausgedehnter Bereiche (Phasen) A der chemischen Zusammensetzung
A: Mo₁₂VaX¹_{b}X²_{c}X³_{d}X⁴ₑX⁵_{f}X⁶_{g}Oₓ,
den Anteil [B]q in Form dreidimensional ausgedehnter Bereiche (Phasen) B der chemischen Zusammensetzung
B : X₁⁷CuₕHᵢO_{y} und
den Anteil [C]ᵣ in Form dreidimensional ausgedehnter Bereiche (Phasen) C der chemischen Zusammensetzung
C: X₁⁸SbⱼHₖO_{z}
enthalten, wobei die Bereiche A, B und gegebenenfalls C relativ zueinander wie in einem Gemisch aus feinteiligem A, feinteiligem B und gegebenenfalls feinteiligem C verteilt sind, und wobei alle Variablen innerhalb der vorgegebenen Bereiche mit der Maßgabe auszuwählen sind, dass der molare Anteil des Elements Mo an der Gesamtmenge aller von Sauerstoff verschiedenen Elemente der Multielementoxidaktivmasse VII 20-mol% bis 80 mol-% beträgt, das molare Verhältnis von in der katalytisch aktiven Multielementoxidmasse VII enthaltenem Mo zu in der katalytisch aktiven Multielementoxidmasse VII enthaltenem V, Mo/V, 15:1 bis 1:1 beträgt, das entsprechende molare Verhältnis Mo/Cu 30:1 bis 1:3 und das entsprechende molare Verhältnis Mo/(Gesamtmenge aus W und Nb) 80:1 bis 1:4 beträgt.

Im erfindungsgemäßen Sinn bevorzugte Multielementoxidaktivmassen VII sind solche, deren Bereiche A eine Zusammensetzung im nachfolgenden Stöchiometrieraster der allgemeinen Formel VIII aufweisen,

Mo₁₂VₐX¹_{b}X²_{c}X⁵_{f}X⁶_{g}Oₓ (VIII),

mit
X¹ = W und/oder Nb,
X² = Cu und/oder Ni,
X⁵ = Ca und/oder Sr,
X⁶ = Si und/oder Al,
a = 2 bis 6,
b = 1 bis 2,
c = 1 bis 3,
f = 0 bis 0,75,
g = 0 bis 10, und
x = eine Zahl die durch die Wertigkeit und Häufigkeit der von Sauerstoff verschiedenen Elemente in (VIII) bestimmt wird.

Der im Zusammenhang mit den Multielementoxidaktivmassen VIII verwendete Begriff "Phase" meint dreidimensional ausgedehnte Bereiche, deren chemische Zusammensetzung von der ihrer Umgebung verschieden ist. Die Phasen sind nicht notwendigerweise röntgenographisch homogen. In der Regel bildet die Phase A eine kontinuierliche Phase, in der Partikel der Phase B und gegebenenfalls C dispergiert sind.

Die feinteiligen Phasen B und gegebenenfalls C bestehen mit Vorteil aus Partikeln, deren Größtdurchmesser, d.h., die längste durch den Schwerpunkt der Partikel gehende Verbindungsstrecke zweier auf der Oberfläche der Partikel befindlicher Punkte bis zu 300 µm, vorzugsweise 0,1 bis 200 µm, besonders bevorzugt 0,5 bis 50 µm und ganz besonders bevorzugt 1 bis 30 µm beträgt. Geeignet sind aber auch Partikel mit einem Größtdurchmesser von 10 bis 80 µm oder 75 bis 125 µm.

Prinzipiell können die Phasen A, B und gegebenenfalls C in den Multielementoxidaktivmassen VII amorph und/oder kristallin vorliegen.

Die den Multielementoxidaktivmassen der allgemeinen Formel VII zugrunde liegenden und anschließend zur Wandlung in Aktivmassen thermisch zu behandelnden innigen Trockengemische können z.B. so erhalten werden, wie es in den Schriften WO 02/24327, DE-A 4405514, DE-A 4440891, DE-A 19528646, DE-A 19740493, EP-A 756894, DE-A 19815280, DE-A 19815278, EP-A 774297, DE-A 19815281, EP-A 668104 und DE-A 19736105 beschrieben ist.

Das Grundprinzip der Herstellung von innigen Trockengemischen, die bei thermischer Behandlung zu Multielementoxidaktivmassen der allgemeinen Formel VII führen, besteht darin, wenigstens eine Multielementoxidmasse B (X₁⁷CuₕHᵢO_{y}) als Ausgangsmasse 1 und gegebenenfalls eine oder mehrere Multielementoxidmassen C (X₁⁸SbⱼHₖO_{z}) als Ausgangsmasse 2 entweder voneinander getrennt oder miteinander vergesellschaftet in feinteiliger Form vorzubilden und anschließend die Ausgangsmassen 1 und gegebenenfalls 2 mit einem Gemisch, das Quellen der elementaren Konstituenten der Multielementoxidmasse A

Mo₁₂VₐX_{b}¹X_{c}²X_{d}³Xₑ⁴X_{f}⁵X_{g}⁶Oₓ (A),

in einer der Stöchiometrie A entsprechenden Zusammensetzung enthält, im gewünschten Mengenverhältnis (gemäß der allgemeinen Formel VII) in innigen Kontakt zu bringen und die dabei resultierende innige Mischung gegebenenfalls zu trocknen.

Das innige in Kontakt bringen der Bestandteile der Ausgangsmassen 1 sowie gegebenenfalls 2 mit dem die Quellen der elementaren Konstituenten der Multimetalloxidmasse A enthaltenden Gemisch (Ausgangsmasse 3) kann sowohl trocken als auch nass erfolgen. Im letzteren Fall muss lediglich darauf geachtet werden, dass die vorgebildeten Phasen (Kristallite) B und gegebenenfalls C nicht in Lösung gehen. In wässrigem Medium ist letzteres bei pH-Werten, die nicht zu stark von 7 abweichen und bei nicht zu hohen Temperaturen üblicherweise gewährleistet. Erfolgt das innige in Kontakt bringen nass, wird abschließend normalerweise zum erfindungsgemäß thermisch zu behandelnden innigen Trockengemisch getrocknet (z.B. durch Sprühtrocknen). Im Rahmen eines trockenen Mischens fällt eine solche Trockenmasse automatisch an. Selbstredend können die feinteilig vorgebildeten Phasen B und gegebenenfalls C auch in ein plastisch verformbares Gemisch, das die Quellen der elementaren Konstituenten der Multimetalloxidmasse A enthält, eingearbeitet werden, wie es die DE-A 10046928 empfiehlt. Natürlich kann das innige in Kontakt bringen der Bestandteile der Ausgangsmassen 1 sowie gegebenenfalls 2 mit den Quellen der Multielementoxidmasse A (Ausgangsmasse 3) auch so erfolgen, wie es die DE-A 19815281 beschreibt.

Die thermische Behandlung zum Erhalt der Aktivmasse und die Formgebung kann wie bei den Multimetalloxidaktivmassen IV bis VI beschrieben erfolgen.

Ganz generell können Multimetalloxidaktivmassen IV bis VII-Katalysatoren mit Vorteil gemäß der Lehre der DE-A 103 25 487 bzw. DE-A 103 25 488 hergestellt werden.

Die Ausführung einer Reaktionsstufe (innerhalb einer Oxidationsreaktorstrasse des erfindungsgemäßen Verfahrens) vom Propen zum Acrolein, kann man mit den für das entsprechende Katalysatorfestbett geeignet beschriebenen Katalysatoren in einfachster Weise und anwendungstechnisch zweckmäßig in einem mit den Festbettkatalysatoren beschickten Rohrbündelreaktor, wie er z.B. in der EP-A 700 714 bzw. der DE-A 4 431 949 oder der WO 03/057653, oder der WO 03/055835, oder der WO 03/059857, oder der WO 03/076373 beschrieben ist, realisieren.

D.h., in einfachster Weise befindet sich das Katalysatorfestbett in den einheitlich beschickten Metallrohren eines Rohrbündelreaktors und um die Metallrohre wird ein Temperiermedium (Einzonenfahrweise), in der Regel eine Salzschmelze, geführt. Salzschmelze (Temperiermedium) und Reaktionsgasgemisch können dabei im einfachen Gleich- oder Gegenstrom geführt werden. Das Temperiermedium (die Salzschmelze) kann aber auch über den Reaktor betrachtet mäanderförmig um die Rohrbündel geführt werden, so dass lediglich über den gesamten Reaktor betrachtet ein Gleich- oder Gegenstrom zur Strömungsrichtung des Reaktionsgasgemischs besteht. Der Volumenstrom des Temperiermediums (Wärmeaustauschmittels) wird dabei üblicherweise so bemessen, dass der Temperaturanstieg (bedingt durch die Exothermie der Reaktion) des Wärmeaustauschmittels von der Eintrittstelle in den Reaktor bis zur Austrittstelle aus dem Reaktor 0 bis 10°C, häufig 2 bis 8°C, oft 3 bis 6°C beträgt. Die Eintrittstemperatur des Wärmeaustauschmittels in den Rohrbündelreaktor beträgt in der Regel 250 bis 450°C, häufig 300 bis 400°C bzw. 300 bis 380°C. In diesen Temperaturbereichen bewegen sich dann auch die zugehörigen Reaktionstemperaturen. Als Wärmeaustauschmittel eignen sich insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle. Aber auch ionische Flüssigkeiten sind einsetzbar.

Zweckmäßigerweise wird das Reaktionsgasausgangsgemisch der Beschickung mit Festbettkatalysator auf die gewünschte Reaktionstemperatur vorerwärmt zugeführt. Vorteilhaft erfolgt die Fertigstellung des Reaktionsgasausgangsgemischs beim erfindungsgemäßen Verfahren vorab und nachfolgend werden die wenigstens zwei parallel betriebenen Oxidationsreaktorsysteme über ein entsprechendes Verteilersystem simultan mit diesem Reaktionsgasausgangsgemisch beschickt.

Insbesondere im Fall von angestrebten hohen (z.B. ≥ 130 NI/I•h, oder ≥ 140 NI/I•h, oder ≥ 150 NI/I•h, oder ≥ 160 NI/I•h, in der Regel jedoch ≤ 600 NI/I•h, häufig ≤ 350 NI/I•h) Belastungen des Katalysatorfestbetts mit Propen erfolgt die Durchführung einer Propenpartialoxidation zu Acrolein zweckmäßig in einem Zwei- oder Mehrzonenrohrbündelreaktor (eine Durchführung im Einzonenrohrbündelreaktor ist jedoch ebenfalls möglich). Eine bevorzugte Variante eines für diesen Zweck erfindungsgemäß einsetzbaren Zweizonenrohrbündelreaktors offenbart die DE-C 2830765. Aber auch die in der DE-C 2513405, der US-A 3147084, der DE-A 2201528, der EP-A 383224 und der DE-A 2903582 offenbarten Zweizonenrohrbündelreaktoren sind geeignet. Eine Verfahrensbeschreibung gibt auch die EP-A 1106598.

D.h., in einfacher Weise befindet sich das erfindungsgemäß wenigstens eine zu verwendende Katalysatorfestbett dann in den einheitlich beschickten Metallrohren eines Rohrbündelreaktors und um die Metallrohre werden zwei voneinander im wesentlichen räumlich getrennte Temperiermedien, in der Regel Salzschmelzen, geführt. Der Rohrabschnitt, über den sich das jeweilige Salzbad erstreckt, repräsentiert eine Reaktionszone.

Vorzugsweise umströmt z.B. ein Salzbad A denjenigen Abschnitt der Rohre (die Reaktionszone A), in welchem sich die oxidative Umsetzung des Propens (beim einfachen Durchgang) bis zum Erreichen eines Umsatzwertes im Bereich von 40 bis 80 mol.% vollzieht und ein Salzbad B umströmt den Abschnitt der Rohre (die Reaktionszone B), in welchem sich die oxidative Anschlußumsetzung des Propens (beim einfachen Durchgang) bis zum Erreichen eines Umsatzwertes von in der Regel wenigstens 93 mol.% vollzieht (bei Bedarf können sich an die Reaktionszonen A, B weitere Reaktionszonen anschließen, die auf individuellen Temperaturen gehalten werden).

Innerhalb der jeweiligen Temperaturzone kann das Salzbad prinzipiell wie bei der Einzonenfahrweise geführt werden. Die Eintrittstemperatur des Salzbades B liegt normalerweise wenigstens 5 bis 10°C oberhalb der Temperatur des Salzbades A. Im übrigen können die Eintrittstemperaturen im für die Einzonenfahrweise empfohlenen Temperaturbereich für die Eintrittstemperatur liegen.

Ansonsten kann die Zwei-Zonen-Hochlastfahrweise der Propenpartialoxidation zu Acrolein z.B. wie in der DE-A 10 30 8836, der EP-A 11 06 598, oder wie in der WO 01/36364, oder der DE-A 19 92 7624, oder der DE-A 19 94 8523, der DE-A 103 13 210, der DE-A 103 13 213 oder wie in der DE-A 199 48 248 beschrieben durchgeführt werden.

Generell eignet sich das erfindungsgemäße Verfahren im Rahmen einer Propenpartialoxidation zu Acrolein für Propenbelastungen des Katalysatorfestbetts von ≤ 70 NI/I•h, oder ≥ 70 NI/I•h, ≥ 90 NI/I•h, ≥ 110 NI/I•h, ≥ 130 NI/I•h, ≥ 140 NI/I•h, ≥ 160 NI/I•h, ≥ 180 NI/I•h, ≥ 240 NI/I•h, ≥ 300 NI/I•h, jedoch normalerweise ≤ 600 NI/I•h. Dabei ist die Belastung auf das Volumen des Katalysatorfestbetts ausschließlich gegebenenfalls mitverwendeter Abschnitte die ausschließlich aus Inertmaterial bestehen bezogen (wie allgemein in dieser Schrift, sofern nicht explizit etwas anderes gesagt wird).

Erfindungsgemäß vorteilhaft werden die Belastungen der relevanten wenigstens zwei parallel betriebenen Oxidationsreaktorsysteme identisch gewählt.

Zur Bereitung des Katalysatorfestbetts für eine erfindungsgemäße Partialoxidation von Propen zu Acrolein können beim erfindungsgemäßen Verfahren nur die entsprechende Multimetalloxidaktivmasse aufweisende Katalysatorformkörper oder auch weitgehend homogene Gemische aus Multimetalloxidaktivmasse aufweisenden Katalysatorformkörpern und keine Multimetalloxidaktivmasse aufweisenden, sich bezüglich der heterogen katalysierten partiellen Gasphasenoxidation von Propen zu Acrolein im wesentlichen inert verhaltenden (aus Inertmaterial bestehenden), Formkörpern (Verdünnungsformkörper) verwendet werden. Als Materialien für solche inerten Formkörper kommen prinzipiell alle diejenigen in Betracht, die sich auch als Trägermaterial für "Propen-zu-Acrolein"-Schalenkatalysatoren eignen. Als solche Materialien kommen z.B. poröse oder unporöse Aluminiumoxide, Siliciumdioxid, Thoriumdioxid, Zirkondioxid, Siliciumcarbid, Silikate wie Magnesium- oder Aluminiumsilikat oder der bereits erwähnte Steatit (z.B. Steatit C-220 der Fa. CeramTec) in Betracht.

Die Geometrie solcher inerter Verdünnungsformkörper kann im Prinzip beliebig sein. D.h., es können beispielsweise Kugeln, Polygone, Vollzylinder oder auch Ringe sein. Bevorzugt wird man als inerte Verdünnungsformkörper solche wählen, deren Geometrie derjenigen der mit ihnen zu verdünnenden Erststufen-Katalysatorformkörper entspricht.

In der Regel ist es günstig, wenn sich für die beschriebene Propenpartialoxidation zu Acrolein die chemische Zusammensetzung der verwendeten Aktivmasse über das Katalysatorfestbett nicht verändert. D.h., die für einen einzelnen Katalysatorformkörper verwendete Aktivmasse kann zwar ein Gemisch aus verschiedenen, z.B. die Elemente Mo und/oder W sowie wenigstens eines der Elemente Bi, Fe, Sb, Sn und Cu enthaltenden, Multimetalloxiden sein, für alle Katalysatorformkörper des Katalysatorfestbetts wird dann jedoch vorteilhaft das gleiche Gemisch verwendet.

Erfindungsgemäß vorteilhaft weisen die relevanten wenigstens zwei parallel betriebenen Oxidationsreaktorsysteme mit Katalysator auf identische Weise beschickte Oxidationsreaktorstrassen auf.

Bevorzugt nimmt bei der Propenpartialoxidation zu Acrolein normalerweise die volumenspezifische (d.h., die auf die Einheit des Volumens normierte) Aktivität innerhalb des Katalysatorfestbetts in Strömungsrichtung des Reaktionsgasausgangsgemischs kontinuierlich, abrupt oder stufenförmig zu.

Die volumenspezifische Aktivität kann dabei z.B. in einfacher Weise dadurch verringert werden, dass man eine Grundmenge von einheitlich hergestellten Katalysatorformkörpern mit Verdünnungsformkörpern homogen verdünnt. Je höher der Anteil der Verdünnungsformkörper gewählt wird, desto geringer ist die in einem bestimmten Volumen des Festbetts enthaltene Aktivmasse bzw. Katalysatoraktivität.

Eine in Strömungsrichtung des Reaktionsgasgemischs über das Katalysatorfestbett wenigstens einmal zunehmende volumenspezifische Aktivität lässt sich somit in einfacher Weise z.B. dadurch einstellen, dass man die Schüttung mit einem hohen Anteil an inerten Verdünnungsformkörpern bezogen auf eine Sorte von Katalysatorformkörpern beginnt, und dann diesen Anteil an Verdünnungsformkörpern in Strömungsrichtung entweder kontinuierlich oder wenigstens einmal oder mehrfach abrupt (z.B. stufenförmig) verringert. Eine Zunahme der volumenspezifischen Aktivität ist aber auch z.B. dadurch möglich, dass man bei gleich bleibender Geometrie und Aktivmassenart eines Schalenkatalysatorformkörpers die Dicke der auf dem Träger aufgebrachten Aktivmassenschicht erhöht oder in einem Gemisch aus Schalenkatalysatoren mit gleicher Geometrie aber mit unterschiedlichem Gewichtsanteil der Aktivmasse den Anteil an Katalysatorformkörpern mit höherem Aktivmassengewichtsanteil steigert. Alternativ kann man auch die Aktivmassen selbst verdünnen, indem man bei der Aktivmassenherstellung z.B. in das zu calcinierende Trockengemisch aus Ausgangsverbindungen inerte verdünnend wirkende Materialien wie hochgebranntes Siliciumdioxid einarbeitet. Unterschiedliche Zusatzmengen an verdünnend wirkendem Material führen automatisch zu unterschiedlichen Aktivitäten. Je mehr verdünnend wirkendes Material zugesetzt wird, desto geringer wird die resultierende Aktivität sein. Analoge Wirkung lässt sich auch z.B. dadurch erzielen, dass man in Mischungen aus Vollkatalysatoren und aus Schalenkatalysatoren (bei identischer Aktivmasse) in entsprechender Weise das Mischungsverhältnis verändert. Selbstredend lassen sich die beschriebenen Varianten auch kombiniert anwenden.

Natürlich können für das Katalysatorfestbett einer erfindungsgemäßen Propenpartialoxidation zu Acrolein aber auch Mischungen aus Katalysatoren mit chemisch unterschiedlicher Aktivmassenzusammensetzung und als Folge dieser verschiedenen Zusammensetzung unterschiedlicher Aktivität verwendet werden. Diese Mischungen können wiederum mit inerten Verdünnungskörpern verdünnt werden.

Vorab und/oder im Anschluss an die Aktivmasse aufweisenden Abschnitte des Katalysatorfestbetts einer erfindungsgemäßen Propenpartialoxidation zu Acrolein können sich ausschließlich aus Inertmaterial (z.B. nur Verdünnungsformkörpern) bestehende Schüttungen befinden Diese können ebenfalls auf die Temperatur des Katalysatorfestbetts gebracht sein. Dabei können die für die Inertschüttung verwendeten Verdünnungsformkörper die gleiche Geometrie wie die zu den Aktivmasse aufweisenden Abschnitte des Katalysatorfestbetts verwendeten Katalysatorformkörper aufweisen. Die Geometrie der für die Inertschüttung verwendeten Verdünnungsformkörper kann aber auch von der vorgenannten Geometrie der Katalysatorformkörper verschieden sein (z.B. kugelförmig anstatt ringförmig).

Häufig weisen die für solche Inertschüttungen verwendeten Formkörper die ringförmige Geometrie 7 mm x 7 mm x 4 mm (Außendurchmesser x Länge x Innendurchmesser) oder die kugelförmige Geometrie mit dem Durchmesser d = 4-5 mm auf.

Vielfach ist beim erfindungsgemäßen Verfahren der Aktivmasse aufweisende Abschnitt des Katalysatorfestbetts für eine Propenpartialoxidation zu Acrolein in Strömungsrichtung des Reaktionsgasgemisches wie folgt strukturiert (erfindungsgemäß bevorzugt in allen der wenigstens zwei erfindungsgemäß parallel betriebenen Oxidationsreaktorsysteme und dies auf die gleiche Weise).

Zunächst auf einer Länge von 10 bis 60 %, bevorzugt 10 bis 50 %, besonders bevorzugt 20 bis 40 % und ganz besonders bevorzugt 25 bis 35 % (d.h., z.B. auf einer Länge von 0,70 bis 1,50 m, bevorzugt 0,90 bis 1,20 m), jeweils der Gesamtlänge des Aktivmasse aufweisenden Abschnitts der Festbettkatalysatorschüttung, ein homogenes Gemisch oder zwei (mit abnehmender Verdünnung) aufeinander folgende homogene Gemische aus Katalysatorformkörpern und Verdünnungsformkörpern (wobei beide vorzugsweise im wesentlichen die gleiche Geometrie aufweisen), wobei der Gewichtsanteil der Verdünnungsformkörper (die Massendichten von Katalysatorformkörpern und von Verdünnungsformkörpern unterscheiden sich in der Regel nur geringfügig) normalerweise 5 bis 40 Gew.-%, vorzugsweise 10 bis 40 Gew.-% oder 20 bis 40 Gew.-% und besonders bevorzugt 25 bis 35 Gew.-% beträgt. Im Anschluss an diese erste Zone befindet sich dann häufig vorteilhaft bis zum Ende der Länge des Aktivmasse aufweisenden Abschnitts des Katalysatorfestbetts (d.h., z.B. auf einer Länge von 2,00 bis 3,00 m, bevorzugt 2,50 bis 3,00 m) entweder eine nur in geringerem Umfang (als in der ersten Zone) verdünnte Schüttung der Katalysatorformkörper, oder, ganz besonders bevorzugt, eine alleinige Schüttung derselben Katalysatorformkörper, die auch in der ersten Zone verwendet worden sind.

Das Vorgenannte trifft insbesondere dann zu, wenn im Katalysatorfestbett als Katalysatorformkörper Vollkatalysatorringe oder Schalenkatalysatorringe eingesetzt werden (insbesondere jene, die in dieser Schrift als bevorzugt angeführt werden). Mit Vorteil weisen im Rahmen der vorgenannten Strukturierung sowohl die Katalysatorformkörper bzw. deren Trägerringe als auch die Verdünnungsformkörper beim erfindungsgemäßen Verfahren im wesentlichen die Ringgeometrie 5 mm x 3 mm x 2 mm (Außendurchmesser x Länge x Innendurchmesser) auf.

Das oben Genannte trifft auch dann zu, wenn anstelle inerter Verdünnungsformkörper Schalenkatalysatorformkörper verwendet werden, deren Aktivmassenanteil um 2 bis 15 Gew.-%-Punkte tiefer liegt, als der Aktivmassenanteil der Schalenkatalysatorformkörper am Ende des Katalysatorfestbetts.

Eine reine Inertmaterialschüttung, deren Länge, bezogen auf die Gesamtlänge des Katalysatorfestbetts, zweckmäßig 1 oder 5 bis 20 % beträgt, leitet in Strömungsrichtung des Reaktionsgasgemischs in der Regel das Katalysatorfestbett ein. Sie wird normalerweise als Aufheizzone für das Reaktionsgasgemisch genutzt.

Üblicherweise sind die Kontaktrohre in den Rohrbündelreaktoren für die Stufe der Partialoxidation von Propen zu Acrolein aus ferritischem Stahl gefertigt und weisen in typischer Weise eine Wanddicke von 1 bis 3 mm auf. Ihr Innendurchmesser beträgt in der Regel (einheitlich) 20 bis 30 mm, häufig 21 bis 26 mm. Anwendungstechnisch zweckmäßig beläuft sich die im Rohrbündelbehälter untergebrachte Anzahl an Kontaktrohen auf wenigstens 5000, vorzugsweise auf wenigstens 10000. Häufig beträgt die Anzahl der im Reaktionsbehälter untergebrachten Kontaktrohre 15000 bis 30000. Rohrbündelreaktoren mit einer oberhalb von 40000 liegenden Anzahl an Kontaktrohren bilden für diese Reaktionsstufe eher die Ausnahme. Innerhalb des Behälters sind die Kontaktrohre im Normalfall homogen verteilt angeordnet, wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. z.B. EP-B 468290).

Die Ausführung der Reaktionsstufe vom Acrolein zur Acrylsäure, kann man mit den für das Katalysatorfestbett dieser Reaktion als geeignet beschriebenen Katalysatoren ebenfalls in einfachster Weise und anwendungstechnisch zweckmäßig in einem mit den Festbettkatalysatoren beschickten Rohrbündelreaktor, wie er z.B. in der EP-A 700 893 bzw. der DE-A 4 431 949 oder der WO 03/057653, oder der WO 03/055835, oder der WO 03/059857, oder der WO 03/076373 beschrieben ist, durchführen.

D.h., in einfachster Weise befindet sich das zu verwendende Katalysatorfestbett in den einheitlich beschickten Metallrohren eines Rohrbündelreaktors und um die Metallrohre wird ein Temperiermedium (Einzonenfahrweise), in der Regel eine Salzschmelze, geführt. Salzschmelze (Temperiermedium) und Reaktionsgasgemisch können dabei im einfachen Gleich- oder Gegenstrom geführt werden. Das Temperiermedium (die Salzschmelze) kann aber auch über den Reaktor betrachtet mäanderförmig um die Rohrbündel geführt werden, so dass lediglich über den gesamten Reaktor betrachtet ein Gleich- oder Gegenstrom zur Strömungsrichtung des Reaktionsgasgemischs besteht. Der Volumenstrom des Temperiermediums (Wärmeaustauschmittels) wird dabei üblicherweise so bemessen, dass der Temperaturanstieg (bedingt durch die Exothermie der Reaktion) des Wärmeaustauschmittels von der Eintrittstelle in den Reaktor bis zur Austrittstelle aus dem Reaktor 0 bis 10°C, häufig 2 bis 8°C, oft 3 bis 6°C beträgt. Die Eintrittstemperatur des Wärmeaustauschmittels in den Rohrbündelreaktor (sie entspricht in dieser Schrift der Temperatur des Katalysatorfestbetts) beträgt in der Regel 220 bis 350°C, häufig 245 bis 285°C bzw. 245 bis 265°C. Als Wärmeaustauschmittel eignen sich insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle. Aber auch ionische Flüssigkeiten sind einsetzbar.

Zweckmäßigerweise wird das Reaktionsgasausgangsgemisch der Beschickung mit Festbettkatalysator auf die gewünschte Reaktionstemperatur vorerwärmt zugeführt.

Insbesondere im Fall von angestrebten hohen (z.B. ≥ 130 NI/I•h, oder ≥ 140 NI/I•h, in der Regel jedoch ≤ 350 NI/I•h, oder ≤ 600 NI/I•h) Belastungen des Katalysatorfestbetts mit Acrolein erfolgt die Durchführung des erfindungsgemäßen Verfahrens einer Acroleinpartialoxidaton zu Acrylsäure zweckmäßig in einem Zwei- oder Mehrzonenrohrbündelreaktor (eine Durchführung im Einzonenrohrbündelreaktor ist jedoch ebenfalls möglich). Eine bevorzugte Variante eines für diesen Zweck erfindungsgemäß einsetzbaren Zweizonenrohrbündelreaktors offenbart die DE-C 2830765. Aber auch die in der DE-C 2513405, der US-A 3147084, der DE-A 2201528, der EP-A 383224 und der DE-A 2903582 offenbarten Zweizonenrohrbündelreaktoren sind geeignet.

D.h., in einfacher Weise befindet sich das erfindungsgemäß zu verwendende wenigstens eine Katalysatorfestbett in den einheitlich beschickten Metallrohren eines Rohrbündelreaktors und um die Metallrohre werden zwei voneinander im wesentlichen räumlich getrennte Temperiermedien, in der Regel Salzschmelzen, geführt. Der Rohrabschnitt, über den sich das jeweilige Salzbad erstreckt, repräsentiert eine Temperatur- bzw. Reaktionszone.

Vorzugsweise umströmt z.B. ein Salzbad C denjenigen Abschnitt der Rohre (die Reaktionszone C), in welchem sich die oxidative Umsetzung des Acroleins (beim einfachen Durchgang) bis zum Erreichen eines Umsatzwertes im Bereich von 55 bis 85 mol.% vollzieht und ein Salzbad D umströmt den Abschnitt der Rohre (die Rekationszone D), in welchem sich die oxidative Anschlussumsetzung des Acroleins (beim einfachen Durchgang) bis zum Erreichen eines Umsatzwertes von in der Regel wenigstens 90 mol.% vollzieht (bei Bedarf können sich an die Reaktionszonen C, D weitere Reaktionszonen anschließen, die auf individuellen Temperaturen gehalten werden).

Innerhalb der jeweiligen Temperaturzone kann das Salzbad prinzipiell wie bei der Einzonenfahrweise geführt werden. Die Eintrittstemperatur des Salzbades D liegt normalerweise wenigstens 5 bis 10°C oberhalb der Temperatur des Salzbades C. Im übrigen können die Eintrittstemperaturen im für die Einzonenfahrweise empfohlenen Temperaturbereich für die Eintrittstemperatur liegen.

Ansonsten kann die Zwei-Zonen-Hochlastfahrweise der Acroleinpartialoxidation zu Acrylsäure z.B. wie in der DE-A 19 94 8523, der EP-A 11 06 598 oder wie in der DE-A 19 94 8248 beschrieben durchgeführt werden.

Das erfindungsgemäße Verfahren eignet sich somit für Acroleinbelastungen des Katalysatorfestbetts von ≤ 70 NI/I•h, oder ≥ 70 NI/I•h, ≥ 90 NI/I•h, ≥ 110 NI/I•h, ≥ 130 NI/I•h, ≥ 180 NI/I•h, 240 NI/I•h, ≥ 300 NI/I•h, jedoch normalerweise ≤ 600 NI/I•h. Dabei ist die Belastung auf das Volumen des Katalysatorfestbetts ausschließlich gegebenenfalls mitverwendeter Abschnitte die ausschließlich aus Inertmaterial bestehen bezogen.

Zur Bereitung des wenigstens einen Katalysatorfestbetts können für eine erfindungsgemäße Partialoxidation von Acrolein zu Acrylsäure nur die entsprechende Multimetalloxidaktivmasse aufweisende Katalysatorformkörper oder auch weitgehend homogene Gemische aus Multimetalloxidaktivmasse aufweisenden Katalysatorformkörpern und keine Multimetalloxidaktivmasse aufweisenden, sich bezüglich der heterogen katalysierten partiellen Gasphasenoxidation im wesentlichen inert verhaltenden (aus Inertmaterial bestehenden), Formkörpern (Verdünnungsformkörper) verwendet werden. Als Materialien für solche inerten Formkörper kommen prinzipiell alle diejenigen in Betracht, die sich auch als Trägermaterial für erfindungsgemäß geeignete "Acrolein-zu-Acrylsäure-Schalenkatalysatoren" eignen. Als solche Materialien kommen z.B. poröse oder unporöse Aluminiumoxide, Siliciumdioxid, Thoriumdioxid, Zirkondioxid, Siliciumcarbid, Silikate wie Magnesium- oder Aluminiumsilikat oder der bereits erwähnte Steatit (z.B. Steatit C-220 der Fa. CeramTec) in Betracht.

Die Geometrie solcher inerter Verdünnungsformkörper kann im Prinzip beliebig sein. D.h., es können beispielsweise Kugeln, Polygone, Vollzylinder oder auch Ringe sein. Erfindungsgemäß bevorzugt wird man als inerte Verdünnungsformkörper solche wählen, deren Geometrie derjenigen der mit ihnen zu verdünnenden Katalysatorformkörper entspricht.

In der Regel ist es im Rahmen einer erfindungsgemäßen Partialoxidation von Acrolein zu Acrylsäure günstig, wenn sich die chemische Zusammensetzung der verwendeten Aktivmasse über das Katalysatorfestbett nicht verändert. D.h., die für einen einzelnen Katalysatorformkörper verwendete Aktivmasse kann zwar ein Gemisch aus verschiedenen die Elemente Mo und V enthaltenden Multimetalloxiden sein, für alle Katalysatorformkörper des Katalysatorfestbetts ist dann jedoch vorteilhaft das gleiche Gemisch zu verwenden.

Bevorzugt nimmt für eine erfindungsgemäße Partialoxidation von Acrolein zu Acrylsäure normalerweise die volumenspezifische (d.h., die auf die Einheit des Volumens normierte) Aktivität innerhalb des Katalysatorfestbetts in Strömungsrichtung des Reaktionsgasgemischs kontinuierlich, abrupt oder stufenförmig zu.

Die volumenspezifische Aktivität kann dabei z.B. in einfacher Weise dadurch verringert werden, dass man eine Grundmenge von einheitlich hergestellten Katalysatorformkörpern mit Verdünnungsformkörpern homogen verdünnt. Je höher der Anteil der Verdünnungsformkörper gewählt wird, desto geringer ist die in einem bestimmten Volumen des Festbetts enthaltene Aktivmasse bzw. Katalysatoraktivität.

Eine in Strömungsrichtung des Reaktionsgasgemischs über das Katalysatorfestbett wenigstens einmal zunehmende volumenspezifische Aktivität lässt sich für ein erfindungsgemäßes Verfahren der Acroleinpartialoxidation zu Acrylsäure somit in einfacher Weise z.B. dadurch einstellen, dass man die Schüttung mit einem hohen Anteil an inerten Verdünnungsformkörpern bezogen auf eine Sorte von Katalysatorformkörpern beginnt, und dann diesen Anteil an Verdünnungsformkörpern in Strömungsrichtung entweder kontinuierlich oder wenigstens einmal oder mehrfach abrupt (z.B. stufenförmig) verringert. Eine Zunahme der volumenspezifischen Aktivität ist aber auch z.B. dadurch möglich, dass man bei gleich bleibender Geometrie und Aktivmassenart eines Schalenkatalysatorformkörpers die Dicke der auf dem Träger aufgebrachten Aktivmassenschicht erhöht oder in einem Gemisch aus Schalenkatalysatoren mit gleicher Geometrie aber mit unterschiedlichem Gewichtsanteil der Aktivmasse den Anteil an Katalysatorformkörpern mit höherem Aktivmassengewichtsanteil steigert. Alternativ kann man auch die Aktivmassen selbst verdünnen, indem man bei der Aktivmassenherstellung z.B. in das zu calcinierende Trockengemisch aus Ausgangsverbindungen inerte verdünnend wirkende Materialien wie hochgebranntes Siliciumdioxid einarbeitet. Unterschiedliche Zusatzmengen an verdünnend wirkendem Material führen automatisch zu unterschiedlichen Aktivitäten. Je mehr verdünnend wirkendes Material zugesetzt wird, desto geringer wird die resultierende Aktivität sein. Analoge Wirkung lässt sich auch z.B. dadurch erzielen, dass man in Mischungen aus Vollkatalysatoren und aus Schalenkatalysatoren (bei identischer Aktivmasse) in entsprechender Weise das Mischungsverhältnis verändert. Selbstredend lassen sich die beschriebenen Varianten auch kombiniert anwenden.

Natürlich können für das Katalysatorfestbett einer erfindungsgemäßen Acroleinpartialoxidation zu Acrylsäure aber auch Mischungen aus Katalysatoren mit chemisch unterschiedlicher Aktivmassenzusammensetzung und als Folge dieser verschiedenen Zusammensetzung unterschiedlicher Aktivität verwendet werden. Diese Mischungen können wiederum mit inerten Verdünnungskörpern verdünnt werden.

Vorab und/oder im Anschluss an die Aktivmasse aufweisenden Abschnitte des Katalysatorfestbetts können sich dabei ausschließlich aus Inertmaterial (z.B. nur Verdünnungsformkörpern) bestehende Schüttungen befinden. Diese können ebenfalls auf die Temperatur des Katalysatorfestbetts gebracht sein. Dabei können die für die Inertschüttung verwendeten Verdünnungsformkörper die gleiche Geometrie wie die zu den Aktivmasse aufweisenden Abschnitte des Katalysatorfestbetts verwendeten Katalysatorformkörper aufweisen. Die Geometrie der für die Inertschüttung verwendeten Verdünnungsformkörper kann aber auch von der vorgenannten Geometrie der Katalysatorformkörper verschieden sein (z.B. kugelförmig anstatt ringförmig).

Häufig weisen die für solche Inertschüttungen verwendeten Formkörper die ringförmige Geometrie 7 mm x 7 mm x 4 mm (Außendurchmesser x Länge x Innendurchmesser) oder die kugelförmige Geometrie mit dem Durchmesser d = 4-5 mm auf.

Vielfach ist bei einem erfindungsgemäßen Verfahren der Acroleinpartialoxidation zu Acrylsäure der Aktivmasse aufweisende Abschnitt des Katalysatorfestbetts in Strömungsrichtung des Reaktionsgasgemisches wie folgt strukturiert (erfindungsgemäß bevorzugt in allen der wenigstens zwei erfindungsgemäß parallel betriebenen Oxidationsreaktorsysteme und dies auf die gleiche Weise).

Zunächst auf einer Länge von 10 bis 60 %, bevorzugt 10 bis 50 %, besonders bevorzugt 20 bis 40 % und ganz besonders bevorzugt 25 bis 35 % (d.h., z.B. auf einer Länge von 0,70 bis 1,50 m, bevorzugt 0,90 bis 1,20 m), jeweils der Gesamtlänge des Aktivmasse aufweisenden Abschnitts der Festbettkatalysatorschüttung, ein homogenes Gemisch oder zwei (mit abnehmender Verdünnung) aufeinander folgende homogene Gemische aus Katalysatorformkörpern und Verdünnungsformkörpern (wobei beide vorzugsweise im wesentlichen die gleiche Geometrie aufweisen), wobei der Gewichtsanteil der Verdünnungsformkörper (die Massendichten von Katalysatorformkörpern und von Verdünnungsformkörpern unterscheiden sich in der Regel nur geringfügig) normalerweise 10 bis 50 Gew.-%, vorzugsweise 20 bis 45 Gew.-% und besonders bevorzugt 25 bis 35 Gew.-% beträgt. Im Anschluss an diese erste Zone befindet sich dann häufig vorteilhaft bis zum Ende der Länge des Aktivmasse aufweisenden Abschnitts des Katalysatorfestbetts (d.h., z.B. auf einer Länge von 2,00 bis 3,00 m, bevorzugt 2,50 bis 3,00 m) entweder eine nur in geringerem Umfang (als in der ersten (bzw. in den ersten beiden) Zone) verdünnte Schüttung der Katalysatorformkörper, oder, ganz besonders bevorzugt, eine alleinige Schüttung derselben Katalysatorformkörper, die auch in der ersten (bzw. den ersten beiden) Zone verwendet worden sind.

Das Vorgenannte trifft insbesondere dann zu, wenn im Katalysatorfestbett als Katalysatorformkörper Schalenkatalysatorringe oder Schalenkatalysatorkugeln eingesetzt werden (insbesondere jene, die in dieser Schrift für eine Acroleinpartialoxidation zu Acrylsäure als bevorzugt angeführt werden). Mit Vorteil weisen im Rahmen der vorgenannten Strukturierung sowohl die Katalysatorformkörper bzw. deren Trägerringe als auch die Verdünnungsformkörper bei einem erfindungsgemäßen Verfahren der Acroleinpartialoxidation zu Acrylsäure im wesentlichen die Ringgeometrie 7 mm x 3 mm x 4 mm (Außendurchmesser x Länge x Innendurchmesser) auf.

Das oben Genannte trifft auch dann zu, wenn anstelle inerter Verdünnungsformkörper Schalenkatalysatorformkörper verwendet werden, deren Aktivmassenanteil um 2 bis 15 Gew.-%-Punkte tiefer liegt, als der Aktivmassenanteil der Schalenkatalysatorformkörper am Ende des Katalysatorfestbetts.

Eine reine Inertmaterialschüttung, deren Länge, bezogen auf die Gesamtlänge des Katalysatorfestbetts, zweckmäßig 5 bis 20 % beträgt, leitet in Strömungsrichtung des Reaktionsgasgemischs in der Regel das Katalysatorfestbett für die Acroleinpartialoxidation ein. Sie wird normalerweise als Aufheizzone für das Reaktionsgasgemisch genutzt.

Üblicherweise sind für die erfindungsgemäße Acroleinpartialoxidation die Kontaktrohre in den Rohrbündelreaktoren aus ferritischem Stahl gefertigt und weisen in typischer Weise eine Wanddicke von 1 bis 3 mm auf. Ihr Innendurchmesser beträgt in der Regel (einheitlich) 20 bis 30 mm, häufig 21 bis 26 mm. Anwendungstechnisch zweckmäßig beläuft sich die im Rohrbündelbehälter untergebrachte Anzahl an Kontaktrohen auf wenigstens 5000, vorzugsweise auf wenigstens 10000. Häufig beträgt die Anzahl der im Reaktionsbehälter untergebrachten Kontaktrohre 15000 bis 30000. Rohrbündelreaktoren mit einer oberhalb von 40000 liegenden Anzahl an Kontaktrohren bilden für die Acroleinpartialoxidation eher die Ausnahme. Innerhalb des Behälters sind die Kontaktrohre im Normalfall homogen verteilt angeordnet, wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. z.B. EP-B 468290).

Wie bereits beschrieben, können beim erfindungsgemäßen Verfahren sowohl die Propen- als auch die Acroleinpartialoxidation in Einzonen- oder in Zweizonenrohrbündelreaktoren durchgeführt werden. Schaltet man die beiden Reaktionsstufen hintereinander, kann aber auch nur die erste Reaktionsstufe in einem Einzonenrohrbündelreaktor und die zweite Reaktionsstufe in einem Zweizonenrohrbündelreaktor (oder umgekehrt) durchgeführt werden. Das Produktgasgemisch der ersten Reaktionsstufe (vorzugsweise nach Mischung über alle parallel betriebenen Oxidationsreaktorsysteme) wird dabei, gegebenenfalls nach Ergänzung mit Inertgas, oder mit molekularem Sauerstoff, oder mit Inertgas und molekularem Sauerstoff sowie gegebenenfalls nach erfolgter direkter und/oder indirekter Zwischenkühlung, unmittelbar der zweiten Reaktionsstufe zugeführt.

Zwischen den Rohrbündelreaktoren der ersten und der zweiten Reaktionsstufe kann sich dabei ein Zwischenkühler befinden, der gegebenenfalls Inertschüttungen enthalten kann.

Selbstredend können für das erfindungsgemäße Verfahren einer zweistufigen Partialoxidation von Propen zu Acrylsäure das Katalysatorfestbett der Propenpartialoxidation und das Katalysatorfestbett der Acroleinpartialoxidation auch räumlich aufeinanderfolgend in einem einzigen, ebenfalls z.B. zwei Temperaturzonen oder mehr aufweisenden, Vielkontaktrohrbündelreaktor untergebracht sein, wie es z.B. die WO 03/059857, die EP-A 911313 und die EP-A 990636 beschreiben. Man spricht in diesem Fall von einem Single-Reaktor-Zweistufenverfahren. Eine oder zwei Temperaturzonen erstrecken sich dabei in der Regel über ein Katalysatorfestbett. Zwischen den beiden Katalysatorfestbetten kann sich zusätzlich eine Inertschüttung befinden, die gegebenenfalls in einer dritten Temperaturzone befindlich ist und separat temperiert wird. Die Kontaktrohre können dabei durchgehend oder durch die Inertschüttung unterbrochen ausgeführt sein.

Das für das Beschickungsgasgemisch der "Propen-zu-Acrolein-Reaktionsstufe" (das Reaktionsgasausgangsgemisch 1) zu verwendende Inertgas kann unabhängig von der für das Katalysatorfestbett gewählten Propenbelastung (und unabhängig davon, ob sich eine "Acrolein-zu-Acrylsäure-Reaktionsstufe" anschließt) zu z.B. ≥ 20 Vol.-%, oder zu ≥ 30 Vol.-%, oder zu ≥ 40 Vol.%, oder zu ≥ 50 Vol.%, oder zu ≥ 60 Vol.-%, oder zu ≥ 70 Vol.-%, oder zu ≥ 80 Vol.-%, oder zu ≥ 90 Vol.-%, oder zu ≥ 95 Vol.-% aus molekularem Stickstoff bestehen.

Das inerte Verdünnungsgas kann dabei aber auch z.B. zu 2 bis 35 bzw. 20 Gew.-% aus H₂O und zu 65 bis 98 Vol.% aus N₂ bestehen.

Bei Propenbelastungen des Katalysatorfestbetts der "Propen-zu-Acrolein-Reaktionsstufe" oberhalb von 250 NI/I•h wird für das erfindungsgemäße Verfahren jedoch die Mitverwendung von inerten Verdünnungsgasen wie Propan, Ethan, Methan, Butan, Pentan, CO₂, CO, Wasserdampf und/oder Edelgasen empfohlen. Selbstverständlich können diese Gase aber auch bereits bei geringeren Propenbelastungen zur Dämpfung der Heißpunktbildung mitverwendet werden.

Der Arbeitsdruck kann bei der erfindungsgemäßen Gasphasenpartialoxidation des Propens zu Acrolein (insbesondere am Anfang der Betriebsdauer eines Katalysatorfestbetts) sowohl unterhalb von Normaldruck (z.B. bis zu 0,5 bar) als auch oberhalb von Normaldruck liegen. Typischerweise wird der Arbeitsdruck bei der Gasphasen-Partialoxidation des Propens bei Werten von 1 bis 5 bar, häufig 1 bis 3 bar liegen.

Normalerweise wird der Reaktionsdruck bei der erfindungsgemäßen Propenpartialoxidation zu Acrolein 100 bar nicht überschreiten. Selbstverständlich kann die erfindungsgemäße Verfahrensweise die Propenpartialoxidation betreffend ganz generell mit den in den Schriften EP-A 990 636, EP-A 11 06 598, EP-A 614 872, DE-A 10 35 822, DE-A 10 23 2748, DE-A 10351269 sowie in der deutschen Anmeldung DE-A 10 2004 025 445 zur Standzeitverlängerung eines Katalysatorbetts empfohlenen Verfahrensweisen auch in Kombination angewendet werden. Es sind so Katalysatorbettstandzeiten von mehreren Jahren erreichbar.

Das molare Verhältnis von O₂:Propen im Reaktionsgasausgangsgemisch 1 für die Propenpartialoxidation zu Acrolein, das beim erfindungsgemäßen Verfahren durch das entsprechende Katalysatorfestbett geführt wird, wird (im wesentlichen unabhängig davon, ob eine Acroleinpartialoxidationsstufe zu Acrylsäure nachfolgt) normalerweise ≥ 1 betragen. Üblicherweise wird dieses Verhältnis bei Werten ≤ 3 liegen. Häufig wird das molare Verhältnis von O₂:Propen im vorgenannten Beschickungsgasgemisch vorteilhaft 1 bis 2 bzw. 1,4 bis 2 betragen. Vielfach wird man das Verfahren der Propenpartialoxidation zu Acrolein mit einem im Reaktionsgasausgangsgemisch 1 vorliegenden Propen:Sauerstoff:Inertgas (einschließlich Wasserdampf) - Volumenverhältnis (NI) von 1:(1 bis 3) : (3 bis 30), vorzugsweise von 1:(1,5 bis 2,3): (10 bis 15) ausführen.

Der Propenanteil im Reaktionsgasausgangsgemisch 1 kann dabei z.B. bei Werten von 4 bis 20 Vol.-%, häufig bei 5 oder 7 bis 15 Vol.-% bzw. 6 oder 8 bis 12 Vol.-% oder 5 bis 8 Vol.-% liegen (jeweils bezogen auf das Gesamtvolumen).

Eine typische Zusammensetzung des Reaktionsgasausgangsgemischs 1 (unabhängig von der gewählten Belastung und unabhängig davon, ob eine Acroleinpartialoxidationsstufe zu Acrylsäure folgt) kann die nachfolgenden Komponenten enthalten:

| | |
|---|---|
| 6 bis 6,5 Vol.-% | Propen, |
| 3 bis 3,5 Vol.% | H₂O, |
| 0,3 bis 0,5 Vol.% | CO, |
| 0,8 bis 1,2 Vol.-% | CO₂, |
| 0,01 bis 0,04 Vol.-% | Acrolein, |
| 10,4 bis 10,7 Vol.-% | O₂ und |

als Restmenge ad 100% molekularer Stickstoff,
oder: 5,4 Vol.-% Propen,
10,5 Vol.-% Sauerstoff,
1,2 Vol.-% COx,
80,5 Vol.-% N₂ und
2,4 Vol.-% H₂O.

Das Reaktionsgasausgangsgemisch 1 kann für die Propenpartialoxidation zu Acrolein erfindungsgemäß aber auch wie folgt zusammengesetzt sein:
6 bis 15 Vol.-% Propen,
4 bis 30 Vol.-% (häufig 6 bis 15 Vol.-%) Wasser,
≥ 0 bis 10 Vol.% (vorzugsweise ≥ 0 bis 5 Vol.-%) an von Propen, Wasser, Sauerstoff und Stickstoff verschiedenen Bestandteilen, soviel molekularem Sauerstoff, dass das molare Verhältnis von enthaltenem molekularem Sauerstoff zu enthaltenem molekularem Propen 1,5 bis 2,5 beträgt und als Restmenge bis zur 100 Vol.-% Gesamtmenge aus molekularem Stickstoff.

Eine andere mögliche Reaktionsgasausgangsgemisch-1-Zusammensetzung kann für die Propenpartialoxidation zu Acrolein erfindungsgemäß enthalten:
6,0 Vol.-% Propen,
60 Vol.-% Luft und
34 Vol.-% H₂O.

Alternativ können auch Reaktionsgasausgangsgemische 1 der Zusammensetzung gemäß Example 1 der EP-A 990 636, oder gemäß Example 2 der EP-A 990 636, oder gemäß Example 3 der EP-A 1 106 598, oder gemäß Example 26 der EP-A 1 106 598, oder gemäß Example 53 der EP-A 1 106 598 für die "Propen-zu-Acrolein- Reaktionsstufe" verwendet werden.

Weitere erfindungsgemäß geeignete Reaktionsgasausgangsgemische 1 für die "Propen-zu-Acrolein-Reaktionsstufe" können im nachfolgenden Zusammensetzungsraster liegen:

| | |
|---|---|
| 7 bis 11 Vol.% | Propen, |
| 6 bis 12 Vol.-% | Wasser, |
| ≥ 0 bis 5 Vol.-% | an von Propen, Wasser, Sauerstoff und Stickstoff verschiedenen Bestandteilen, |

soviel molekularem Sauerstoff, dass das molare Verhältnis von enthaltenem Sauerstoff zu enthaltenem molekularem Propen 1,4 bis 2,2 beträgt, und
als Restmenge bis zur 100 Vol.-% Gesamtmenge molekularer Stickstoff.

Als im Reaktionsgasausgangsgemisch 1 zu verwendendes Propen kommen vor allem polymer grade Propen und chemical grade Propen in Betracht, wie es z.B. die DE-A 10232748 beschreibt. In erfindungsgemäß vorteilhafter Weise kann als Propenquelle für eine erfindungsgemäße heterogen katalysierte Propen-Gasphasen-Partialoxidation mit molekularem Sauerstoff zu Acrolein und/oder Acrylsäure (bzw. Ammoxidation zu Acrylnitril) in wenigstens zwei parallel betriebenen Oxidationsreaktorsystemen aber auch eine heterogen katalysierte Dehydrierung und/oder Oxidehydrierung von Propan zu Propen fungieren, wie es z.B. die Schriften DE-A 102 45 585, WO 03/076370, DE-A 103 16 039, DE-A 33 13 573, US 3,161,670, WO 01/96270, WO 01/96271 und WO 03/011804 beschreiben. In vorteilhafter Weise wird dabei das partiell zu oxidierende Propen von Propan begleitet.

Vorzugsweise bedient dabei ein Dehydrierreaktor wenigstens zwei erfindungsgemäß parallel betriebene Oxidationsreaktorsysteme mit Propen.

Das Reaktionsgasausgangsgemisch 1 liegt dann vorzugsweise im folgenden Zusammensetzungsraster:

| | |
|---|---|
| 7 bis 15 Vol.-% | O₂, |
| 5 bis 10 Vol.-% | Propylen, |
| 10 bzw. 15 bis 40 Vol.-% | Propan, |
| 25 bis 60 Vol.-% | Stickstoff, |
| 1 bis 5 Vol.-% | Summe aus CO, CO₂ und H₂O und |
| 0 bis 5 Vol.-% | sonstige Bestandteile, |

wobei gegebenenfalls enthaltener Ammoniak unberücksichtigt ist.

An dieser Stelle sei noch erwähnt, dass, unabhängig davon, ob sich eine "Acrolein-zu-Acrylsäure-Reaktionsstufe" anschließt, ein Teil des Beschickungsgasgemischs der "Propen-zu-Acrolein-Reaktionsstufe" sogenanntes Kreisgas sein kann. Hierbei handelt es sich, wie bereits beschrieben, um Gas, das nach der Zielproduktabtrennung aus dem Produktgas im dem Oxidationsreaktorsystem erfindungsgemäß folgenden Abtrennsystem (für Acrolein und/oder Acrylsäure) verbleibt und in der Regel zum Teil als weitgehend inertes Verdünnungsgas zum Beschicken der Propen- und/oder der sich gegebenenfalls anschließenden Acrolein-Reaktionsstufe rückgeführt wird.

Als Sauerstoffquelle wird normalerweise Luft eingesetzt.

Die Belastung des Katalysatorfestbetts (ausschließlich reiner Inertabschnitte) mit Reaktionsgasausgangsgemisch 1 wird insbesondere bei einem erfindungsgemäßen Verfahren zur Herstellung von Acrolein und/oder Acrylsäure aus Propen in typischer Weise 1000 bis 10000 NI/I•h, meist 1000 bis 5000 NI/I•h, häufig 1500 bis 4000 NI/I•h betragen.

Schließt sich an die Propenpartialoxidation zu Acrolein eine Acroleinpartialoxidation an, so wird gegebenenfalls nach erfolgter Zwischenkühlung das Produktgasgemisch der Propen-Reaktionsstufe der Acrolein-Reaktionsstufe zugeführt. Erfindungsgemäß vorteilhaft werden dabei vorab die Produktgasströme der wenigstens zwei Propenpartialoxidationen zu Acrolein miteinander gemischt. Der in der Acrolein-Reaktionsstufe benötigte Sauerstoff kann dabei bereits als Überschuss dem Reaktionsgasausgangsgemisch 1 für die Propen-Reaktionsstufe zugesetzt werden und somit Bestandteil des Produktgasgemischs der Propen-Reaktionsstufe sein. In diesem Fall kann das, gegebenenfalls zwischengekühlte, Produktgasgemisch der Propen-Reaktionsstufe unmittelbar das Beschickungsgasgemisch der Acrolein-Reaktionsstufe sein. Der für den zweiten Oxidationsschritt vom Acrolein zur Acrylsäure benötigte Sauerstoff kann aber dem Produktgasgemisch der Propen-Reaktionsstufe auch vor dem Eintritt desselben in die Acrolein-Reaktionsstufe erst teilweise oder vollständig zugesetzt werden, z.B. in Form von Luft. Mit dieser Zugabe kann dabei eine Direktkühlung des Produktgasgemischs der Acrolein-Reaktionsstufe verbunden sein.

Bereits aus dem vorgenannten Zusammenhang resultierend, kann das im Beschickungsgasgemisch für eine Acrolein-Reaktionsstufe (dem Reaktionsgasausgangsgemisch 2) enthaltene Inertgas (unabhängig davon, ob eine Propen-Reaktionsstufe vorausgeht) zu z.B. ≥ 20 Vol.-%, oder zu ≥ 30 Vol.-%, oder zu ≥ 40 Vol.-%, oder zu ≥ 50 Vol.%, oder zu ≥ 60 Vol.-%, oder zu ≥ 70 Vol.-%, oder zu ≥ 80 Vol.%, oder zu ≥ 90 Vol.-%, oder zu ≥ 95 Vol.-% aus molekularem Stickstoff bestehen.

Häufig wird das inerte Verdünnungsgas im Beschickungsgas für die Acrolein-Reaktionsstufe aber zu 5 bis 25 bzw. 20 Gew.-% aus H₂O (kann z.B. in einer vorausgehenden Propen-Reaktionsstufe gebildet und/oder gegebenenfalls zugesetzt werden) und zu 70 bis 90 Vol.-% aus N₂ bestehen.

Bei Acroleinbelastungen des Katalysatorfestbetts für die Partialoxidation von Acrolein zu Acrylsäure oberhalb von 250 NI/I•h wird für das erfindungsgemäße Verfahren jedoch die Mitverwendung von inerten Verdünnungsgasen wie Propan, Ethan, Methan, Butan, Pentan, CO₂, Wasserdampf und/oder Edelgasen empfohlen. Selbstverständlich können diese Gase aber auch bereits bei geringeren Acroleinbelastungen mitverwendet werden.

Der Arbeitsdruck kann bei der erfindungsgemäßen Gasphasenpartialoxidation des Acroleins zu Acrylsäure (insbesondere am Anfang der Betriebsdauer eines Katalysatorfestbetts) sowohl unterhalb von Normaldruck (z.B. bis zu 0,5 bar) als auch oberhalb von Normaldruck liegen. Typischerweise wird der Arbeitsdruck bei der Gasphasen-Partialoxidation des Acroleins bei Werten von 1 bis 5 bar, häufig 1 bis 3 bar liegen.

Normalerweise wird der Reaktionsdruck bei der erfindungsgemäßen Acroleinpartialoxidation 100 bar nicht überschreiten. Selbstverständlich kann die erfindungsgemäße Verfahrensweise ganz generell auch mit den in den Schriften EP-A 990 636, EP-A 11 06 598, EP-A 614 872, DE-A 10 35 0822, DE-A 10 23 2748, DE-A 10351269 sowie in der deutschen Anmeldung DE-A 10 2004 025 445 zur Standzeitverlängerung eines Katalysatorbetts empfohlenen Verfahrensweisen in Kombination angewendet werden. Es sind so Katalysatorbettstandzeiten von mehreren Jahren erreichbar.

Das molare Verhältnis von O₂:Acrolein im Beschickungsgasgemisch für eine Acrolein-Reaktionsstufe, das beim erfindungsgemäßen Verfahren durch das entsprechende Katalysatorfestbett geführt wird, wird (unabhängig davon, ob eine Propen-Reaktionsstufe vorausgeht oder nicht) normalerweise ≥ 1 betragen. Üblicherweise wird dieses Verhältnis bei Werten ≤ 3 liegen. Häufig wird das molare Verhältnis von O₂:Acrolein im vorgenannten Beschickungsgasgemisch erfindungsgemäß 1 bis 2 bzw. 1 bis 1,5 betragen. Vielfach wird man das erfindungsgemäße Verfahren mit einem im Reaktionsgasausgangsgemisch 2 (Beschickungsgasgemisch für die Acrolein-Reaktionsstufe) vorliegenden Acrolein:Sauerstoff:Wasserdampf:Inertgas- Volumenverhältnis (NI) von 1:(1 bis 3): (0 bis 20): (3 bis 30), vorzugsweise von 1:(1 bis 3): (0,5 bis 10) : (7 bis 20) ausführen.

Der Acroleinanteil im Beschickungsgasgemisch für die Acrolein-Reaktionsstufe kann z.B. (unabhängig davon, ob eine Propen-Reaktionsstufe vorausgeht oder nicht) bei Werten von 3 oder 6 bis 15 Vol.-%, häufig bei 4 oder 6 bis 10 Vol.-% bzw. 5 bis 8 Vol.-% liegen (jeweils bezogen auf das Gesamtvolumen). Die Belastung des Katalysatorfestbetts (hier ausschließlich reiner Inertabschnitte) mit Beschickungsgasgemisch (Reaktionsgasausgangsgemisch 2) wird bei einem erfindungsgemäßen "Acrolein-zu-Acrylsäure-Verfahren" in typischer Weise wie für die "Propen-zu-Acrolein-Reaktionsstufe" 1000 bis 10000 NI/I•h, meist 1000 bis 5000 NI/I•h, häufig 1500 bis 4000 NI/I•h betragen.

Bei der Ausübung des erfindungsgemäßen Verfahrens wird man frische Katalysatorfestbetten für die Partialoxidation von Propen zu Acrolein normalerweise so betreiben, dass man nach Festlegung der Zusammensetzung des Reaktionsgasausgangsgemischs 1 und Festlegung der Belastung der Katalysatorfestbetten für die Propenpartialoxidation mit Reaktionsgasausgangsgemisch 1 die Temperatur der Katalysatorfestbetten (bzw. die Eintrittstemperatur des Temperiermediums in die Temperierzone des Rohrbündelreaktors) so einstellt, dass der Umsatz U^{Pro} des Propens bei einmaligem Durchgang des Reaktionsgasgemischs 1 durch die Katalysatorfestbetten wenigstens 93 mol.-% beträgt. Bei Verwendung günstiger Katalysatoren sind auch Werte für U^{Pro} ≥ 94 mol.-%, oder ≥ 95 mol.%, oder ≥ 96 mol.-%, oder ≥ 97 mol.-% und häufig sogar mehr möglich.

Bei kontinuierlicher Ausübung der heterogen katalysierten Partialoxidation von Propen zu Acrolein wird man die Zusammensetzung des Reaktionsgasausgangsgemischs 1 und die Belastung der entsprechenden Katalysatorfestbetten mit Reaktionsgasausgangsgemisch 1 im wesentlichen konstant beibehalten (gegebenenfalls wird die Belastung an die fluktuierende Marktnachfrage angepasst). Einem Abfallen der Aktivität der Katalysatorfestbetten über die Zeit wird man unter diesen Produktionsbedingungen normalerweise zunächst dadurch entgegenwirken, dass man von Zeit zu Zeit die Temperatur der Katalysatorfestbetten (die Eintrittstemperatur des Temperiermediums in die Temperaturzone der Rohrbündelreaktoren) erhöht (die Fließgeschwindigkeit des Temperiermediums wird normalerweise im wesentlichen ebenfalls beibehalten), um den Propenumsatz bei einmaligem Durchgang des Reaktionsgasgemischs im angestrebten Zielkorridor (d.h., bei U^{Pro} ≥ 93 mol.-%, bzw. ≥ 94 mol.-%, bzw. ≥ 95 mol.-%, bzw. ≥ 96 mol.-%, bzw. ≥ 97 mol.-%) zu halten.

Vorteilhaft wird man weiterhin so verfahren, dass man die Gasphasenpartialoxidation erfindungsgemäß von Zeit zu Zeit unterbricht, um bei einer Temperatur des Katalysatorfestbetts von 250 bis 550°C ein, wie in der DE-A 10351269 beschrieben, aus molekularem Sauerstoff, Inertgas und gegebenenfalls Wasserdampf bestehendes Gasgemisch G durch das Katalysatorfestbett zu führen. Anschließend wird die Partialoxidation des Propens unter weitgehendem Beibehalt der Verfahrensbedingungen fortgesetzt (die Wiederherstellung der Propenbelastung der Katalysatorfestbetten erfolgt vorzugsweise langsam) und die Temperatur der Katalysatorfestbetten so eingestellt, dass der Propenumsatz den angestrebten Zielwert erreicht. In der Regel wird dieser Temperaturwert bei gleichem Umsatz bei einem etwas niedrigeren Wert liegen als die Temperatur, die das Katalysatorfestbett vor der Unterbrechung der Partialoxidation und der Behandlung mit dem Gasgemisch G aufwies. Von diesem Temperaturwert der Katalysatorfestbetten ausgehend wird die Partialoxidation unter weitgehendem Beibehalt der übrigen Bedingungen fortgesetzt und dabei dem Abfall der Aktivität der Katalysatorfestbetten über die Zeit in zweckmäßiger Weise wiederum dadurch entgegengewirkt, dass man von Zeit zu Zeit die Temperatur der Katalysatorfestbetten erhöht. Innerhalb z.B. eines darauffolgenden Kalenderjahres wird die Partialoxidation erfindungsgemäß zweckmäßig wiederum wenigstens einmal unterbrochen, um das Gasgemisch G durch die Katalysatorfestbetten zu führen. Danach wird die Partialoxidation wie beschrieben wieder aufgenommen u.s.w.. Befriedigt die erzielte Zielproduktselektivität nicht mehr, wird man wie beschrieben z.B. in einer der wenigstens zwei relevanten Oxidationsreaktorstrassen einen Teil- oder Vollkatalysatorwechsel durchführen und anschließend erfindungsgemäß weiterverfahren.

In entsprechender Weise wird man bei der Ausübung des erfindungsgemäßen Verfahrens frische Katalysatorfestbetten für die Partialoxidation von Acrolein zu Acrylsäure normalerweise so betreiben, dass man nach Festlegung des Betriebs dieser Reaktionsstufe und der Zusammensetzung des Reaktionsgasausgangsgemischs 2 und Festlegung der Belastung der entsprechenden Katalysatorfestbetten mit Reaktionsgasausgangsgemisch 2 die Temperatur der Katalysatorfestbetten (bzw. die Eintrittstemperatur des Temperiermediums in die Temperierzone der Rohrbündelreaktoren) so einstellt, dass der Umsatz U^{Acr} des Acroleins bei einmaligem Durchgang des Reaktionsgasausgangsgemischs 2 durch die Katalysatorfestbetten wenigstens 90 mol.-% beträgt. Bei Verwendung günstiger Katalysatoren sind auch Werte für U^{Acr} ≥ 92 mol.-%, oder ≥ 94 mol.-%, oder ≥ 96 mol.-%, oder ≥ 98 mol.-%, und häufig sogar ≥ 99 mol.-% und mehr möglich.

Bei kontinuierlicher Ausübung der heterogen katalysierten Partialoxidation von Acrolein zu Acrylsäure wird man die Zusammensetzung des Reaktionsgasausgangsgemischs 2 und die Belastung der entsprechenden Katalysatorfestbetten mit Reaktionsgasausgangsgemisch 2 im wesentlichen konstant beibehalten (gegebenenfalls wird die Belastung an die fluktuierende Marktnachfrage angepasst). Einem Abfallen der Aktivität der Katalysatorfestbetten über die Zeit wird man unter diesen Produktionsbedingungen normalerweise dadurch entgegenwirken, dass man von Zeit zu Zeit die Temperatur der Katalysatorfestbetten (die Eintrittstemperatur des Temperiermediums in die Temperaturzone des Rohrbündelreaktors) erhöht (die Fließgeschwindigkeit des Temperiermediums wird normalerweise im wesentlichen ebenfalls beibehalten), um den Acroleinumsatz bei einmaligem Durchgang des Beschickungsgasgemischs im angestrebten Zielkorridor (d.h., bei Werten von ≥ 90 mol.-%, bzw. ≥ 92 mol.-%, bzw. ≥ 94 mol.-%, bzw. ≥ 96 mol.-%, oder ≥ 98 mol.-%, bzw. ≥ 99 mol.-%,) zu halten.

Vorteilhaft wird man weiterhin so verfahren, dass man, z.B. bevor die vorgenommene Temperaturerhöhung der Katalysatorfestbetten dauerhaft ≥ 10°C oder ≥ 8°C beträgt (bezogen auf die zuvor eingestellte Temperatur des selben Katalysatorfestbetts), die Gasphasenpartialoxidation wenigstens einmal unterbricht, um bei einer Temperatur der Katalysatorfestbetten von 200 bis 450°C (bei einer zweistufigen Partialoxidation von Propen zu Acrylsäure über die Katalysatorfestbetten der Propenoxidation zu Acrolein führend) das Gasgemisch G durch das Katalysatorfestbett der Partialoxidation von Acrolein zu Acrylsäure zu führen. Anschließend wird die Partialoxidation unter weitgehendem Beibehalt der Verfahrensbedingungen fortgesetzt (die Wiederherstellung der Acroleinbelastung des entsprechenden Katalysatorfestbetts erfolgt vorzugsweise langsam, z.B. so wie in der DE-A 10337788 beschrieben) und die Temperatur der Katalysatorfestbetten so eingestellt, dass der Acroleinumsatz den angestrebten Zielwert erreicht. In der Regel wird dieser Temperaturwert bei gleichem Umsatz bei einem etwas niedrigeren Wert liegen als die Temperatur, die das Katalysatorfestbett vor der Unterbrechung der Partialoxidation und der Behandlung mit dem Gasgemisch G aufwies. Von diesem Temperaturwert des Katalysatorfestbetts ausgehend wird die Partialoxidation des Acroleins unter weitgehendem Beibehalt der übrigen Bedingungen fortgesetzt und dabei dem Abfall der Aktivität der Katalysatorfestbetten über die Zeit in zweckmäßiger Weise wiederum dadurch entgegengewirkt, dass man von Zeit zu Zeit die Temperatur der Katalysatorfestbetten erhöht. Beispielsweise bevor die durchgeführte Temperaturerhöhung der Katalysatorfestbetten dauerhaft ≥ 10°C oder ≥ 8°C beträgt, wird die Partialoxidation wiederum unterbrochen, um das Gasgemisch G (gegebenenfalls über das Katalysatorfestbett einer Propen-Reaktionsstufe führend) durch das Katalysatorfestbett der Acroleinpartialoxidation von Acrolein zu Acrylsäure zu führen. Danach wird die Partialoxidation wie beschrieben wieder aufgenommen usw.. Befriedigt die erzielte Zielproduktselektivität nicht mehr, wird man wie beschrieben z.B. in einer der wenigstens zwei relevanten Oxidationsstrassen einen Teil- oder Vollkatalysatorwechsel durchführen und anschließend erfindungsgemäß weiterverfahren.

Generell sollte eine Acrolein zu Acrylsäure heterogen katalysierte Partialoxidation so betrieben werden, dass der Sauerstoffgehalt im resultierenden Produktgasstrom noch 1,5 bis 3,5 Vol.% beträgt.

Die Abtrennung der Acrylsäure wird man erfindungsgemäß bevorzugt aus dem Gemisch der Produktgasströme vornehmen. Diese Acrylsäureabtrennung und die damit in der Regel einhergehende Kreisgasbildung kann man z.B. wie in den Schriften WO 97/48669, US-A 2004/0242826, WO 01/96271 und US 6,410,785 beschrieben in einer Zielproduktabtrennstrasse vornehmen.

Da die Trennwirkung von Trennkolonnen normalerweise mit zunehmender Anzahl von theoretischen Trennstufen wächst, ist es erfindungsgemäß z.B. möglich, über längere Betriebszeiten mit kleineren (d.h. eine geringere Anzahl an theoretischen Trennstufen aufweisenden) und damit kostengünstigeren Trennkolonnen die erwünschte Reinheit des Roh-Zielproduktes zu erzielen.

Werden n (≥2) Oxidationsreaktorsysteme in erfindungsgemäßer Weise so betrieben, dass der Gemischstrom Zielverbindungen aus allen n-Oxidationsreaktorsystemen enthält, ist es erfindungsgemäß günstig, wenn das Betriebsalter der n Katalysatorbeschickungen der n Oxidationsreaktorsysteme auf der Zeitachse so zeitversetzt liegen, dass der Betriebsaltersunterschied zwischen aufeinanderfolgenden Punkten auf der Zeitachse im wesentlichen gleich groß ist und keine zwei Punkte aufeinanderliegen.

Auf etwas andere Art und Weise ausgedrückt stellt die vorliegende Erfindung ein Verfahren zur Herstellung wenigstens einer organischen Zielverbindung zur Verfügung, das umfasst:
a) die heterogen katalysierte Gasphasen-Partialoxidation wenigstens einer organischen Vorläuferverbindung mit molekularem Sauerstoff in zwei parallel betriebenen, Katalysatorbeschickungen enthaltenden, Oxidationsreaktorsystemen unter Erhalt von zwei die Zielverbindung jeweils enthaltenden Produktgasströmen, von denen jeweils einer auf eines der beiden Oxidationsreaktorsysteme zurückgeht und
b) anschließende Abtrennung der wenigstens einen Zielverbindung aus den zwei Produktgasströmen unter Erzeugung von einem Roh-Zielproduktstrom, bei dem man,
c) vor der Abtrennung die zwei Produktgasströme, oder im Verlauf der Abtrennung zwei auf dem Weg von den zwei Produktgasströmen zu dem einen Roh-Zielproduktstrom gegebenenfalls erzeugte Zielprodukt enthaltende Folgeströme und/oder nach der Abtrennung aus den zwei Produktgasströmen im Verlauf der Abtrennung erzeugte Roh-Zielproduktströme miteinander zu einem Gemischstrom vermischt,
   dadurch gekennzeichnet,
   dass eine der beiden Katalysatorbeschickungen der zwei parallel betriebenen Oxidationsreaktorsysteme wenigstens eine Katalysatorteilmenge enthält, an der die heterogen katalysierte Gasphasen-Partialoxidation bereits länger durchgeführt wird, als an allen Katalysatorteilmengen der anderen Katalysatorbeschickung.

Natürlich kann beim erfindungsgemäßen Verfahren mehr als eine organische Zielverbindung simultan erzeugt werden. Ein Beispiel dafür ist die Propanpartialoxidation, bei der Acrolein und Acrylsäure in der Regel simultan gebildet werden. In ähnlicher Weise können Acrylsäure und Acrylnitril bei einer Partialammoxidation von Propen und/oder Propan dann simultan gebildet werden, wenn der Ammoniakgehalt des Reaktionsgasgemischs entsprechend unterstöchiometrisch gewählt wird. Alternativ kann man auch von einem Reaktionsgasgemisch ausgehen, das mehr als eine Vorläuferverbindung enthält. Abschließend sei festgehalten, dass das Prinzip der erfindungsgemäßen Verfahrensweise analog auch auf katalysierte Veresterungen oder auf andere katalysierte Reaktionen angewendet werden kann. Erfindungsgemäß wesentlich ist auch, dass ein Nebenkomponentenauslaß immer die spezifikationsgerechte Zielverbindung bildet.

Die vorliegende Erfindung umfasst auch Verfahren gemäß den Ansprüchen 23 bis 25. Dabei kann die organische Zielverbindung insbesondere Acrylsäure und/oder Methacrylsäure sein. Als (z. B. ein- oder mehrwertige) Alkohole kommen insbesondere Alkanole, insbesondere C₁- bis C₈-Alkanole (insbesondere einwertige), d. h. z. B. Methanol, Ethanol, 2-Ethylhexanol, n-Butanol und/oder tert.-Butanol in Betracht.

### Beispiele und Vergleichsbeispiele (zweistufige heterogen katalysierte Gasphasen-Partialoxidation von Propen zu Acrylsäure)

### A) Allgemeiner Versuchsaufbau

### I. Reaktoren für die erste Reaktionsstufe von Propen zu Acrolein

Ein Reaktor bestand aus einem doppelwandigen Zylinder aus Edelstahl (zylindrisches Führungsrohr, umgeben von einem zylindrischen Außenbehälter). Die Wanddicken betrugen überall 2 bis 5 mm.

Der Innendurchmesser des äußeren Zylinders betrug 91 mm. Der Innendurchmesser des Führungsrohres betrug ca. 60 mm.

Oben und unten war der doppelwandige Zylinder durch einen Deckel beziehungsweise Boden abgeschlossen.

Das Kontaktrohr (400 cm Gesamtlänge, 26 mm Innendurchmesser, 30 mm Außendurchmesser, 2 mm Wandstärke, Edelstahl) war durch das zylindrische Führungsrohr geführt im zylindrischen Behälter so untergebracht, dass es am oberen bzw. unteren Ende desselben (abgedichtet) durch den Deckel bzw. Boden jeweils gerade herausragte. Das Wärmeaustauschmittel (Salzschmelze, bestehend aus 53 Gew.-% Kaliumnitrat, 40 Gew.-% Natriumnitrit und 7 Gew.-% Natriumnitrat) befand sich im zylindrischen Behälter mit Verbindung in das Führungsrohr. Um über die gesamte Kontaktrohrlänge (400 cm) möglichst gleichmäßige thermische Randbedingungen an der Außenwand des Kontaktrohres zu gewährleisten, wurde das Wärmeaustauschmittel mittels einer Propellerpumpe zum Zweck seiner Temperierung zunächst durch den zylindrischen Behälter und danach zum Zweck der Temperierung des Kontaktrohres durch den Zwischenraum zwischen Führungsrohr und Kontaktrohr gepumpt. Anschließend wurde es in den zylindrischen Behälter zurückgeführt.

Durch eine auf den Außenmantel des zylindrischen Behälters aufgebrachte elektrische Heizung konnte die Temperatur des Wärmeaustauschmittels auf das gewünschte Niveau geregelt werden. Im übrigen bestand Luftkühlung.

| | |
|---|---|
| Reaktorbeschickung: | Über den Erststufenreaktor betrachtet wurden Salzschmelze und das Reaktionsgasausgangsgemisch 1 im Gleichstrom geführt. Das Reaktionsgasausgangsgemisch 1 trat von unten |

| | |
|---|---|
| | in das Kontaktrohr ein. Es wurde jeweils mit einer Temperatur von 165°C ins Kontaktrohr geführt. |
| | Die Salzschmelze trat ebenfalls von unten mit einer Temperatur T^{ein} in das zylindrische Führungsrohr ein und oben mit einer Temperatur T^{aus} aus dem zylindrischen Führungsrohr aus, die bis zu 2°C oberhalb von T^{ein} lag. Die Eintrittstemperatur T^{ein} war stets so bemessen (ca. 320°C), dass sich in allen Fällen ein Propenumsatz von 97,5 ± 0,1 mol-% bei einfachem Durchgang des Reaktionsgasgemischs durchs Kontaktrohr ergab. |
| Kontaktrohrbeschickung: | |
| (von unten nach oben) | Abschnitt A: 90 cm Länge |
| | Vorschüttung aus Steatit-Kugeln eines Durchmessers von 4-5 mm. |
| | Abschnitt B: 100 cm Länge |
| | Katalysatorbeschickung mit einem homogenen Gemisch aus 30 Gew.-% an Steatitringen der Geometrie 5 mm x 3 mm x 2 mm (Außendurchmesser x Länge x Innendurchmesser) und 70 Gew.-% Vollkatalysator aus Abschnitt C. |
| | Abschnitt C: 200 cm Länge |
| | Katalysatorbeschickung mit ringförmigem (5mm x 3 mm x 2 mm = Außendurchmesser x Länge x Innendurchmesser) Vollkatalysator gemäß Beispiel 1 der DE-A 10046957 (Stöchiometrie: |
| | [Bi₂W₂O₉x2WO₃]_{0,5} [Mo₁₂Co_{5,5}Fe_{2,94}Si_{1,59}K_{0,08}Oₓ]₁). |
| | Abschnitt D: 10 cm Länge |
| | Nachschüttung aus Steatit-Ringen der Geometrie 7 mm x 3 mm x 4 mm (Außendurchmesser x Länge x Innendurchmesser) |

Zwei wie vorstehend beschriebene Reaktoren wurden parallel betrieben.

### II. Zwischenkühlung und eventuell Sauerstoffzwischeneinspeisung (Luft als Sekundärgas)

Die die beiden Erststufenreaktoren verlassenden Produktgasströme wurden zum Zweck der Zwischenkühlung (indirekt mittels Luft) gemeinsam durch ein Verbindungsrohr (40 cm Länge, 26 mm Innendurchmesser, 30 mm Außendurchmesser, 2 mm Wandstärke, Edelstahl, umwickelt mit 1 cm Isoliermaterial) geführt, das auf einer Länge von 20 cm zentriert untergebracht, mit einer Inertschüttung aus Steatit-Ringen der Geometrie 7 mm x 3 mm x 4 mm (Außendurchmesser x Länge x Innendurchmesser) beschickt und Y-förmig unmittelbar an die Erststufenkontaktrohre angeflanscht war.

Das Gemisch aus den Produktgasströmen trat in allen Fällen mit einer Temperatur von mehr als 320°C in das Verbindungsrohr ein und verließ es mit einer oberhalb von 200°C und unterhalb von 270°C gelegenen Temperatur.

Am Ende des Verbindungsrohres konnte dem abgekühlten Gemisch aus den Produktgasströmen je nach Bedarf auf den Druck des Gemischstroms komprimierte Luft zudosiert werden. Das resultierende Reaktionsgasgemisch 2 wurde zu gleichen Anteilen unmittelbar in die beiden parallel angeordneten Zweitstufenkontaktrohre geführt, an welchen das oben genannte Verbindungsrohr mit seinem anderen Ende ebenfalls Y-förmig angeflanscht war.

### III. Reaktoren für die zweite Reaktionsstufe von Acrolein zu Acrylsäure

Es wurden Kontaktrohr-Festbettreaktoren verwendet, die mit jenen für die erste Reaktionsstufe baugleich waren. Salzschmelze und Reaktionsgasgemisch wurden über den einzelnen Reaktor betrachtet ebenfalls im Gleichstrom geführt. Die Salzschmelze trat unten in das Führungsrohr ein, das Reaktionsgasausgangsgemisch 2 ebenfalls. Die Eintrittstemperatur T^{ein} der Salzschmelze wurde stets so eingestellt (ca. 26,3°C), dass sich in allen Fällen ein Acroleinumsatz von 99,3 ± 0,1 mol% bei einfachem Durchgang des Reaktionsgasgemischs ergab. T^{aus}der Salzschmelze lag bis zu 2 °C oberhalb von T^{ein}.

Die Kontaktrohrbeschickung (von unten nach oben) war:
Abschnitt A: 70 cm Länge
   Vorschüttung aus Steatit-Ringen der Geometrie 7 mm x 3 mm x 4 mm (Außendurchmesser x Länge x Innendurchmesser).
Abschnitt B: 100 cm Länge
   Katalysatorbeschickung mit einem homogenen Gemisch aus 30 Gew.-% an Steatit-Ringen der Geometrie 7 mm x 3 mm x 4 mm (Außendurchmesser x Länge x Innendurchmesser) und 70 Gew.-% Schalenkatalysator aus Abschnitt C.
Abschnitt C: 200 cm Länge
   Katalysatorbeschickung mit ringförmigem (7 mm x 3 mm x 4 mm = Außendurchmesser x Länge x Innendurchmesser) Schalenkatalysator gemäß Herstellungsbeispiel 5 der DE-A 100 46 928 (Stöchiometrie: Mo₁₂V₃W_{1,2}Cu_{2,4}Oₓ).
Abschnitt D: 30 cm Länge
   Nachschüttung aus Steatit-Kugeln eines Durchmessers von 4-5 mm.

### IV. Abtrennung der Acrylsäure aus dem Gemisch der Produktgasströme der zweiten Reaktionsstufe

Die beiden aus den zweiten Reaktionsstufen kommenden Produktgasströme wurden zusammengeführt und der resultierende Produktgasstrom mittels Wasser das 350 gew.ppm Hydrochinon (HQ) als Polymerisationsinhibitor enthielt (Temperatur = 4°C) in einem Venturi-Abscheider (gleichen im Aufbau den Venturi-Rohren und beschleunigen das Gasgemisch an der engsten Stelle des Venturi-Rohres, spritzen gleichzeitig das Kühlwasser ein und mischen intensiv im hohe Druckverluste ergebenden turbulenten Strömungsfeld; nachgeschaltete Abscheider scheiden die Flüssigphase ab) einer Direktkühlung unterworfen und das dabei resultierende Gemisch einem Flüssigphasenabscheider zugeführt. Über einen Wärmetauscher wurde die abgeschiedene wässrige Phase in den Venturi-Abscheider rückgeführt (360 l/h). Überschüssige wässrige Phase wurde kontinuierlich abgeführt.

Der auf eine Temperatur von 30°C abgekühlte Gemischgasstrom wurde von unten in eine Absorptionskolonne geführt, die 11 Glocken-Böden in äquidistanter Anordnung enthielt (Bodenabstand: 54 mm; Bodendurchmesser: 12 mm) und dem Gegenstrom von 1,10 kg/h mittels HQ stabilisierten Wassers als Absorptionsmittel ausgesetzt (am Kolonnenkopf mit einer Temperatur von 2°C aufgegeben). Dem Kolonnensumpf wurden stündlich 1,7 kg einer ca. 40 gew.-%igen wässrigen Acrylsäure entnommen. Das die Absorptionskolonne am Kopf verlassende Restgas wurde je nach Bedarf der Verbrennung zugeführt und/oder als Kreisgas zur Bildung des Reaktionsgasausgangsgemisches 1 verwendet (über einen Kompressor zu den Erststufenreaktoren rückgeführt).

### B) In Abhängigkeit von den Katalysatorbeschickungen und der Zusammensetzung der Reaktionsgasausgangsgemische erzielte Ergebnisse

### Vergleichsbeispiel 1

Beide Erststufenreaktoren und beide Zweitstufenreaktoren waren mit frischen Katalysatoren beschickt.

Die Zusammensetzung des beiden Erststufenreaktoren zugeführten Reaktionsgasausgangsgemischs war:

| | |
|---|---|
| 5,3 Vol.-% | Propen, |
| 2,4 Vol.-% | Wasser, |
| 0,7 Vol.-% | von Propen, Wasser, Sauerstoff und Stickstoff verschiedene Bestandteile, |
| | molekulare Sauerstoff in einer Menge, dass das molare Verhältnis von enthaltenem molekularem Sauerstoff zu enthaltenem Propen 1,52 betrug, und |
| als Restmenge bis 100 Vol.-% molekularer Stickstoff. | |

Die Propenbelastung der beiden Erststufenkontaktrohrschickungen betrug 110 NI/I•h. Frischsauerstoffquelle war Luft. Je Vol.-% Frischpropen enthielt das Reaktionsgasausgangsgemisch 8 Vol.-% Kreisgas. Dem Gemisch aus den Produktgasströmen der ersten Reaktionsstufen wurde Sekundärluft zudosiert. Ihre Menge wurde so bemessen, dass das Verhältnis von Sekundärluft/Frischpropen Geweils in NI) 1,45 betrug. Damit ergab sich ein Restsauerstoffgehalt von 3,0 Vol.% im Produktgas der zweiten Reaktionsstufe. Die Versuchsanlage wurde so über 28 Wochen kontinuierlich betrieben. Die in Abhängigkeit von der Betriebsdauer (in Wochen) erzielte Selektivität der Zielproduktbildung (Acrylsäure) S^{AA} sowie die in Abhängigkeit von der Betriebsdauer erzielten Nebenproduktselektivitäten für Essigsäure (S^{HAC}) und Formaldehyd (S^{F}), jeweils in mol-% bezogen auf umgesetztes Propen, zeigt die nachfolgende Tabelle 1 (stets bezogen auf den Ausgang der zweiten Reaktionsstufe). Ebenso zeigt die Tabelle 1 das molare Verhältnis V von Acrylsäure zu Essigsäure im wässrigen Absorbat.

**Tabelle 1**

| Betriebsdauer | S^{AA} | S^{HAc} | S^{F} | V |
|---|---|---|---|---|
| Inbetriebnahme | 86,8 | 2 | 1,2 | 43,4 |
| 4 | 88 | 1,9 | 1,1 | 46,3 |
| 8 | 88,7 | 1,8 | 1,05 | 49,3 |
| 12 | 89,4 | 1,75 | 0,95 | 51,4 |
| 16 | 89,9 | 1,7 | 0,9 | 52,4 |
| 20 | 90,3 | 1,65 | 0,85 | 54,7 |
| 24 | 90,8 | 1,62 | 0,82 | 56,0 |
| 28 | 91,2 | 1,6 | 0,8 | 57 |

Die weitere Auftrennung des wässrigen , das Zielprodukt enthaltenden, Absorbats, muss für ein Verhältnis V = 43,4 ausgelegt sein.

### Beispiel 1

Zunächst wurde Vergleichsbeispiel 1 wiederholt. Nachdem die Wiederholung des Vergleichsbeispiels 1 ohne Unterbrechung 2200 kg Acrylsäure produziert hatte, wurde der Betrieb unterbrochen und nur die Katalysatorbeschickung einer der beiden Oxidationsreaktorstrassen in beiden Stufen durch eine entsprechende, aber frische Katalysatorbeschickung ersetzt. Dann wurde wie im Vergleichsbeispiel 1 beschrieben weiterverfahren. Die nach der Unterbrechung in Abhängigkeit von der Betriebsdauer (Wochen) erzielten Ergebnisse zeigt die Tabelle 2.

**Tabelle 2**

| Betriebsdauer | S^{AA} | s^{HAc} | S^{F} | V |
|---|---|---|---|---|
| Inbetriebnahme | 89 | 1,7 | 1,0 | 52,4 |
| 4 | 89,6 | 1,6 | 0,9 | 56 |
| 8 | 89,9 | 1,6 | 0,9 | 56,2 |
| 12 | 90,2 | 1,6 | 0,9 | 56,4 |
| 16 | 90,5 | 1,5 | 0,8 | 60,3 |
| 20 | 90,7 | 1,5 | 0,8 | 60,5 |
| 24 | 91,0 | 1,5 | 0,8 | 60,7 |
| 28 | 91,2 | 1,5 | 0,8 | 60,8 |

Die weitere Auftrennung des wässrigen, das Zielprodukt enthaltenden, Absorbats muss lediglich für ein Verhältnis V = 52,4 ausgelegt sein.

### Vergleichsbeispiel 2

Das Vergleichsbeispiel 1 wurde wiederholt (die Verdünnung im Abschnitt B der zweiten Reaktionsstufe wurde jedoch zu 40 Gew.-% gewählt), die Zusammensetzung des Beschickungsgemischs für die ersten Reaktionsstufen wurde jedoch wie folgt gewählt:

| | |
|---|---|
| 7,3 Vol-% | Propen, |
| 10 Vol.% | Wasser, |
| 0,7 Vol.-% | von Propen, Wasser, Sauerstoff und Stickstoff verschiedene Bestandteile, |
| | molekularer Sauerstoff in einer Menge, dass das molare Verhältnis von enthaltenem molekularem Sauerstoff zu enthaltenem Propen 1,73 betrug, und |
| als Restmenge bis 100 Vol.-% molekularer Stickstoff. | |

Sekundärluft wurde nicht zudosiert. Je Vol.-% Frischpropen enthielt das Reaktionsgasausgangsgemisch 3,5 Vol.-% Kreisgas.

Tabelle 3 enthält die in Abhängigkeit von der Betriebszeit (in Wochen) resultierenden Ergebnisse.

**Tabelle 3**

| Betriebsdauer | S^{AA} | S^{Hac} | S^{F} | V |
|---|---|---|---|---|
| Inbetriebnahme | 86,7 | 2,25 | 1,35 | 38,5 |
| 4 | 87,9 | 2,15 | 1,25 | 40,9 |
| 8 | 88,7 | 2,05 | 1,2 | 43,3 |
| 12 | 89,3 | 2 | 1,1 | 44,7 |
| 16 | 89,5 | 1,95 | 1,05 | 45,9 |
| 20 | 90,1 | 1,9 | 1 | 47,4 |
| 24 | 90,3 | 1,87 | 0,97 | 48,3 |
| 28 | 90,6 | 1,85 | 0,95 | 49,0 |

Die weitere Auftrennung des wässrigen, das Zielprodukt enthaltenden, Absorbats muss für ein Verhältnis V = 38,5 ausgelegt sein.

### Beispiel 2

Zunächst wurde Vergleichsbeispiel 2 wiederholt. Nachdem die Wiederholung des Vergleichsbeispiels 2 ohne Unterbrechung 2200 kg Acrylsäure produziert hatte, wurde der Betrieb unterbrochen und nur die Katalysatorbeschickung einer der beiden Oxidationsreaktorstrassen in beiden Stufen durch eine entsprechende, aber frische Katalysatorbeschickung ersetzt. Dann wurde wie im Vergleichsbeispiel 2 beschrieben weiterverfahren. Die nach der Unterbrechung in Abhängigkeit von der Betriebsdauer (Wochen) erzielten Ergebnis zeigt die Tabelle 4.

**Tabelle 4**

| Betriebsdauer | S^{AA} | S^{Hac} | S^{F} | V |
|---|---|---|---|---|
| Inbetriebnahme | 88,8 | 2,1 | 1,2 | 42,3 |
| 4 | 89,4 | 2,0 | 1,1 | 44,7 |
| 8 | 89,8 | 1,9 | 1,1 | 47,3 |
| 12 | 90,1 | 1,9 | 1,0 | 47,4 |
| 16 | 90,2 | 1,9 | 1,0 | 47,5 |
| 20 | 90,5 | 1,8 | 1,0 | 50,3 |
| 24 | 90,6 | 1,8 | 0,9 | 50,3 |
| 28 | 90,8 | 1,8 | 0,9 | 50,4 |

Die weitere Auftrennung des wässrigen, das Zielprodukt enthaltenden, Absorbats muss lediglich für ein Verhältnis V = 42,3 ausgelegt sein.

Eine Vorrichtung gemäß Anspruch 22 eignet sich zur Durchführung des erfindungsgemäßen Verfahrens.

Verfahren gemäß Anspruch 14 bis 20 bilden z.B. die Grundlage für eine Anwendung der erfindungsgemäßen Verfahrensweise.

## Patentansprüche

1. Verfahren zur Herstellung wenigstens einer organischen Zielverbindung durch
a) heterogen katalysierte Gasphasen-Partialoxidation wenigstens einer organischen Vorläuferverbindung mit molekularem Sauerstoff in wenigstens zwei parallel betriebenen, Katalysatorbeschickungen enthaltenden, Oxidationsreaktorsystemen unter Erhalt von wenigstens zwei die Zielverbindung jeweils enthaltenden und jeweils auf eines der wenigstens zwei Oxidationsreaktorsysteme zurückgehenden Produktgasströmen und
b) anschließende Abtrennung der wenigstens einen Zielverbindung aus den wenigstens zwei Produktgasströmen unter Erzeugung von wenigstens einem Roh-Zielproduktstrom, bei dem man
c) vor der Abtrennung wenigstens zwei der wenigstens zwei Produktgasströme, oder im Verlauf der Abtrennung wenigstens zwei der auf dem Weg von den wenigstens zwei Produktgasströmen zu dem wenigstens einen Roh-Zielproduktstrom gegebenenfalls erzeugten Zielprodukt enthaltenden Folgeströme und/oder nach der Abtrennung aus den wenigstens zwei Produktgasströmen im Verlauf der Abtrennung gegebenenfalls erzeugte Roh-Zielproduktströme miteinander zu einem Gemischstrom vermischt,
**dadurch gekennzeichnet,**
**dass** wenigstens eine der Katalysatorbeschickungen der wenigstens zwei parallel betriebenen Oxidationsreaktorsysteme, an denen die im Gemischstrom enthaltenen Zielverbindungen gebildet wurden, wenigstens eine Katalysatorteilmenge enthält, an der die heterogen katalysierte GasphasenPartialoxidation bereits länger durchgeführt wird, als an allen Katalysatorteilmengen der wenigstens einen anderen Katalysatorbeschickung.

2. Verfahren nach Anspruch 1, **dadurch gekennzeichnet, dass** die wenigstens eine der Katalysatorbeschickungen der wenigstens zwei parallel betriebenen Oxidationsreaktorsysteme, an denen die im Gemischstrom enthaltenen Zielverbindungen gebildet wurden, wenigstens eine Katalysatorteilmenge enthält, an der die heterogen katalysierte Gasphasen-Partialoxidation bereits wenigstens 30 Kalendertage länger durchgeführt wird, als an allen Katalysatorteilmengen der wenigstens einen anderen Katalysatorbeschickung.

3. Verfahren nach Anspruch 1 oder 2, **dadurch gekennzeichnet, dass** die heterogen katalysierte Gasphasen-Partialoxidation in zwei parallel betriebenen Oxidationsreaktorsystemen und die Abtrennung der Zielverbindung aus dem Gemischstrom der beiden Produktströme vorgenommen wird.

4. Verfahren nach einem der Ansprüche 1 bis 3, **dadurch gekennzeichnet, dass** die wenigstens eine organische Vorläuferverbindung Propylen, iso-Buten, Ethylen, Ethan, Propan, iso-Butan, Acrolein, Methacrolein, Butadien, o-, m-, p-Xylol und/oder Naphtalin ist.

5. Verfahren nach einem der Ansprüche 1 bis 4, **dadurch gekennzeichnet, dass** die heterogen katalysierte Gasphasen-Partialoxidation die zweistufige heterogen katalysierte Partialoxidation von Propen zu Acrylsäure ist.

6. Verfahren nach einem der Ansprüche 1 bis 4, **dadurch gekennzeichnet, dass** die heterogen katalysierte Gasphasen-Partialoxidation die einstufige heterogen katalysierte Partialoxidation von Propan zu Acrylsäure ist.

7. Verfahren nach einem der Ansprüche 1 bis 4, **dadurch gekennzeichnet, dass** die heterogen katalysierte Gasphasen-Partialoxidation die zweistufige heterogen katalysierte Partialoxidation von iso-Buten zu Methacrylsäure ist.

8. Verfahren nach einem der Ansprüche 1 bis 7, **dadurch gekennzeichnet, dass** die wenigstens zwei parallel betriebenen Oxidationsraktorsysteme aus zwei parallel betriebenen Tandem-Rohrbündelreaktoranordnungen bestehen.

9. Verfahren nach Anspruch 5, **dadurch gekennzeichnet, dass** die Katalysatoren der ersten Reaktionsstufe Mo, Bi und Fe enthaltende Multimetalloxidmassen aufweisen.

10. Verfahren nach einem der Ansprüche 5 oder 9, **dadurch gekennzeichnet, dass** die Katalysatoren der zweiten Reaktionsstufe Mo und V enthaltende Multimetalloxidmassen sind.

11. Verfahren nach einem der Ansprüche 1 bis 10, **dadurch gekennzeichnet, dass** die Abtrennung der wenigstens einen Zielverbindung aus den wenigstens zwei Produktgasströmen eine fraktionierende Kondensation umfasst.

12. Verfahren nach einem der Ansprüche 1 bis 10, **dadurch gekennzeichnet, dass** die Abtrennung der wenigstens einen Zielverbindung aus den wenigstens zwei Produktgasströmen eine Absorption umfasst.

13. Verfahren nach einem der Ansprüche 1 bis 10, **dadurch gekennzeichnet, dass** die Abtrennung der wenigstens einen Zielverbindung aus den wenigstens zwei Produktgasströmen eine Rektifikation, oder eine Kristallisation oder beides umfasst.

14. Verfahren zur Herstellung wenigstens einer organischen Zielverbindung durch heterogen katalysierte Gasphasen-Partialoxidation wenigstens einer organischen Vorläuferverbindung mit molekularem Sauerstoff in wenigstens zwei parallel betriebenen, Katalysatorbeschickungen enthaltenden, Oxidationsreaktorsystemen unter Erhalt von wenigstens zwei die Zielverbindung jeweils enthaltenden und jeweils auf eines der wenigstens zwei Oxidationsreaktorsysteme zurückgehenden Produktgasströmen, **dadurch gekennzeichnet, dass** man zunächst einen Gesamtstrom an die wenigstens eine organische Vorläuferverbindung enthaltendem Reaktionsgasausgangsgemisch erzeugt und diesen anschließend über ein Verteilersystem den wenigstens zwei parallel betriebenen Oxidationsreaktorsystemen mit der Maßgabe zuführt, dass wenigstens eine der Katalysatorbeschickungen der wenigstens zwei parallel betriebenen Oxidationsreaktorsysteme wenigstens eine Katalysatorteilmenge enthält, an der die heterogen katalysierte Gasphasen-Partialoxidation bereits länger durchgeführt wird, als an allen Katalysatorteilmengen der wenigstens einen anderen Katalysatorbeschickung.

15. Verfahren nach Anspruch 14, **dadurch gekennzeichnet, dass** die wenigstens eine der Katalysatorbeschickungen der wenigstens zwei parallel betriebenen Oxidationsreaktorsysteme wenigstens eine Katalysatorteilmenge enthält, an der die heterogen katalysierte Gasphasen-Partialoxidation bereits wenigstens 30 Kalendertage länger durchgeführt wird, als an allen Katalysatorteilmengen der wenigstens einen anderen Katalysatorbeschickung.

16. Verfahren nach Anspruch 14 oder 15, **dadurch gekennzeichnet, dass** die wenigstens eine organische Vorläuferverbindung Propylen, iso-Buten, Ehtylen, Ethan, Propan, iso-Butan, Acrolein, Methacrolein, Butadien, o-, m-, p-Xylol und/oder Naphtalin ist.

17. Verfahren nach einem der Ansprüche 14 bis 16, **dadurch gekennzeichnet, dass** die heterogen katalysierte Gasphasen-Partialoxidation die zweistufige heterogen katalysierte Partialoxidation von Propen zu Acrylsäure ist.

18. Verfahren nach einem der Ansprüche 14 bis 16, **dadurch gekennzeichnet, dass** die heterogen katalysierte Gasphasen-Partialoxidation die einstufige heterogen katalysierte Partialoxidation von Propan zu Acrylsäure ist.

19. Verfahren nach einem der Ansprüche 14 bis 18, **dadurch gekennzeichnet, dass** die wenigstens zwei parallel betriebenen Oxidationsreaktorsysteme aus zwei parallel betriebenen Tandem-Rohrbündelreaktoranordnungen bestehen.

20. Verfahren nach Anspruch 17, **dadurch gekennzeichnet, dass** die Katalysatoren der ersten Reaktionsstufe Mo, Bi und Fe enthaltende Multimetalloxidmassen und die Katalysatoren der zweiten Reaktionsstufe Mo und V enthaltende Multimetalloxidmassen sind.

21. Verfahren nach Anspruch 5 oder 17, **dadurch gekennzeichnet, dass** das Propen den wenigstens zwei parallel betriebenen Oxidationsreaktorsystemen aus einer in einem Dehydrierreaktor durchgeführten heterogen katalysierten Dehydrierung und/oder Oxydehydrierung von Propan zu Propen zugeführt wird.

22. Vorrichtung, umfassend zwei Oxidationsreaktorsysteme, die mit Katalysatoren beschickt sind, die sich zur Herstellung einer organischen Zielverbindung durch heterogen katalysierte Partialoxidation einer organischen Vorläuferverbindung eignen und an deren Ausgang sich jeweils eine Leitung für die Abfuhr des die Zielverbindung enthaltenden Produktgasstroms aus dem jeweiligen Oxidationsreaktorsystem befindet, die mit zunehmender Entfernung von den beiden Oxidationsreaktorsystemen zu einer Produktgasleitung zusammengeführt werden, welche zu einer Vorrichtung führt, in der der Produktgasstrom teilweise oder vollständig kondensiert werden kann, **dadurch gekennzeichnet, dass** die Katalysatorbeschickung eines der beiden Oxidationsreaktorsysteme wenigstens eine Katalysatorteilmenge enthält, an der bereits mehr Zielprodukt erzeugt worden ist, als an den Katalysatorteilmengen der Katalysatorbeschickung des anderen Oxidationsreaktorsystems.

23. Verfahren nach einem der Ansprüche 1 bis 21, **dadurch gekennzeichnet, dass** die wenigstens eine organische Zielverbindung wenigstens eine ethylenisch ungesättigte Kohlenstoff-Kohlenstoff-Doppelbindung aufweist und sich ein Verfahren zur Herstellung von Polymerisaten anschließt, in die von der wenigstens einen organischen Zielverbindung einpolymerisiert wird.

24. Verfahren nach einem der Ansprüche 1 bis 21, **dadurch gekennzeichnet, dass** die wenigstens eine organische Zielverbindung wenigstens eine Carboxylgruppe aufweist und sich ein Verfahren zur Herstellung von Ester der organischen Zielverbindung durch Umsetzung derselben mit einem Alkohol anschließt.

25. Verfahren nach Anspruch 24, **dadurch gekennzeichnet, dass** die wenigstens eine organische Zielverbindung zusätzlich wenigstens eine ethylenisch ungesättigte Kohlenstoff-Kohlenstoff-Doppelbindung aufweist und sich ein Verfahren zur Herstellung von Polymerisaten anschließt, in die wenigstens ein Ester einpolymerisiert wird.

## Claims

1. A process for preparing at least one organic target compound by
a) heterogeneously catalyzed gas phase partial oxidation of at least one organic precursor compound with molecular oxygen in at least two oxidation reactor systems which comprise catalyst charges and are operated in parallel to obtain at least two product gas streams each comprising the target compound and each stemming from one of the at least two oxidation reactor systems and
b) subsequent removal of the at least one target compound from the at least two product gas streams to obtain at least one crude target product stream, by
c) before the removal, mixing together at least two of the at least two product gas streams, or, in the course of the removal, mixing together at least two of any target product-comprising subsequent streams obtained on the route from the at least two product gas streams to the at least one crude target product stream, and/or, after the removal from the at least two product gas streams, mixing together any crude target product streams obtained in the course of the removal, to form a mixture stream,
wherein
at least one of the catalyst charges of the at least two oxidation reactor systems operated in parallel, over which the target compounds comprised in the mixture stream were formed, comprises at least one portion of catalyst over which the heterogeneously catalyzed gas phase partial oxidation has already been carried out longer than over all portions of catalyst of the at least one other catalyst charge.

2. The process according to claim 1, wherein the at least one of the catalyst charges of the at least two oxidation reactor systems operated in parallel, over which the target compounds comprised in the mixture stream were formed, comprises at least one portion of catalyst over which the heterogeneously catalyzed gas phase partial oxidation has already been carried out for at least 30 calendar days longer than over all portions of catalyst of the at least one other catalyst charge.

3. The process according to claim 1 or 2, wherein the heterogeneously catalyzed gas phase partial oxidation is undertaken in two oxidation reactor systems operated in parallel, and the target compound is removed from the mixture stream of the two product streams.

4. The process according to any of claims 1 to 3, wherein the at least one organic precursor compound is propylene, isobutene, ethylene, ethane, propane, isobutane, acrolein, methacrolein, butadiene, o-, m-, p-xylene and/or naphthalene.

5. The process according to any of claims 1 to 4, wherein the heterogeneously catalyzed gas phase partial oxidation is the two-stage heterogeneously catalyzed partial oxidation of propene to acrylic acid.

6. The process according to any of claims 1 to 4, wherein the heterogeneously catalyzed gas phase partial oxidation is the one-stage heterogeneously catalyzed partial oxidation of propane to acrylic acid.

7. The process according to any of claims 1 to 4, wherein the heterogeneously catalyzed gas phase partial oxidation is the two-stage heterogeneously catalyzed partial oxidation of isobutene to methacrylic acid.

8. The process according to any of claims 1 to 7, wherein the at least two oxidation reactor systems operated in parallel consist of two tandem tube bundle reactor arrangements operated in parallel.

9. The process according to claim 5, wherein the catalysts of the first reaction stages comprise multimetal oxide compositions comprising Mo, Bi and Fe.

10. The process according to either of claims 5 or 9, wherein the catalysts of the second reaction stage are multimetal oxide compositions comprising Mo and V.

11. The process according to any of claims 1 to 10, wherein the removal of the at least one target compound from the at least two product gas streams comprises a fractional condensation.

12. The process according to any of claims 1 to 10, wherein the removal of the at least one target compound from the at least two product gas streams comprises an absorption.

13. The process according to any of claims 1 to 10, wherein the removal of the at least one target compound from the at least two product gas streams comprises a rectification, or a crystallization or both.

14. A process for preparing at least one organic target compound by heterogeneously catalyzed gas phase partial oxidation of at least one organic precursor compound with molecular oxygen in at least two oxidation reactor systems which comprise catalyst charges and are operated in parallel to obtain at least two product gas streams each comprising the target compound and each stemming from one of the at least two oxidation reactor systems, wherein an overall stream of starting reaction gas mixture comprising the at least one organic precursor compound is initially obtained and this is subsequently fed via a distributor system to the at least two oxidation reactor systems operated in parallel, with the proviso that at least one of the catalyst charges of the at least two oxidation reactor systems operated in parallel comprises at least one portion of catalyst over which the heterogeneously catalyzed gas phase partial oxidation has already been carried out for longer than over all portions of catalyst of the at least one other catalyst charge.

15. The process according to claim 14, wherein the at least one of the catalyst charges of the at least two oxidation reactor systems operated in parallel comprises at least one portion of catalyst over which the heterogeneously catalyzed gas phase partial oxidation has already been carried out for at least 30 calendar days longer than over all portions of catalyst of the at least one other catalyst charge.

16. The process according to claim 14 or 15, wherein the at least one organic precursor compound is propylene, isobutene, ethylene, ethane, propane, isobutane, acrolein, methacrolein, butadiene, o-, m-, p-xylene and/or naphthalene.

17. The process according to any of claims 14 to 16, wherein the heterogeneously catalyzed gas phase partial oxidation is the two-stage heterogeneously catalyzed partial oxidation of propene to acrylic acid.

18. The process according to any of claims 14 to 16, wherein the heterogeneously catalyzed gas phase partial oxidation is the one-stage heterogeneously catalyzed partial oxidation of propane to acrylic acid.

19. The process according to any of claims 14 to 18, wherein the at least two oxidation reactor systems operated in parallel consist of two tandem tube bundle reactor arrangements operated in parallel.

20. The process according to claim 17, wherein the catalysts of the first reaction stage are multimetal oxide compositions comprising Mo, Bi and Fe, and the catalysts of the second reaction stage are multimetal oxide compositions comprising Mo and V.

21. The process according to claim 5 or 17, wherein the propene is fed to the at least two oxidation reactor systems operated in parallel from a heterogeneously catalyzed dehydrogenation and/or oxydehydrogenation of propane to propene carried out in a dehydrogenation reactor.

22. An apparatus comprising two oxidation reactor systems which are charged with catalysts which are suitable for the preparation of an organic target compound by heterogeneously catalyzed partial oxidation of an organic precursor compound and at whose outlet is disposed in each case a line for the removal of the product gas stream comprising the target compound from the particular oxidation reactor system, which lines are combined with increasing distance from the two oxidation reactor systems to give a product gas line which leads to an apparatus in which the product gas stream can be condensed partly or fully, wherein the catalyst charge of one of the two oxidation reactor systems comprises at least one portion of catalyst over which more target product has already been generated than over the portions of catalyst of the catalyst charge of the other oxidation reactor system.

23. The process according to any of claims 1 to 21, wherein the at least one organic target compound has at least one ethylenically unsaturated carbon-carbon double bond and which is followed by a process for preparing polymers in which at least one organic target compound is polymerized.

24. The process according to any of claims 1 to 21, wherein the at least one organic target compound has at least one carboxyl group and which is followed by a process for preparing esters of the organic target compound by reacting them with an alcohol.

25. The process according to claim 24, wherein the at least one organic target compound additionally has at least one ethylenically unsaturated carbon-carbon double bond and which is followed by a process for preparing polymers, into which at least one ester is polymerized.

## Revendications

1. Procédé de fabrication d'au moins un composé cible organique par
a) oxydation partielle en phase gazeuse sous catalyse hétérogène d'au moins un composé précurseur organique avec de l'oxygène moléculaire dans au moins deux systèmes de réacteur d'oxydation exploités en parallèle, contenant des garnissages catalytiques, pour obtenir au moins deux courants gazeux de produits contenant chacun le composé cible et dérivant chacun d'un des au moins deux systèmes de réacteur d'oxydation, puis
b) séparation du au moins un composé cible des au moins deux courants gazeux de produits pour former au moins un courant de produit cible brut, selon lequel
c) avant la séparation, au moins deux des au moins deux courants gazeux de produits, ou, au cours de la séparation, au moins deux des courants dérivés contenant le produit cible éventuellement formés pendant la transformation des au moins deux courants gazeux de produits en le au moins un courant de produit cible brut, et/ou, après la séparation, les courants de produit cible brut éventuellement formés au cours de la séparation à partir des au moins deux courants gazeux de produits sont mélangés les uns avec les autres en un courant de mélange,
**caractérisé en ce que**
au moins un des garnissages catalytiques des au moins deux systèmes de réacteur d'oxydation exploités en parallèle, sur lequel les composés cibles contenus dans le courant de mélange ont été formés, contient au moins une quantité partielle de catalyseur, sur laquelle l'oxydation partielle en phase gazeuse sous catalyse hétérogène est déjà réalisée plus longtemps que sur toutes les quantités partielles de catalyseur du au moins un autre garnissage catalytique.

2. Procédé selon la revendication 1, **caractérisé en ce qu'**au moins un des garnissages catalytiques des au moins deux systèmes de réacteur d'oxydation exploités en parallèle, sur lequel les composés cibles contenus dans le courant de mélange ont été formés, contient au moins une quantité partielle de catalyseur, sur laquelle l'oxydation partielle en phase gazeuse sous catalyse hétérogène est déjà réalisée pendant au moins 30 jours de plus que sur toutes les quantités partielles de catalyseur du au moins un autre garnissage catalytique.

3. Procédé selon la revendication 1 ou 2, **caractérisé en ce que** l'oxydation partielle en phase gazeuse sous catalyse hétérogène est réalisée dans deux systèmes de réacteur d'oxydation exploités en parallèle et la séparation du composé cible est réalisée à partir du courant de mélange des deux courants de produits.

4. Procédé selon l'une quelconque des revendications 1 à 3, **caractérisé en ce que** le au moins un composé précurseur organique est le propylène, l'isobutène, l'éthylène, l'éthane, le propane, l'isobutane, l'acroléine, la méthacroléine, le butadiène, l'o-, m-, p-xylène et/ou la naphtaline.

5. Procédé selon l'une quelconque des revendications 1 à 4, **caractérisé en ce que** l'oxydation partielle en phase gazeuse sous catalyse hétérogène est l'oxydation partielle sous catalyse hétérogène à deux étapes de propène en acide acrylique.

6. Procédé selon l'une quelconque des revendications 1 à 4, **caractérisé en ce que** l'oxydation partielle en phase gazeuse sous catalyse hétérogène est l'oxydation partielle sous catalyse hétérogène à une étape de propane en acide acrylique.

7. Procédé selon l'une quelconque des revendications 1 à 4, **caractérisé en ce que** l'oxydation partielle en phase gazeuse sous catalyse hétérogène est l'oxydation partielle sous catalyse hétérogène à deux étapes d'isobutène en acide méthacrylique.

8. Procédé selon l'une quelconque des revendications 1 à 7, **caractérisé en ce que** les au moins deux systèmes de réacteur d'oxydation exploités en parallèle sont constitués de deux agencements de réacteur à faisceau de tubes en tandem exploités en parallèle.

9. Procédé selon la revendication 5, **caractérisé en ce que** les catalyseurs de la première étape de réaction comprennent des masses d'oxyde de plusieurs métaux contenant Mo, Bi et Fe.

10. Procédé selon l'une quelconque des revendications 5 ou 9, **caractérisé en ce que** les catalyseurs de la seconde étape de réaction sont des masses d'oxyde de plusieurs métaux contenant Mo et V.

11. Procédé selon l'une quelconque des revendications 1 à 10, **caractérisé en ce que** la séparation du au moins un composé cible à partir des au moins deux courants gazeux de produits comprend une condensation fractionnée.

12. Procédé selon l'une quelconque des revendications 1 à 10, **caractérisé en ce que** la séparation du au moins un composé cible à partir des au moins deux courants gazeux de produits comprend une absorption.

13. Procédé selon l'une quelconque des revendications 1 à 10, **caractérisé en ce que** la séparation du au moins un composé cible à partir des au moins deux courants gazeux de produits comprend une rectification ou une cristallisation ou les deux.

14. Procédé de fabrication d'au moins un composé cible organique par oxydation partielle en phase gazeuse sous catalyse hétérogène d'au moins un composé précurseur organique avec de l'oxygène moléculaire dans au moins deux systèmes de réacteur d'oxydation exploités en parallèle, contenant des garnissages catalytiques, pour obtenir au moins deux courants gazeux de produits contenant chacun le composé cible et dérivant chacun d'un des au moins deux systèmes de réacteur d'oxydation, **caractérisé en ce qu'**un courant total est tout d'abord formé à partir du mélange gazeux réactionnel initial contenant au moins un composé précurseur organique, puis celui-ci est introduit par un système de distribution dans les au moins deux systèmes de réacteur d'oxydation exploités en parallèle, à condition qu'au moins un des garnissages catalytiques des au moins deux systèmes de réacteur d'oxydation exploités en parallèle contienne au moins une quantité partielle de catalyseur sur laquelle l'oxydation partielle en phase gazeuse sur catalyse hétérogène est déjà réalisée plus longtemps que sur toutes les quantités partielles de catalyseur du au moins un autre garnissage catalytique.

15. Procédé selon la revendication 14, **caractérisé en ce qu'**au moins un des garnissages catalytiques des au moins deux systèmes de réacteur d'oxydation exploités en parallèle contient au moins une quantité partielle de catalyseur sur laquelle l'oxydation partielle en phase gazeuse sur catalyse hétérogène est déjà réalisée pendant au moins 30 jours de plus que sur toutes les quantités partielles de catalyseur du au moins un autre garnissage catalytique.

16. Procédé selon la revendication 14 ou 15, **caractérisé en ce que** le au moins un composé précurseur organique est le propylène, l'isobutène, l'éthylène, l'éthane, le propane, l'isobutane, l'acroléine, la méthacroléine, le butadiène, l'o-, m-, p-xylène et/ou la naphtaline.

17. Procédé selon l'une quelconque des revendications 14 à 16, **caractérisé en ce que** l'oxydation partielle en phase gazeuse sous catalyse hétérogène est l'oxydation partielle sous catalyse hétérogène à deux étapes de propène en acide acrylique.

18. Procédé selon l'une quelconque des revendications 14 à 16, **caractérisé en ce que** l'oxydation partielle en phase gazeuse sous catalyse hétérogène est l'oxydation partielle sous catalyse hétérogène à une étape de propane en acide acrylique.

19. Procédé selon l'une quelconque des revendications 14 à 18, **caractérisé en ce que** les au moins deux systèmes de réacteur d'oxydation exploités en parallèle sont constitués de deux agencements de réacteur à faisceau de tubes en tandem exploités en parallèle.

20. Procédé selon la revendication 17, **caractérisé en ce que** les catalyseurs de la première étape de réaction sont des masses d'oxyde de plusieurs métaux contenant Mo, Bi et Fe, et les catalyseurs de la seconde étape de réaction sont des masses d'oxyde de plusieurs métaux contenant Mo et V.

21. Procédé selon la revendication 5 ou 17, **caractérisé en ce que** le propène est introduit dans les au moins deux systèmes de réacteur d'oxydation exploités en parallèle à partir d'une déshydrogénation et/ou d'une oxydéshydrogénation de propane en propène sous catalyse hétérogène réalisée dans un réacteur de déshydrogénation.

22. Dispositif, comprenant deux systèmes de réacteur d'oxydation, qui sont garnis avec des catalyseurs, qui sont appropriés pour la fabrication d'un composé cible organique par oxydation partielle sous catalyse hétérogène d'un composé précurseur organique, et à la sortie desquels se trouve à chaque fois une conduite pour le déchargement du courant gazeux de produits contenant le composé cible de chaque système de réacteur d'oxydation, qui sont réunies à mesure qu'elles s'éloignent des deux systèmes de réacteur d'oxydation en une conduite de courant gazeux de produits, qui conduit à un dispositif dans lequel le courant gazeux de produits peut être condensé en partie ou en totalité, **caractérisé en ce que** le garnissage catalytique d'un des deux systèmes de réacteur d'oxydation contient au moins une quantité partielle de catalyseur sur laquelle davantage de produit cible a déjà été formé que sur les quantités partielles de catalyseur du garnissage catalytique de l'autre système de réacteur d'oxydation.

23. Procédé selon l'une quelconque des revendications 1 à 21, **caractérisé en ce que** le au moins un composé cible organique comprend au moins une double liaison carbone-carbone éthyléniquement insaturée, et un procédé de fabrication de polymères s'ensuit, lors duquel une polymérisation du au moins un composé cible organique est réalisée.

24. Procédé selon l'une quelconque des revendications 1 à 21, **caractérisé en ce que** le au moins un composé cible organique comprend au moins un groupe carboxyle, et un procédé de fabrication d'esters du composé cible organique par mise en réaction de celui-ci avec un alcool s'ensuit.

25. Procédé selon la revendication 24, **caractérisé en ce que** le au moins un composé cible organique comprend en outre au moins une double liaison carbone-carbone éthyléniquement insaturée, et un procédé de fabrication de polymères s'ensuit, lors duquel au moins un ester est polymérisé.
